# EUROPEAN PATENT APPLICATION

(11) **EP 3 903 825 A1**
(43) Date of publication of application: **03.11.2021**
(21) Application number: 20748044.3
(22) Date of filing: 31.01.2020
(51) Int. Cl.: A61K 47/54, A61K 47/64, A61K 47/65, A61P 35/00, A61P 37/02

(54) **BI-LIGAND DRUG CONJUGATE AND USE THEREOF**

(30) Priority: 30.01.2019 WO PCT/CN2019/073962; 16.01.2020 CN 202010048593
(71) Applicant: Coherent Biopharma (Suzhou), Limited, Industrial Park Suzhou Jiangsu 215123 (CN)
(72) Inventor: HUANG, Baohua Robert, Suzhou, Jiangsu 215123 (CN); TAN, Wei, Suzhou, Jiangsu 215123 (CN); DAI, Jian, Suzhou, Jiangsu 215123 (CN); WANG, Zhongbo, Suzhou, Jiangsu 215123 (CN); LIU, Xiaodong, Suzhou, Jiangsu 215123 (CN); HU, Xinli, Suzhou, Jiangsu 215123 (CN); XIE, Xueyuan, Suzhou, Jiangsu 215123 (CN); SHAO, Jun, Suzhou, Jiangsu 215123 (CN)
(74) Representative: Lahrtz, Fritz
(86) International application number: PCT/CN2020/074117
(87) International publication number: WO 2020/156513

(57) **Abstract**

The present disclosure discloses a conjugate compound or a pharmaceutically acceptable salt thereof, comprising a payload and two targeting molecules. Further disclosed herein is a pharmaceutical composition, comprising the conjugate compound or the pharmaceutically acceptable salt thereof. In addition, The present disclosure additionally relates to a method for delivering a payload to a subject in need thereof, and a method for treating a disease, wherein the methods both comprise administering to the subject a therapeutically effective amount of the conjugate compound or the pharmaceutically acceptable salt thereof, or the pharmaceutical composition as disclosed herein.

SEQUENCE LISTING

<110> COHERENT BIOPHARMA (SUZHOU) CO., LTD.; COHERENT BIOPHARMA (CAYMAN)
CO., LTD.

<120> BI-LIGAND DRUG CONJUGATE AND USE THEREOF

<130> J73849PCEP

<160> 20

<170> PatentIn version 3 .5

<210> 1
<211> 7
<212> PRT
<213> Artificial Sequence

<220>
<223> Recognition site for Matripase
<400> 1
Lys Ser Arg Ala Glu Asp Glu
1               5

<210> 2
<211> 6
<212> PRT
<213> Artificial Sequence

<220>
<223> Recognition site for MMP-2
<400> 2
Pro Leu Gly Leu Ala Gly
1               5

<210> 3
<211> 4
<212> PRT
<213> Artificial Sequence

<220>
<223> Recognition site for prostate-specific antigen
<400> 3
Ser Ser Leu Tyr
1

<210> 4
<211> 7
<212> PRT
<213> Artificial Sequence

<220>
<223> Recognition site for TMPRSS2
<400> 4
Leu Leu Arg Ser Leu Ile Gly

1               5


<210> 5
<211> 4
<212> PRT
<213> Artificial Sequence

<220>
<223> Recognition site for activated protein C
<400> 5
Leu Val Lys Arg
1


<210> 6
<211> 4
<212> PRT
<213> Artificial Sequence

<220>
<223> Recognition site for factor Ixa
<400> 6
Leu Val Val Arg
1


<210> 7
<211> 4
<212> PRT
<213> Artificial Sequence

<220>
<223> Recognition site for factor VIIa
<400> 7
Gln Leu Thr Arg
1


<210> 8
<211> 4
<212> PRT
<213> Artificial Sequence

<220>
<223> Recognition site for factor Xa
<400> 8
Leu Glu Gly Arg
1


<210> 9
<211> 6
<212> PRT
<213> Artificial Sequence

<220>
<223> Recognition site for calpain-a

<400> 9
Pro Leu Phe Ala Glu Pro
1               5

<210> 10
<211> 6
<212> PRT
<213> Artificial Sequence

<220>
<223> Recognition site for calpain-2
<400> 10
Gly Leu Gly Ser Glu Pro
1               5

<210> 11
<211> 5
<212> PRT
<213> Artificial Sequence

<220>
<223> Recognition site for enteropeptidase
<400> 11
Asp Asp Asp Asp Lys
1               5

<210> 12
<211> 4
<212> PRT
<213> Artificial Sequence

<220>
<223> Recognition site for MMP-8
<400> 12
Gly Pro Ser Gly
1

<210> 13
<211> 4
<212> PRT
<213> Artificial Sequence

<220>
<223> Recognition site for proprotein convertase 5
<400> 13
Arg Ser Lys Arg
1

<210> 14
<211> 4
<212> PRT
<213> Artificial Sequence

<220>
<223> Recognition site for calpain-3
<400> 14
Val Gly Val Phe
1

<210> 15
<211> 14
<212> PRT
<213> Artificial Sequence

<220>
<223> P10
<400> 15
Cys Lys Glu Phe Leu His Pro Ser Lys Val Asp Leu Pro Arg
 1               5                   10

<210> 16
<211> 11
<212> PRT
<213> Artificial Sequence

<220>
<400> 16
Glu His Trp Ser Tyr Gly Leu Arg Pro Gly Cys
 1               5                   10

<210> 17
<211> 14
<212> PRT
<213> Artificial Sequence

<220>
<400> 17
Ala Gly Cys Lys Asn Phe Phe Trp Lys Thr Phe Thr Ser Cys
 1               5                   10

<210> 18
<211> 11
<212> PRT
<213> Artificial Sequence

<220>
<221> MISC_FEATURE
<222> (6) ..(6)
<223> Xaa=D-Lys
<400> 18
Glu His Trp Ser Tyr Xaa Leu Arg Pro Gly Cys
 1               5                   10

<210> 19
<211> 9
<212> PRT

<213> Artificial Sequence

<220>
<223> R9
<400> 19
Arg Arg Arg Arg Arg Arg Arg Arg Arg
1               5

<210> 20
<211> 13
<212> PRT
<213> Artificial Sequence

<220>
<223> Tat peptide
<400> 20
Gly Arg Lys Lys Arg Arg Gln Arg Arg Arg Pro Pro Gln
1               5                   10

## Description

### Technical Field

The present application relates to the field of biomedical chemistry. More particularly, the present application relates to a bi-ligand drug conjugate, a pharmaceutical composition comprising the bi-ligand drug conjugate, a method for delivering a payload to a subject in need thereof by using the bi-ligand drug conjugate, and a method for treating a disease with the bi-ligand drug conjugate.

### Background Art

In general, diseased cells and normal cells are significantly different in both pathological and physiological characteristics, and one of the differences lies in that diseased cells have specific or overexpressed materials (such as antigens, chemical signals and receptors) on their surfaces, whereas these materials are absent or lowly expressed in normal cells. On this basis, an antibody-drug conjugate (ADC) and a polypeptide-drug conjugate (PDC) are developed for the treatment of diseases. At present, some ADC- and PDC-based drugs have been marketed or have entered clinical researches; however, due to their design rationale, ADC- and PDC-based drugs have a great limitation in clinical application.

Due to the complexity and relatively large molecular weight of ADCs, the development thereof faces many difficulties, including lack of suitable targets, difficulty in production and poor drug stability. Currently, ADCs are mainly used in the field of tumour treatment. In some instances, the affinity of a targeting antibody to a cancer cell surface antigen can reach 10⁻⁹-10⁻¹² (Kd, mole/litre). Therefore, when having a high specificity to target cells, ADCs also have a high specificity to normal cells with the same targeting receptor as the target cells. Moreover, it may take a long time (1 to 3 weeks) to metabolize ADCs in vivo, during which ADCs continuously kill normal cells, leading to significantly increased toxic and side effects. Therefore, ADCs are more applicable to diseases characterized by a very large difference in the amount of cell surface antigens between tumour cells and normal cells. However, very few diseases known in the art can meet such strict requirement.

PDCs have been used to treat a variety of diseases in clinical or pre-clinical researches, but in such case chemotherapeutics are simply connected to polypeptides, or polypeptides are added to nanoparticles or polymer materials embedded with chemotherapeutic drugs, which will make most of the polypeptides unable to enter cells due to their large molecular weights and bearing charges. Therefore, most of the PDCs are currently only suitable for an extracellular treatment and the application scopes and efficacy of PDCs are severely limited.

A drug conjugate compound can also be a ligand-drug conjugate (LDC), wherein the ligand may be a peptide or a small molecule. However, in terms of bioavailability, stability, efficacy, toxicity, etc., there are various problems in the application of LDCs. For example, due to large molecular weights, lipophilicity or other properties, many ligands are incapable of entering cells, which limits their therapeutic applications. In addition, ligands usually have low efficacy when conjugated with conventional chemotherapeutics (such as doxorubicin and paclitaxel), and when conjugated with highly effective drug molecules (such as MMAE and DM1), ligands may produce a high toxicity, thereby poisoning and even killing animals before a therapeutically effective amount for treating a tumour is achieved.

Therefore, there is also an urgent need in the art to obtain an improved LDC, which can act on highly-expressed receptors widely existing on the surface of diseased cells, broaden a targeting range and a treatment window, enhance drug efficacy and avoid drug side effects.

### Summary of the Invention

In one aspect, the present application discloses a conjugate compound or a pharmaceutically acceptable salt thereof, comprising a payload and two targeting molecules, wherein the two targeting molecules are a synergistic molecule moiety and a prostate-specific membrane antigen ligand moiety, respectively.

In another aspect, the present application discloses a conjugate compound or a pharmaceutically acceptable salt thereof, comprising a payload and two targeting molecules, wherein the two targeting molecules are a synergistic molecule moiety and a ligand moiety represented by formula (I), respectively:

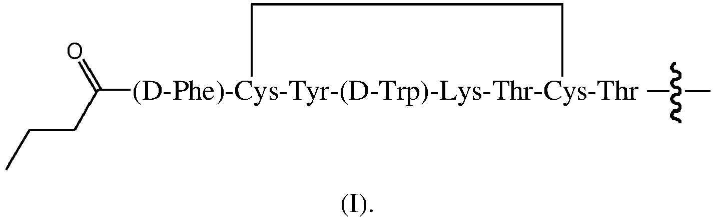

In another aspect, the present application discloses a conjugate compound or a pharmaceutically acceptable salt thereof, comprising a payload and two targeting molecules, wherein the two targeting molecules are a synergistic molecule moiety and P10, respectively, and the payload is camptothecin or any derivative thereof.

In some embodiments, the prostate-specific membrane antigen ligand comprised in the conjugate compound or the pharmaceutically acceptable salt thereof has the following structure:

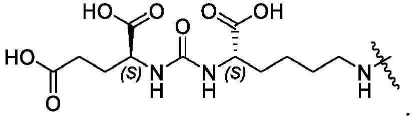

In some embodiments, the prostate-specific membrane antigen ligand comprised in the conjugate compound or the pharmaceutically acceptable salt thereof has the following structure:

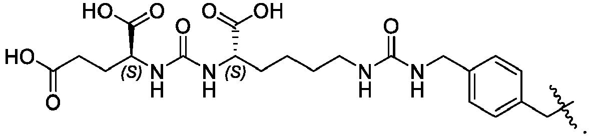

In some embodiments, the prostate-specific membrane antigen ligand comprised in the conjugate compound or the pharmaceutically acceptable salt thereof has the following structure:

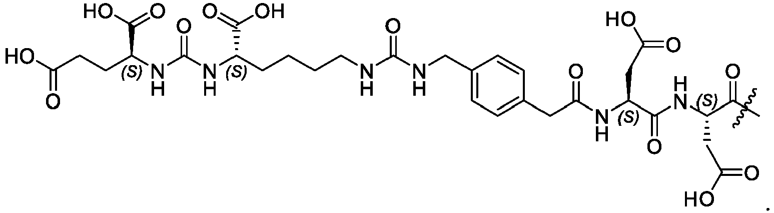

In some embodiments, the prostate-specific membrane antigen ligand comprised in the conjugate compound or the pharmaceutically acceptable salt thereof has the following structure:

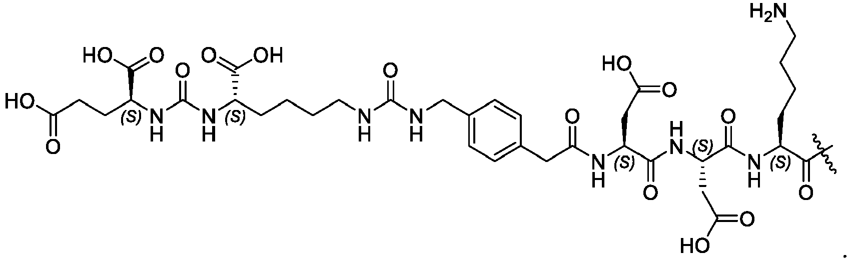

In some embodiments, the two targeting molecules comprised in the conjugate compound or the pharmaceutically acceptable salt thereof are different.

In some embodiments, the synergistic molecule comprised in the conjugate compound or the pharmaceutically acceptable salt thereof is a cell-interacting molecule.

In some embodiments, the two targeting molecules comprised in the conjugate compound or the pharmaceutically acceptable salt thereof are cell-interacting molecules that interact with different cell molecules.

In some embodiments, the synergistic molecule comprised in the conjugate compound or the pharmaceutically acceptable salt thereof is an endocytosis molecule capable of mediating endocytosis.

In some embodiments, the synergistic molecule comprised in the conjugate compound or the pharmaceutically acceptable salt thereof binds to a molecule selected from the group consisting of FOLR1, TRPV6, FOLH1 (PMSA), GNRHR, Her2, Trop2, Her3, NECTIN4, LRP1, GLUT1, EGFR1, AXL, CA9, CD44, Claudin18.2, APN, DLL3, CEACAM5, FZD10, TFRC, MET, IGFR1, SSTR2, CCKBR, LFA1, ICAM, GPR87, GM-CSF, GM-CSFR, TIM3, TLR family, CD40, CD40L, OX40, OX40L, GITRL, GITR, 4-BBL, 4-1BB, CD70, CD27, ICOSL, ICOS, HHLA2, CD28, CD86/80, CD28, MHCII antigen, TCR, CTLA-4, CD155, CD122, CD113, IGIT, PD-L1, PD1, Galectin-9, TIM-3, HVEM, BTLA, CD160, VISTA, B7-H4, B7-H3, phosphatidylserine, HHLA2, LAG3, Galectin-3, LILRB4, SIGLEC15, NKG2A, NKG2D, SLAMF7, KIR2DL1, KIR2DL2, KIR2DL3, FGFR1, FGFR2, FGFR4, NeuGcGM3 and CXCR4.

In some embodiments, the conjugate compound or the pharmaceutically acceptable salt thereof comprises a prostate-specific membrane antigen ligand moiety and a synergistic molecule moiety, wherein the synergistic molecule moiety binds to a molecule selected from the group consisting of FOLR1, TRPV6, FOLH1 (PMSA), SSTR2 and GNRHR.

In some embodiments, the conjugate compound or the pharmaceutically acceptable salt thereof comprises a ligand moiety represented by formula (I) and a synergistic molecule moiety, wherein the synergistic molecule moiety binds to a molecule selected from the group consisting of FOLR1, TRPV6, SSTR2 and GNRHR.

In some embodiments, the conjugate compound or the pharmaceutically acceptable salt thereof comprises P10 and a synergistic molecule moiety, wherein the synergistic molecule binds to a molecule selected from the group consisting of FOLR1, TRPV6, FOLH1 (PMSA) and GNRHR.

In some embodiments, the synergistic molecule comprised in the conjugate compound or the pharmaceutically acceptable salt thereof is folate or any analogue thereof. In some embodiments, the analogue of folate is selected from the group consisting of 5-methyltetrahydrofolate, 5-formyltetrahydrofolate, methotrexate, and 5,10-methylenetetrahydrofolate.

In some embodiments, the conjugate compound or the pharmaceutically acceptable salt thereof comprises one, two, three, four or more payloads. In some embodiments, the payload is selected from the group consisting of a small molecule compound, a nucleotide, a peptide and a protein. In some embodiments, the payload is a small molecule compound. In some embodiments, the small molecule compound is selected from the group consisting of camptothecin and any derivative thereof, auristatin and any derivative thereof, maytansine and any derivative thereof, a radionuclide complex, a cyclooxygenase-2 inhibitor, paclitaxel and any derivative thereof, epothilone and any derivative thereof, bleomycin and any derivative thereof, dactinomycin and any derivative thereof, plicamycin and any derivative thereof, and mitomycin C. In some embodiments, the small molecule compound is camptothecin or any derivative thereof, auristatin or any derivative thereof, maytansine or any derivative thereof, a radionuclide complex or a cyclooxygenase-2 inhibitor.

In some embodiments, the payload comprised in the conjugate compound or the pharmaceutically acceptable salt thereof of the present application is linked to at least one targeting molecule via a linker.

In some embodiments, the linker comprised in the conjugate compound or the pharmaceutically acceptable salt thereof of the present application is a peptide linker, a disulphide linker, a pH-dependent linker or a combination of the above-mentioned linkers.

In some embodiments, the peptide linker is cleavable under a certain physiological environment by protease cleavage or reduction. In some embodiments, the peptide linker is selected from the group consisting of cysteine, lysine, lysine-lysine, valine-citrulline, phenylalanine-lysine, valine-lysine, cysteine-lysine, cysteine-glutamic acid, aspartic acid-aspartic acid, and aspartic acid-aspartic acid-lysine, and optionally, the carboxylic acid in the above-mentioned amino acids is amidated.

In some embodiments, the disulphide linker is selected from the group consisting of DMDS, MDS, DSDM, and NDMDS.

In some embodiments, the pH-dependent linker is a cis-aconitic anhydride.

In some embodiments, the linker of the conjugate compound or the pharmaceutically acceptable salt thereof of the present application has the following structure: 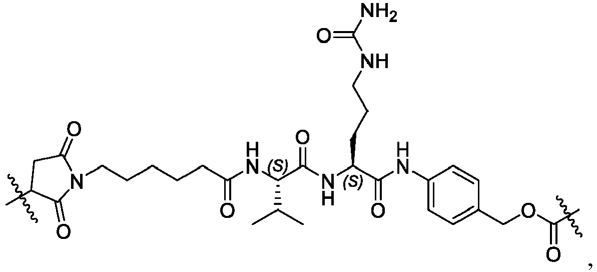 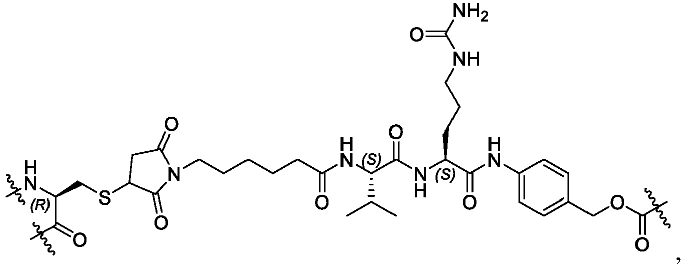 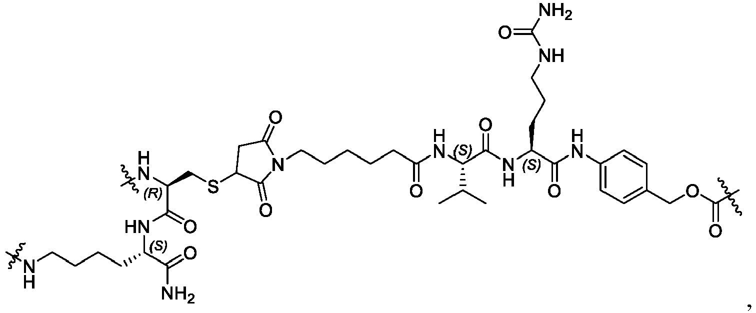 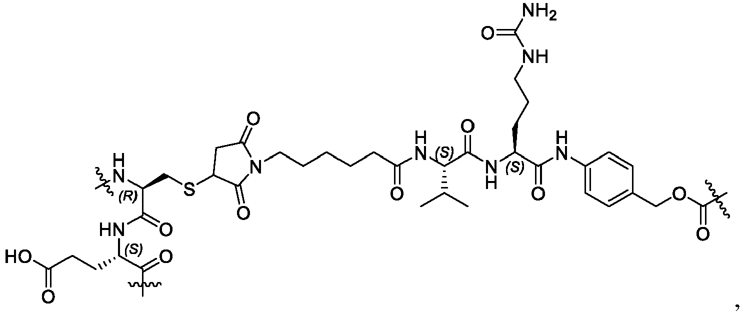 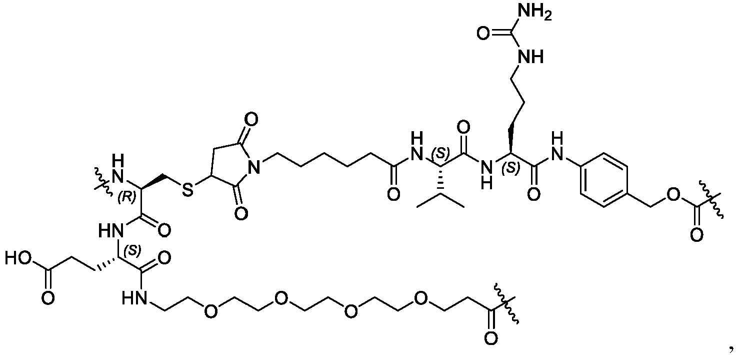 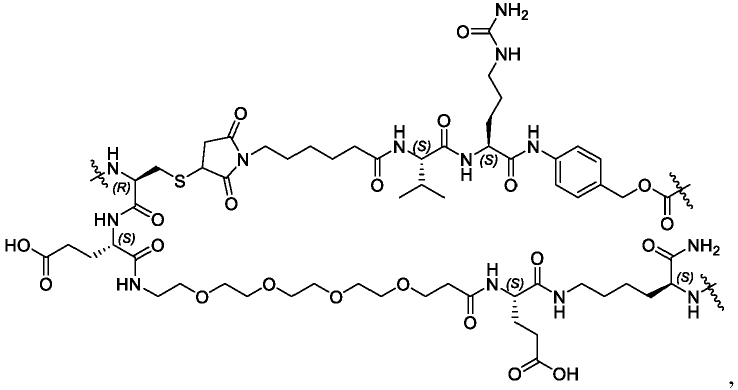 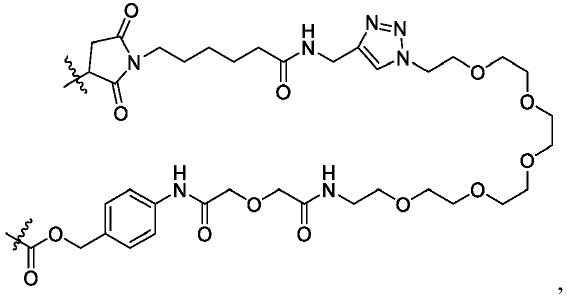 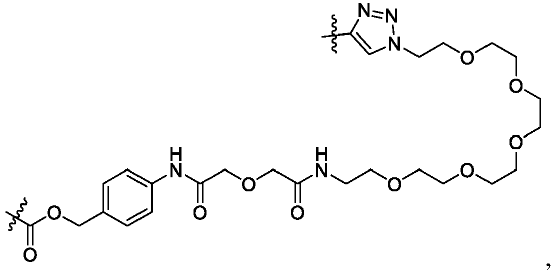 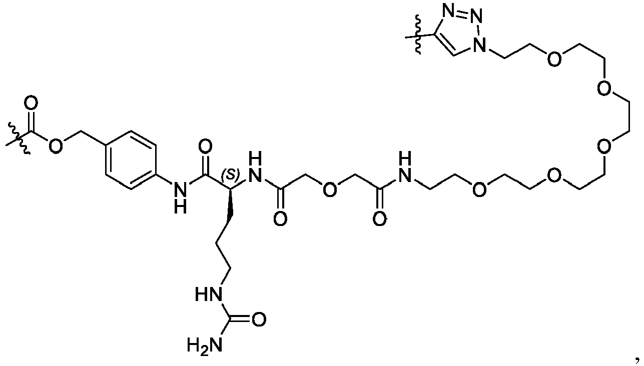 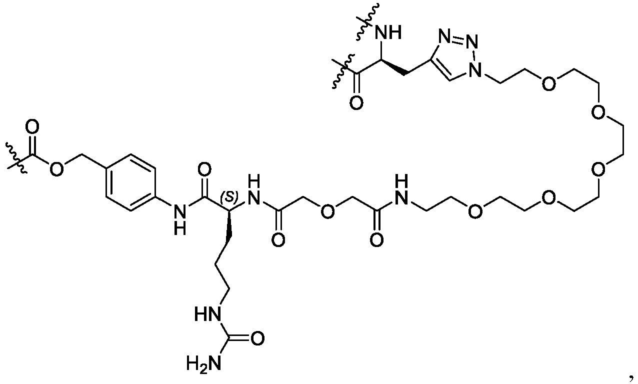 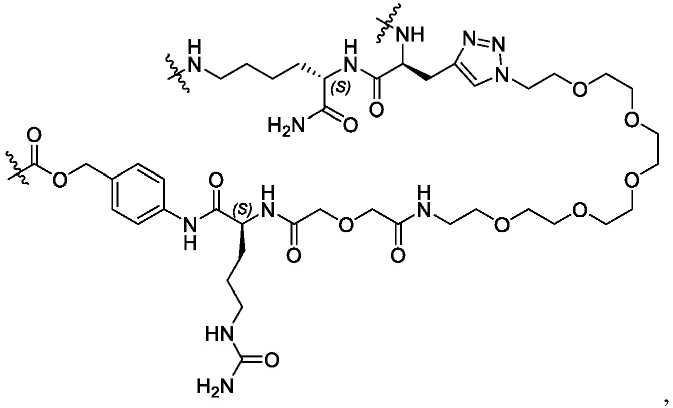 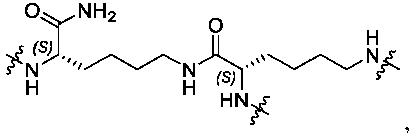 or the linker is a combination of the above-mentioned structures and a peptide linker.

In some embodiments, the two targeting molecules comprised in the conjugate compound or the pharmaceutically acceptable salt thereof of the present application are linked to each other via a spacer. In some embodiments, the spacer described in the present application comprises an amino acid sequence selected from the group consisting of SEQ ID NOs: 1-14, Arg-Arg, Ala-Ser-Asn, Ala-Ala-Ala, Ser-Ser-Arg, Pro-Arg and Pro-Leu-Gly.

In some embodiments, the conjugate compound of the present application is CB-20B which has the following structural formula:

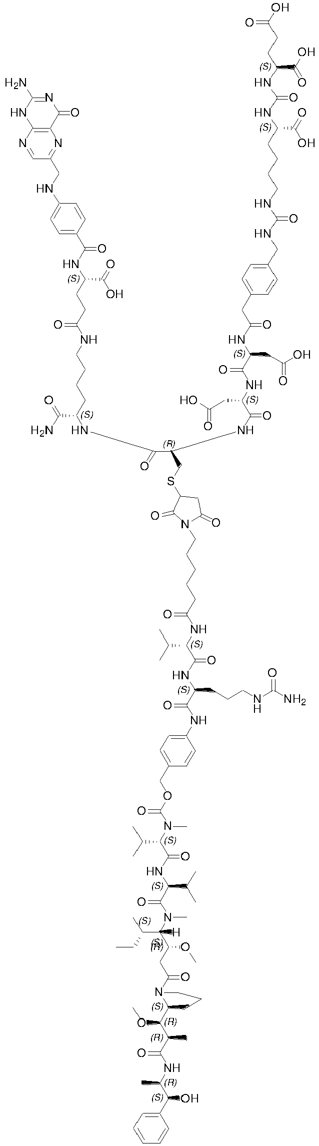

In some embodiments, the conjugate compound of the present application is CB-20BK which has the following structural formula:

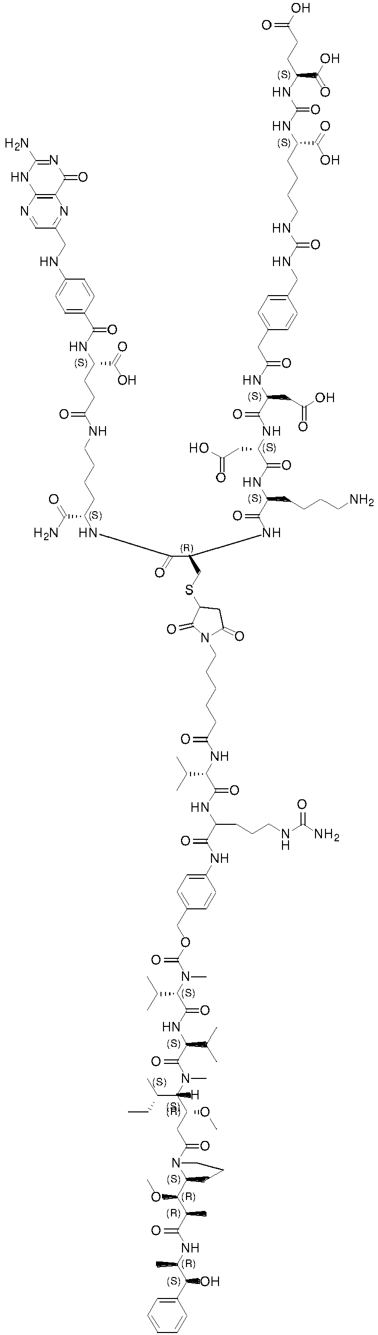

In some embodiments, the conjugate compound of the present application is CB-60S which has the following structural formula:

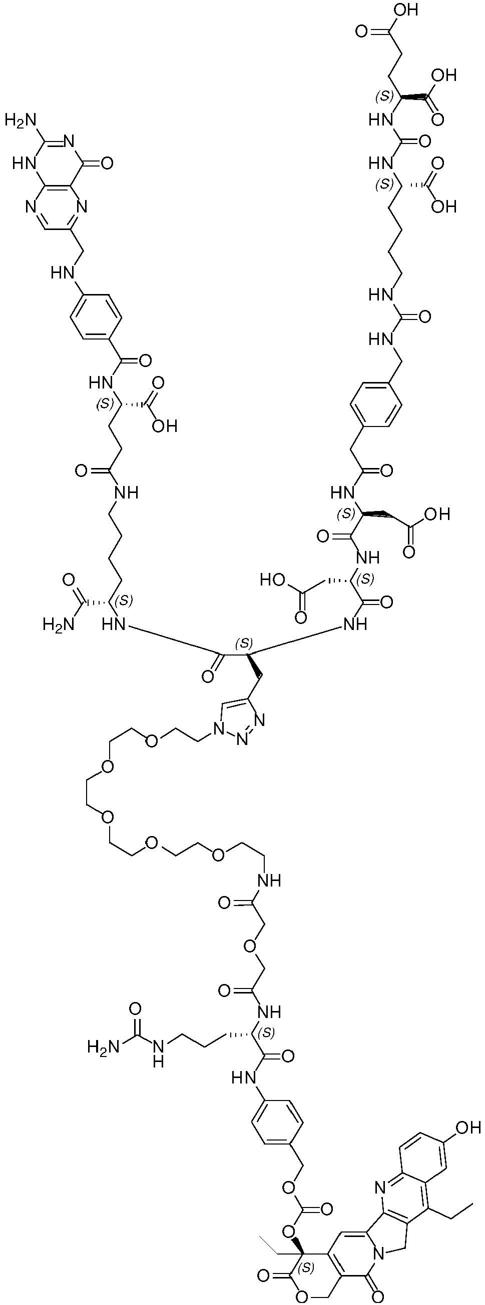

In some embodiments, the conjugate compound of the present application is CB-60SK which has the following structural formula:

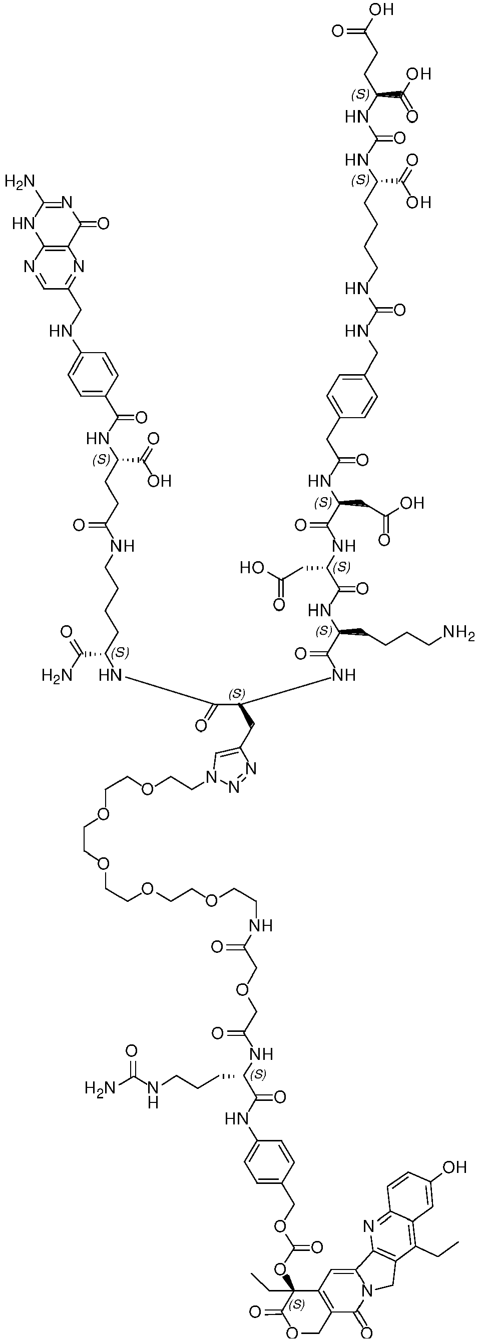

In some embodiments, the conjugate compound of the present application is CB-20C which has the following structural formula:

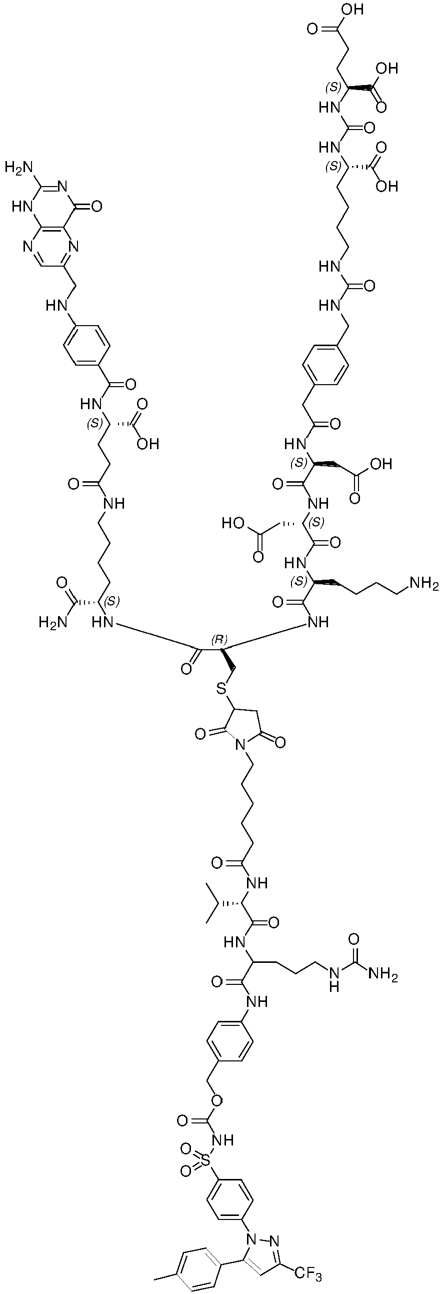

In some embodiments, the conjugate compound of the present application is CB-1020 which has the following structural formula:

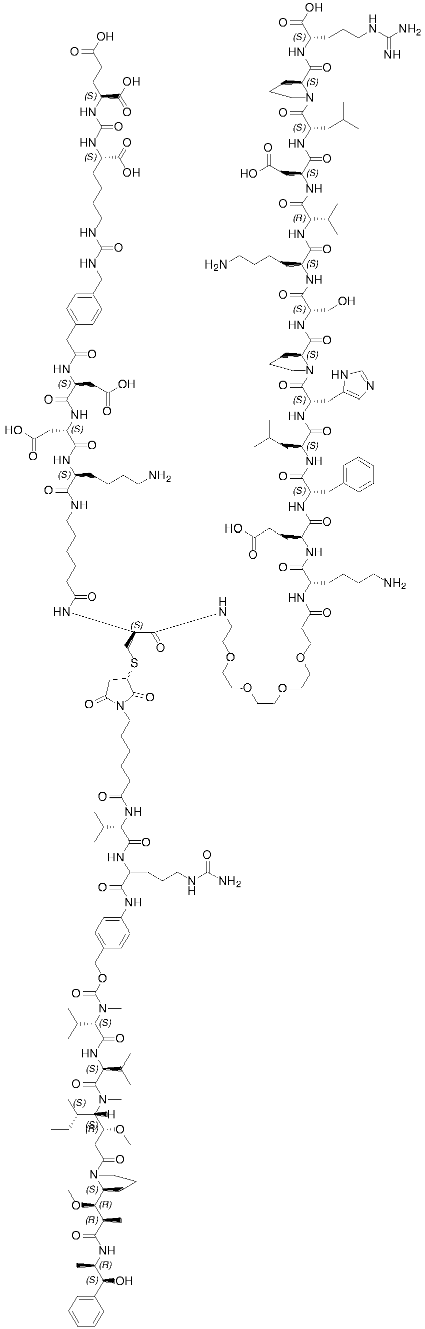

In some embodiments, the conjugate compound of the present application is CB-1320 which has the following structural formula:

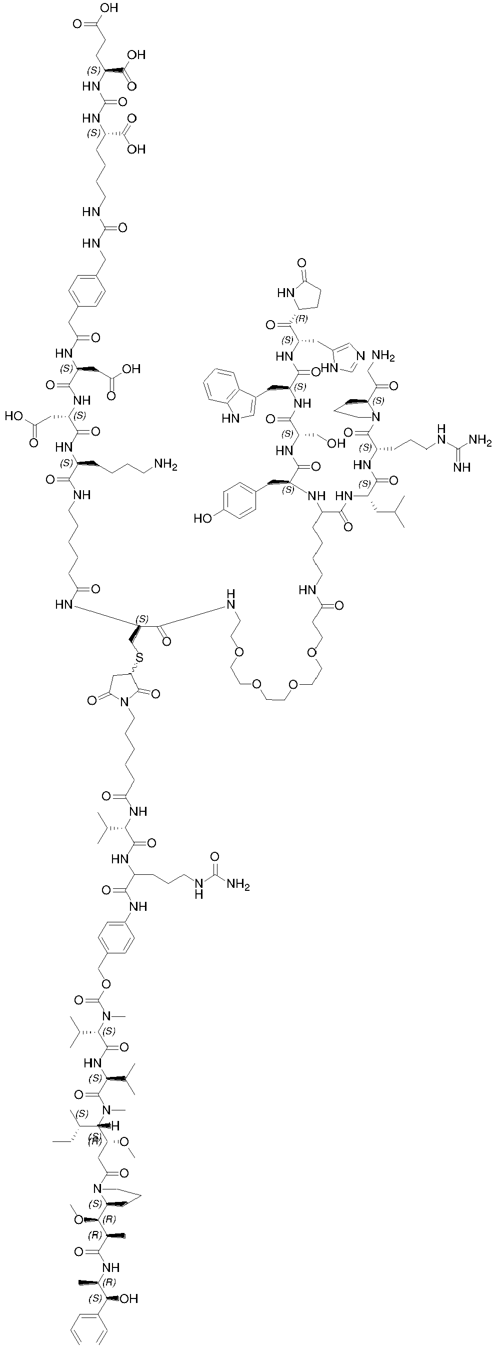

In some embodiments, the conjugate compound of the present application is CB-1820 which has the following structural formula:

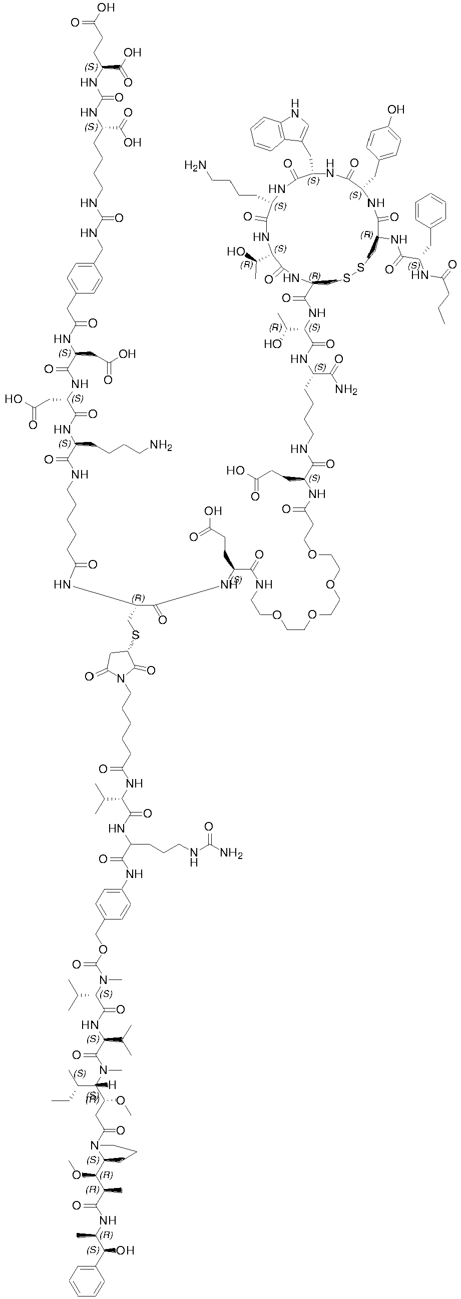

In some embodiments, the conjugate compound of the present application is CR19428 which has the following structural formula:

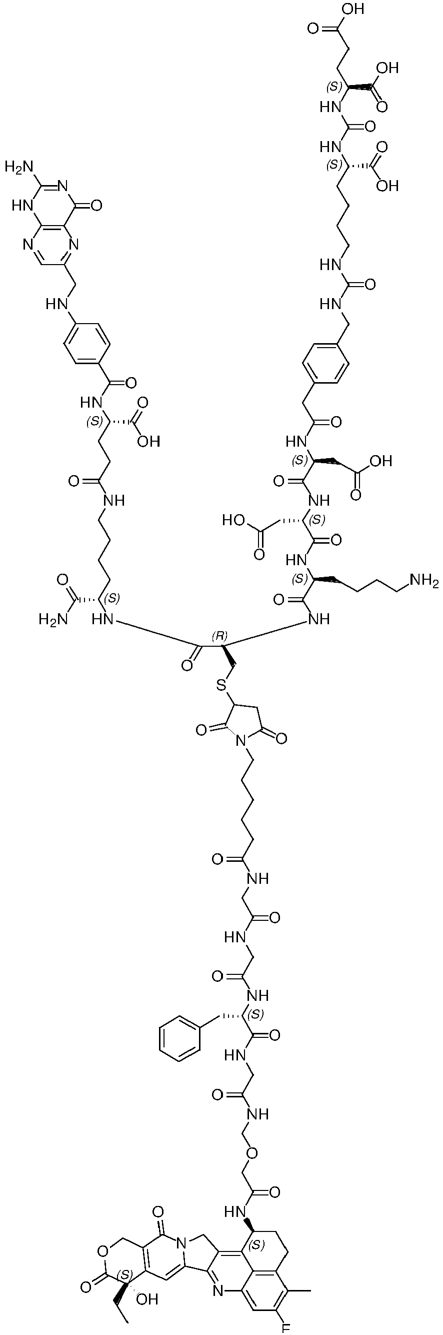

In some embodiments, the conjugate compound of the present application is 20R-SM09 which has the following structural formula:

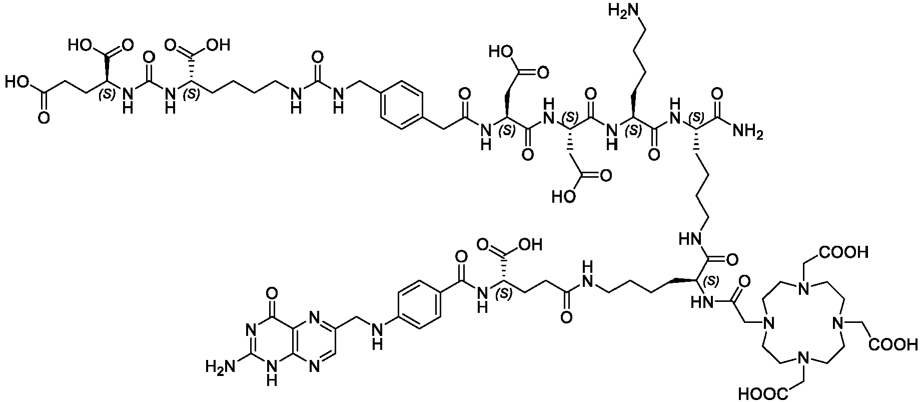

In some embodiments, the conjugate compound of the present application is CB-20R which has the following structural formula: 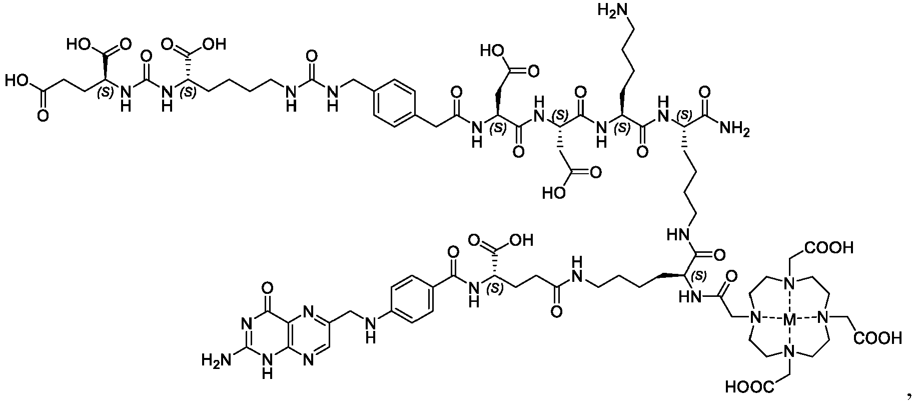 wherein M is a radionuclide.

In some embodiments, the conjugate compound of the present application is CB-18G which has the following structural formula:

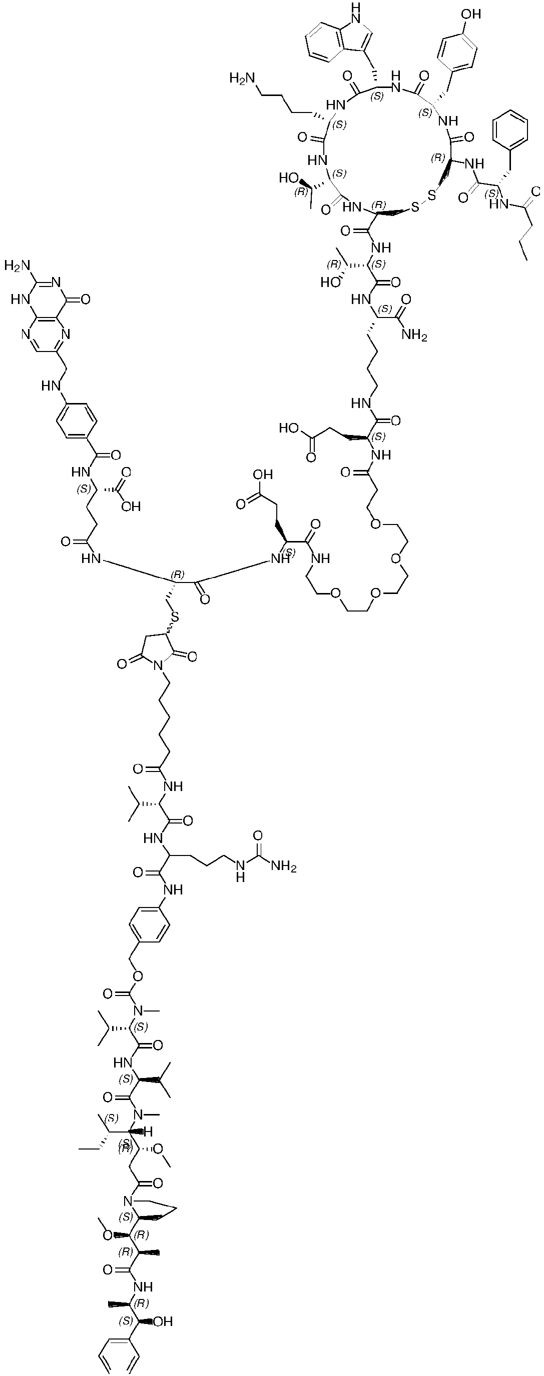

In some embodiments, the conjugate compound of the present application is CR19426 which has the following structural formula:

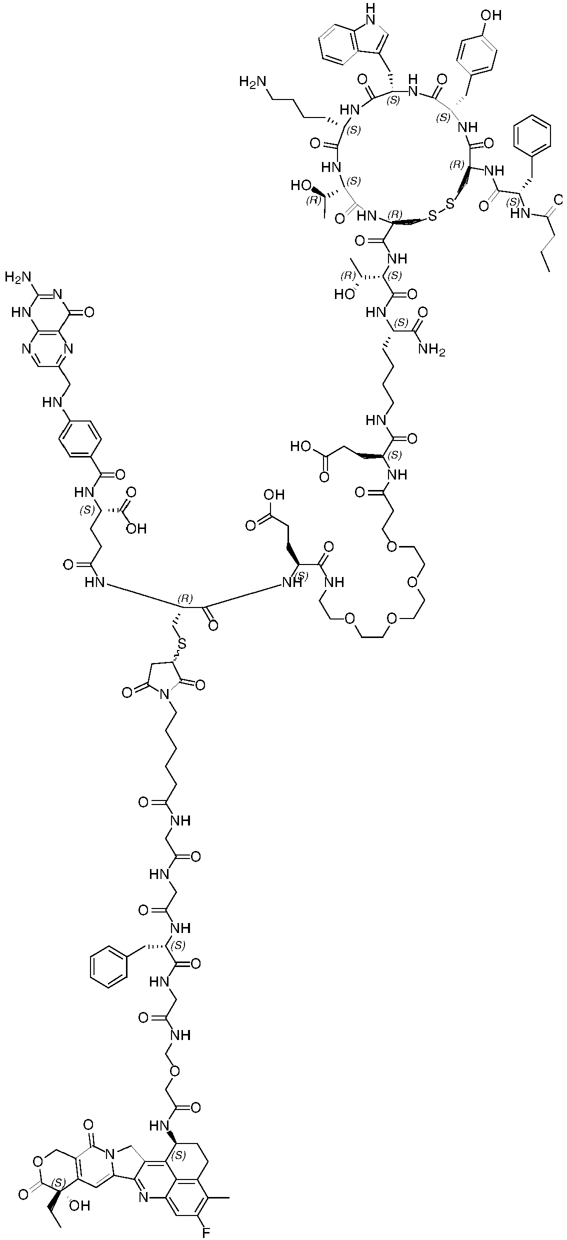

In some embodiments, the conjugate compound of the present application is CB-10S which has the following structural formula:

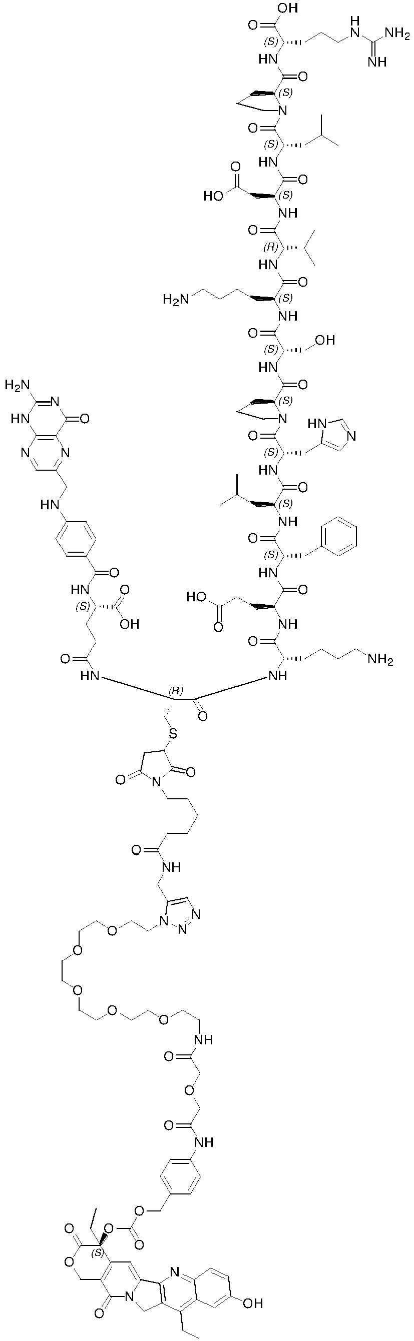

In some embodiments, the conjugate compound of the present application is CR19425 which has the following structural formula:

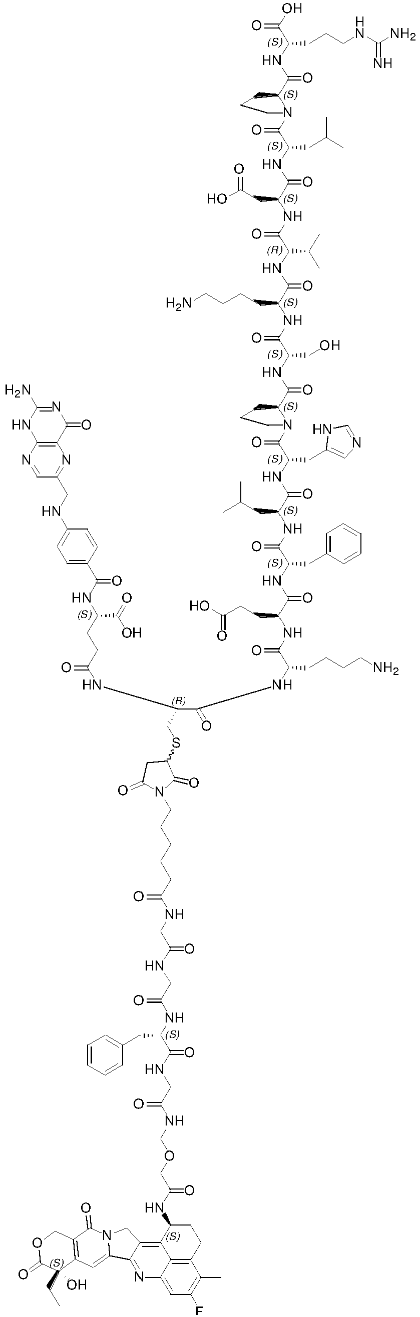

In some embodiments, the conjugate compound of the present application is CB-50S which has the following structural formula:

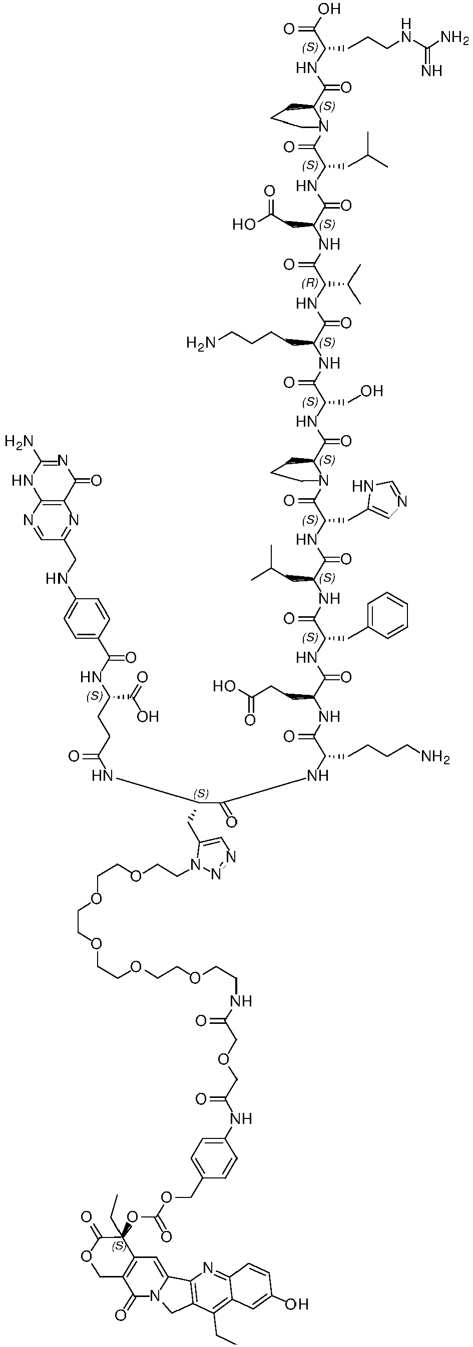

In another aspect, the present application discloses a pharmaceutical composition comprising the conjugate compound or the pharmaceutically acceptable salt thereof of the present application, and a pharmaceutically acceptable carrier.

In some embodiments, the composition is administered intravenously, subcutaneously, orally, intramuscularly or intraventricularly.

In another aspect, the present application discloses a method for delivering a payload to a subject in need thereof, comprising administering to the subject a therapeutically effective amount of the conjugate compound or the pharmaceutically acceptable salt thereof of the present application, or the pharmaceutical composition of the present application.

In another aspect, the present application discloses a method for treating a disease in a subject, comprising administering to the subject a therapeutically effective amount of the conjugate compound or the pharmaceutically acceptable salt thereof of the present application, or the pharmaceutical composition of the present application.

In some embodiments, the method for treating a disease in a subject of the present application further comprises administering one or more therapeutic agents in combination with the conjugate compound or the pharmaceutically acceptable salt thereof, or the pharmaceutical composition.

In yet another aspect, the present application discloses use of the conjugate compound or the pharmaceutically acceptable salt thereof of the present application, or the pharmaceutical composition of the present application in the preparation of a drug for treating a disease in a subject.

In yet another aspect, the present application discloses the conjugate compound or the pharmaceutically acceptable salt thereof of the present application, or the pharmaceutical composition of the present application for treating a disease in a subject.

In some embodiments, the disease is selected from the group consisting of a cancer, an immunological disease, a cardiovascular disease, a metabolic disease, and a neurological disease.

In some embodiments, the cancer is selected from the group consisting of prostatic cancer, breast cancer, lung cancer, renal cancer, leukaemia, ovarian cancer, gastric cancer, uterine cancer, endometrial carcinoma, liver cancer, colon cancer, thyroid cancer, pancreatic cancer, colorectal cancer, oesophageal cancer, skin cancer, lymphoma, and multiple myeloma.

In some embodiments, the immunological disease is an autoimmune disease. In some embodiments, the autoimmune disease is selected from the group consisting of connective tissue disease, systemic sclerosis, rheumatoid arthritis, and systemic lupus erythematosus.

In some embodiments, the cardiovascular disease is selected from the group consisting of angina, myocardial infarction, stroke, heart attack, hypertensive heart disease, rheumatic heart disease, cardiomyopathy, arrhythmia, and congenital heart disease.

In some embodiments, the metabolic disease is selected from the group consisting of diabetes, gout, obesity, hypoglycaemia, hyperglycaemia, and dyslipidaemia.

In some embodiments, the neurological disease is selected from the group consisting of Alzheimer's disease, Parkinson's disease, Huntington's disease, head injury, multiple sclerosis, vertigo, coma, and epilepsy.

### Brief Description of the Drawings

Fig. 1 shows chemical structural formulas of conjugate compounds CB-20B, CB-20BK, CB-60S, CB-60SK, CB-20C, CB-1020, CB-1320, CB-1820, CR19428, 20R-SM09, CB-20R, CB-18G, CR19426, CB-10S, CR19425 and CB-50S.
Fig. 2A shows a curve of Cy5-pep-20BK binding and endocytosis by different cells (from top to bottom: LNCaP cells, SKOV3 cells, DU145 cells and NCI-H460 cells) over time. Fig. 2B shows a curve of Cy5-pep-20AK binding and endocytosis by different cells (from top to bottom: LNCaP cells, SKOV3 cells, DU145 cells and NCI-H460 cells) over time.
Fig. 3 shows fluorescence images of Cy5-FA binding and endocytosis by different cells over time, wherein the fluorescence shown as a complete circle is the fluorescence of cell nuclei, and the fluorescence distributed in a spotty pattern is the fluorescence of Cy5-FA.

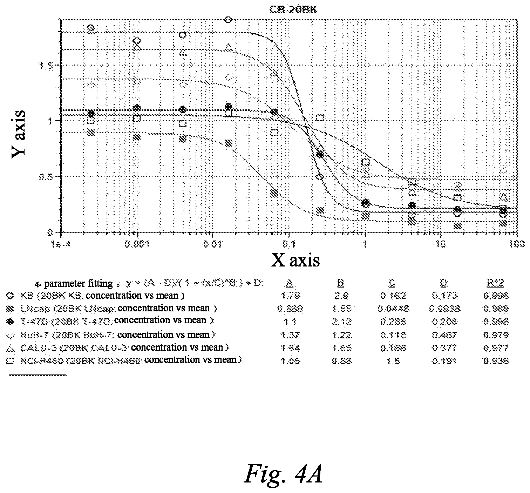

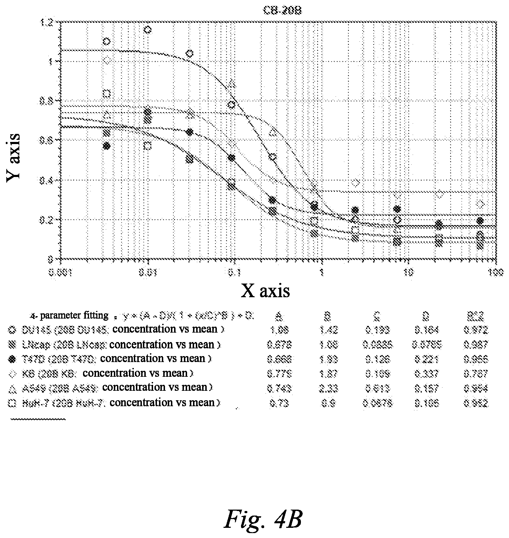

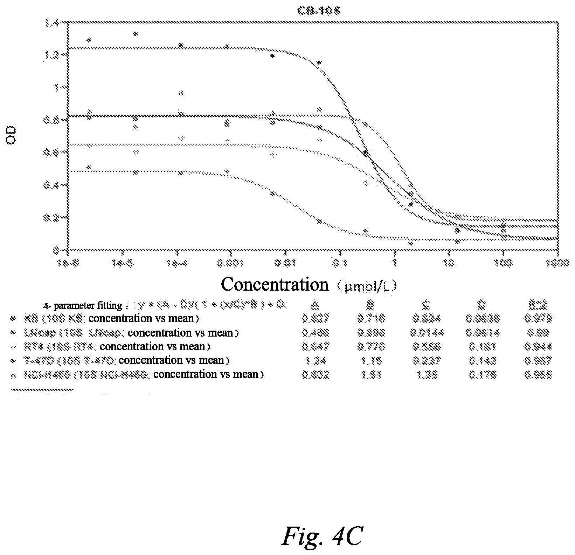

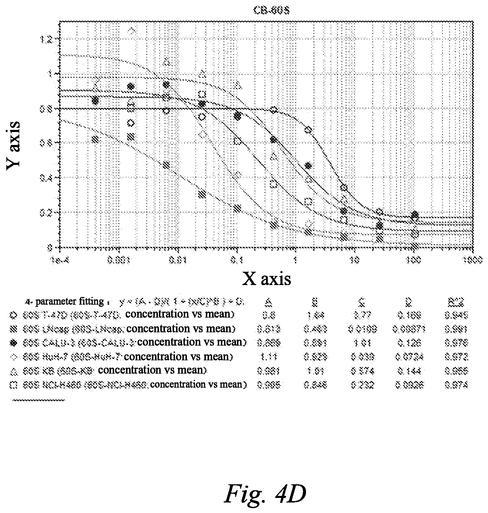

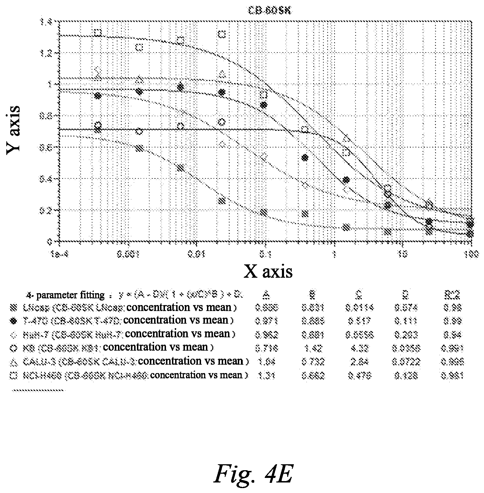

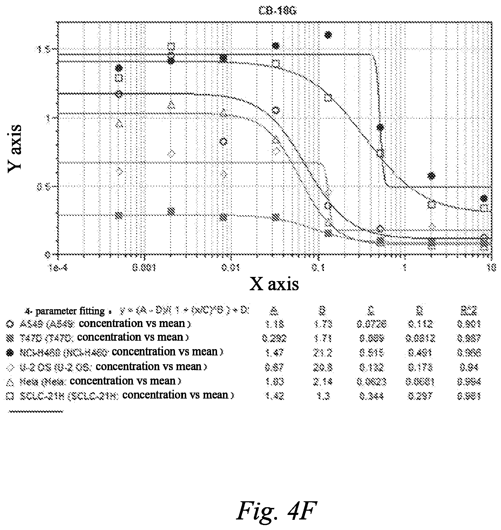

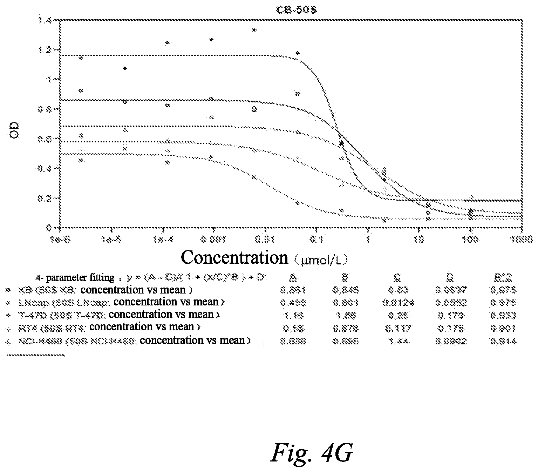

Fig. 4A shows an inhibitory activity of conjugate compound CB-20BK on the amplification of tumour cells as shown. Fig. 4B shows an inhibitory activity of conjugate compound CB-20B on the amplification of tumour cells as shown. Fig. 4C shows an inhibitory activity of conjugate compound CB-10S on the amplification of tumour cells as shown. Fig. 4D shows an inhibitory activity of conjugate compound CB-60S on the amplification of tumour cells as shown. Fig. 4E shows an inhibitory activity of conjugate compound CB-60SK on the amplification of tumour cells as shown. Fig. 4F shows an inhibitory activity of conjugate compound CB-18G on the amplification of tumour cells as shown. Fig. 4G shows an inhibitory activity of conjugate compound CB-50S on the amplification of tumour cells as shown.
Figs. 5A-5E show tumour suppressive effects of conjugate compound CB-20BK in mice.
Figs. 6A-6C show tumour suppressive effects of conjugate compound CB-20B in mice.
Figs. 7A-7E show tumour suppressive effects of conjugate compound CB-18G in mice.
Figs. 8A-8B show effects of CBP-1018 for injection on tumour volumes of LU2505 lung cancer model and LU1206 lung cancer model.

### Detailed Description of Embodiments

While various aspects and embodiments will be disclosed in the present application, it is apparent that a person skilled in the art may make various equivalent changes and modifications to the aspects and embodiments without deviating from the subject spirit and scope of the present application. The various aspects and embodiments disclosed in the present application are only for the purposes of illustration and are not intended to be limiting, with the true scope being indicated by the appended claims. All publications, patents or patent applications cited in the present application are incorporated by reference in their entirety. Unless defined otherwise, the technical and scientific terms as used herein have the same meanings as commonly understood by a person skilled in the art to which the present application belongs.

As used herein and in the appended claims, the singular forms "a", "an", and "the" include plural reference unless the context clearly dictates otherwise. The terms "a" (or "an"), "one or more" and "at least one" can be used interchangeably herein. It is also to be noted that the terms "comprise/comprising", "include/including", and "have/having" can be used interchangeably.

As used herein and in the appended claims, the term "analogue" includes a structural analogue and a functional analogue. The structural analogue refers to a class of compounds with similar chemical structures, which may comprise one or more different atoms or one or more different functional groups. The functional analogue refers to a class of compounds that have the same or similar chemical, biological or pharmacological effects. For example, the analogues of folate include 5-methyltetrahydrofolate, 5-formyltetrahydrofolate, methotrexate, and 5,10-methylenetetrahydrofolate.

As used herein and in the appended claims, the term "derivative" refers to a relatively complex compound derived from a parent compound molecule in which one or more atoms or atomic groups are substituted with other atoms or atomic groups. For example, camptothecin derivatives include irinotecan, SN-38, Dxd, topotecan, GI-147211C, 9-aminocamptothecin, 7-hydroxymethyl camptothecin, 7-aminomethyl camptothecin, 10-hydroxycamptothecin, (20S)-camptothecin, 9-nitrocamptothecin, gimatecan, karenitecin, silatecan, exatecan, diflomotecan, belotecan, lurtotecan and S39625

In one aspect, the present application discloses a conjugate compound or a pharmaceutically acceptable salt thereof, comprising a payload and two targeting molecules, wherein the two targeting molecules are a synergistic molecule moiety and a prostate-specific membrane antigen ligand moiety, respectively.

In another aspect, the present application discloses a conjugate compound or a pharmaceutically acceptable salt thereof, comprising a payload and two targeting molecules, wherein the two targeting molecules are a synergistic molecule moiety and a ligand moiety represented by formula (I), respectively:

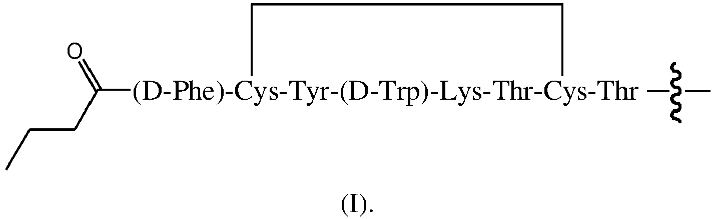

In another aspect, the present application discloses a conjugate compound or a pharmaceutically acceptable salt thereof, comprising a payload and two targeting molecules, wherein the two targeting molecules are a synergistic molecule moiety and P10, respectively, and the payload is camptothecin or any derivative thereof.

The term "payload" as used herein refers to a molecule or material to be delivered to a target cell or tissue. Without limitation, the payload may be any molecule or material that is intended for use in the diagnosis, treatment, or prevention of a disease in a subject. In some embodiments, the payload has a molecular weight of less than or equal to about 5 kDa. In some embodiments, the payload has a molecular weight of less than or equal to about 1.5 kDa. In some embodiments, the payload is a drug or a diagnostic reagent that has been deemed safe and effective for use by appropriate drug approval and registration agencies (such as FDA, EMEA, or NMPA).

In some embodiments, the payload of the present application is a small molecule compound, a nucleotide (such as a DNA, a plasmid DNA, an RNA, an siRNA, an antisense oligonucleotide and an aptamer), a peptide, or a protein (for example, an enzyme). In some embodiments, the payload is a small molecule compound.

In some embodiments, the payloads of the present application include, but are not limited to: an anticancer drug, a radioactive substance, a vitamin, an anti-AIDS drug, an antibiotic, an immunosuppressant, an antiviral drug, an enzyme inhibitor, a neurotoxin, an opioid, a regulator of cell-extracellular matrix interaction, a vasodilator, an antihypertensive drug, a hypnotic, an antihistamine, an anticonvulsant, a muscle relaxant, an anti-Parkinson substance, an anticonvulsant and a drug for inhibiting muscle contraction, an antiparasitic drug and/or an anti-antiprotozoal drug, an analgesic drug, an antipyretic, a steroidal or non-steroidal anti-inflammatory drug, an anti-angiogenic factor, an antisecretory factor, an anticoagulant and/or an antithrombotic agent, a local anaesthetic, a prostaglandin, an antidepressant, an antipsychotic, an antiemetic, or an imaging agent.

In some embodiments, the payload of the present application has a free amino or carboxyl group before being conjugated with the conjugate compound of the present application, and the payload is conjugated with the conjugate compound through an acylation reaction between the above-mentioned amino or carboxyl group and the corresponding part (for example, a linker) of the conjugate compound. In some embodiments, a modification on the above-mentioned free amino or carboxyl group (for example, by conjugating with the conjugate compound of the present application) may significantly reduce the activity of the payload (for example, by at least 50%, 60%, 70%, 80%, 90%, 95%, 98% or 99%).

The "small molecule compound" as used herein refers to a compound having a molecular weight of less than or equal to about 2 kDa. In some embodiments, the small molecule compound has a molecular weight of less than or equal to about 1.5 kDa. In some preferred embodiments, the small molecule compound has a molecular weight of less than or equal to about 1 kDa, 800 Da, 700 Da, 600 Da, or 500 Da. In some embodiments, the small molecule compound of the present application is selected from the group consisting of camptothecin and any derivative thereof (for example, SN38 or Dxd), auristatin and any derivative thereof (for example, MMAE and MMAF), maytansine and any derivative thereof, a cyclooxygenase-2 inhibitor (for example, celecoxib), a radionuclide complex, paclitaxel and any derivative thereof, epothilone and any derivative thereof, bleomycin and any derivative thereof, dactinomycin and any derivative thereof, plicamycin and any derivative thereof, and mitomycin C. In some embodiments, the small molecule compound is camptothecin or any derivative thereof, auristatin or any derivative thereof, a radionuclide complex or a cyclooxygenase-2 inhibitor. In some embodiments, the small molecule compound described in the present application is a drug for relieving or treating a cancer. In some embodiments, the small molecule compound described in the present application is a drug for relieving or treating an autoimmune disease.

The term "camptothecin" as used herein refers to a cytotoxic alkaloid, mainly derived from Camptotheca acuminata (Nyssaceae) and showing a strong anti-tumour activity. The camptothecin and derivative thereof of the present application include a camptothecin and a derivative thereof that are already in existence or will be produced later. The camptothecin and derivative thereof of the present application include, but are not limited to: camptothecin, irinotecan, SN-38, Dxd, GI-147211C, topotecan, 9-aminocamptothecin, 7-hydroxymethyl camptothecin, 7-aminomethyl camptothecin, 10-hydroxycamptothecin, (20S)-camptothecin, 9-nitrocamptothecin, gimatecan, karenitecin, silatecan, exatecan, diflomotecan, belotecan, lurtotecan and S39625.

The term "auristatin and any derivative thereof/auristatin or any derivative thereof" as used herein refers to a natural anti-tumour product, aplysiatoxin 10, and a series of derivatives thereof, wherein such compounds interfere with microscopic self-assembly to arrest cells in a mitotic phase, which has a strong lethality to the cells. The auristatin and any derivative thereof of the present application include auristatin and any derivative thereof that are already in existence or will be produced later. The auristatin and derivative thereof of the present application include, but are not limited to auristatin, monomethyl auristatin E (MMAE), monomethyl auristatin F (MMAF), monomethyl auristatin D (MMAD), AFP and AFHPA.

The term "cyclooxygenase-2 inhibitor" as used herein is a class of specific cyclooxygenase-2 inhibitors. Cyclooxygenase-2 participates in the development and infiltration of malignant tumours through a variety of mechanisms. Cyclooxygenase-2 inhibitors can inhibit tumour cell migration and adhesion and intravascular infiltration, thereby inhibiting the occurrence and development of malignant tumours. The cyclooxygenase-2 inhibitors of the present application include cyclooxygenase-2 inhibitors that are already in existence or will be produced later. Cyclooxygenase-2 inhibitors include, but are not limited to celecoxib, rofecoxib, parecoxib, valdecoxib and etoricoxib.

The term "radionuclide complex" as used herein refers to a special class of complexes containing radionuclides, wherein the chelating agent in the complex can be chelated with the radionuclide and provide a linking part that more stably binds to a target substance. The term "radionuclide" as used herein refers to an element that can spontaneously emit radiation (such as α-rays, β-rays, or γ-rays). The radionuclides of the present application include all radionuclides for treatment and diagnosis that are already in existence or will be produced later. The radionuclides of the present application include, but are not limited to ⁶⁷Cu, ⁶⁴Cu, ⁹⁰Y, ¹⁰⁹Pd, ¹¹¹Ag, ¹⁴⁹Pm, ¹⁵³Sm, ¹⁶⁵Ho, ¹⁶⁶Ho, ¹⁷⁷Lu, ¹⁸⁶Re, ¹⁸⁸Re, ^{99m}Tc, ⁶⁷Ga, ⁶⁸Ga, ¹¹¹In, ⁹⁰Y, ¹⁷⁷Lu, ¹⁸⁶Re, ¹⁸⁸Re, ¹⁹⁷Au, ¹⁹⁸Au, ¹⁹⁹Au, ¹⁰⁵Rh, ¹⁶¹Tb, ¹⁴⁹Pm, ⁴⁴Sc, ⁴⁷Sc, ⁷⁰As, ⁷¹As, ⁷²As, ⁷³As, ⁷⁴As, ⁷⁶As, ⁷⁷As, ²¹²Pb, ²¹²Bi, ²¹³Bi, ²²⁵Ac, ^{117m}Sn, ⁶⁷Ga, ²⁰¹Tl, ¹²³I, ¹³¹I, ¹⁶⁰Gd, ¹⁴⁸Nd, ⁸⁹Sr and ²¹¹At. In some embodiments, the chelating agent is a macrocyclic chelating agent. The chelating agents of the present application include, but are not limited to H2dedpa, H4octapa, H2azapa, DTPA, CHX-A"-DTPA, DTPA-bis anhydride, Maleimide-DTPA, DTPA(tBu)4, DiamSar CB-TE2A, Cyclam, DO2A, DOTA, OTA-GA(tBu)₄, Maleimide-DOTA-GA, p-NCS-Bz-DOTA-GA, NH2-DOTA-GA, DOTA-GA anhydride, DOTA-tris(tBu) ester, Propargyl-DOTA-tris(tBu) ester, DO3AM-acetic acid, DO3AM-N-(2-aminoethyl)ethanamide, DO3AtBu-N-(2-aminoethyl)ethanamide, DOTA-di(tBu)ester, DOTA-tris(tBu)ester NHS ester, DOTA-NHS ester, Propargyl-DOTA-tris(tBu) ester, DOTADOTA-GA anhydride, DOTA-GA(tBu)₄, p-NCS-Bz-DOTA-GA, NH2-DOTA-GA, Maleimide-DOTA-GA, AGuIX, Gado-H, CYCLEN, DO2AtBu, DO3AtBu, DO3AEt, DO3AM, DOTAEt, DOTPrEt, cis-Glyoxal-Cyclen, Mono-N-Benzyl-Cyclen, trans-N-Dibenyl-Cyclen, TriBOC-Cyclen, Mono-N-Benzyl-TACN, DiBOC-TACN, Cross-bridge-Cyclam (CB-Cyclam), (13)aneN4, TACN, TACN·3HCl, TACD, Mono-N-benzyl-TACD, DiBOC-TACD, 1,7-Dioxa-4,10-diazacyclododecane, C-Methyl-Ester-Cyclam, C-Carboxylic-Acid-Cyclam, trans-N-Dimethyl-Cyclam, TETRAM, TETAEt, TETAMEt2, TETAMMe2, TETAM, CPTA, CB-Cyclam derivatives, CB-TE2A, Methylamino-(13)aneN4, Bis-(13)aneN4, Oxo-(13)aneN4, Mono-N-Benzyl-(13)aneN4, TriBOC-(13)aneN4, TRITRAM, TRI3AEt, TRI3AtBu, TRITAM, TRITA, Mono-N-Benzyl-Cyclam, Formaldehyde-Cyclam, cis-Glyoxal-Cyclam, Dioxocyclam, Oxocyclam, trans-N-Dibenzyl-Cyclam, TriBOC-Cyclam, DOTP, DOTMA, TETA, DOTAM, DiAmSar, CB-Cyclam, CB-TE2A, NOTA, NOTAM, NH₂-NODA-GA, Iodo-NODA-GA, NCS-MP-NODA, NH2-MPAA-NODA, NODA-GA(tBu)₃, NODA-GA-NHS ester, Maleimide-NODA-GA, NOTA-NHS ester, Maleimide-NOTA, Propargyl-NOTA(tBu)₂, p-NCS-benzyl-NODA-GA, NOTA(tBu)₂, NCS-MP-NODA, NH₂-MPAA-NODA, NH₂-NODA-GA, Iodo-NODA-GA and TACN.

In some embodiments, the conjugate compound or the pharmaceutically acceptable salt thereof of the present application comprises one payload. In some embodiments, the conjugate compound or the pharmaceutically acceptable salt thereof of the present application comprises two or more payloads. For example, the conjugate compound or the pharmaceutically acceptable salt thereof of the present application comprises 1, 2, 3, 4, 5, 6, 7, 8, 9, 10, 11, 12, 13, 14, 15, 16, 17, 18, 19, 20 or more payloads. In a conjugate compound containing multiple payloads, each of the payloads may be identical to or different from each other. In some embodiments, at least two of the payloads are different from each other.

The term "targeting molecule" as used herein refers to any molecule or moiety capable of targeting the conjugate compound of the present application to a target site, a target tissue, a target organ, a target cell, or a target intracellular region. In some embodiments, the targeting molecule allows the conjugate compound of the present application to be distributed more in a target site, a target tissue, a target organ, a target cell or a target intracellular region compared with a non-target site, a non-target tissue, a non-target organ, a non-target cell or a non-target intracellular region, for example, at least 10% more, 20% more, 50% more, 80% more, 100% more, 150% more, 200% more, 300% more, 400% more, 500% more, etc. In some embodiments, the targeting molecule allows a conjugate compound containing a targeting molecule, compared with a conjugate compound containing no targeting molecule, to be distributed more in a target site, a target tissue, a target organ, a target cell or a target intracellular region, for example, at least 10% more, 20% more, 50% more, 80% more, 100% more, 150% more, 200% more, 300% more, 400% more, 500% more, etc. In some embodiments, the targeting molecule can trigger or promote a conjugate compound containing such targeting molecules to specifically bind to a target molecule, trigger or promote endocytosis of the conjugate compound by a target cell, and trigger or promote the conjugate compound to be enriched around a target cell and/or enter a target cell.

In some embodiments, the conjugate compound of the present application comprises at least two targeting molecules. In some embodiments, the two or more targeting molecules comprised in the conjugate compound of the present application are identical or different. In some embodiments, at least two targeting molecules of the two or more targeting molecules comprised in the conjugate compound of the present application are different. In some embodiments, the two or more targeting molecules comprised in the conjugate compound of the present application are all different from each other. In some embodiments, at least two targeting molecules of the two or more targeting molecules comprised in the conjugate compound of the present application can specifically bind to different cell surface proteins or markers. In some embodiments, the two or more targeting molecules comprised in the conjugate compound of the present application can specifically bind to different cell surface proteins or markers.

In some embodiments, the conjugate compound of the present application comprises at least two targeting molecules, at least one of which is a synergistic molecule.

The term "synergistic molecule" as used herein refers to any molecule or moiety that is capable of working synergistically with other targeting molecules comprised in the conjugate compound of the present application to better trigger or promote the conjugate compound to specifically bind to a target molecule, trigger or promote the endocytosis of the conjugate compound by a target cell, trigger or promote the conjugate compound to be enriched around a target cell and/or enter a target cell, and/or cause the conjugate compound, in a different manner, to specifically bind to a target cell and be maintained. In some embodiments, the synergistic molecule allows the conjugate compound of the present application to be distributed more in a target site, a target tissue, a target organ, a target cell or a target intracellular region compared with a non-target site, a non-target tissue, a non-target organ, a non-target cell or a non-target intracellular region, for example, at least 10% more, 20% more, 50% more, 80% more, 100% more, 150% more, 200% more, 300% more, 400% more, 500% more, etc. In some embodiments, the synergistic molecule allows a conjugate compound containing a synergistic molecule, compared with a conjugate compound containing no synergistic molecule, to be distributed more in a target site, a target tissue, a target organ, a target cell or a target intracellular region, for example, at least 10% more, 20% more, 50% more, 80% more, 100% more, 150% more, 200% more, 300% more, 400% more, 500% more, etc. In some embodiments, the synergistic molecule allows a conjugate compound containing a synergistic molecule, compared with a conjugate compound containing no synergistic molecule, to have a higher activity on a target cell, for example, at least 10% higher, 20% higher, 50% higher, 80% higher, 100% higher, 150% higher, 200% higher, 300% higher, 400% higher, 500% higher, etc.

In some embodiments, the synergistic molecule of the present application is a cell-interacting molecule.

The term "cell-interacting molecule" as used herein refers to a molecule that is capable of interacting with a cell surface material of a target cell to trigger or promote a conjugate compound containing such cell-interacting molecules to specifically bind to a cell, to trigger or promote endocytosis of the conjugate compound by a target cell, and/or to trigger or promote the conjugate compound to be enriched around a target cell and/or enter a target cell.

The cell-interacting molecule may be a small chemical molecule or a large biomolecule. In some embodiments, the cell-interacting molecule is a small molecule compound or a polypeptide. In some embodiments, the cell-interacting molecule is a small molecule compound, or a polypeptide comprising 2-50, 2-40, 2-30, 2-25, 2-22, 2-20, 2-18, 2-15, 2-12, 2-10, 2-8, 4-50, 5-50, 5-40, 5-30, 5-25, 5-22, 5-20, 5-18, 5-15, 5-12, 5-10, 6, 7, 8, or 9 amino acids.

In some embodiments, the targeting molecule is a ligand capable of binding to a cell surface receptor or other molecules. In some embodiments, at least one of the targeting molecules is a ligand capable of binding to a cell surface receptor or other molecules.

The ligands of the present application can include a variety of chemical or biological molecules, which can have a specific binding affinity to a selected target, wherein the selected target can be, for example, a cell surface receptor, a cell surface antigen, a cell, a tissue, an organ, etc. In some embodiments, the ligand can specifically bind to a protein or a marker expressed on the surface of a target cell. In some embodiments, the ligand of the present application binds to a cell surface protein or marker with an affinity of 10⁻⁶-10⁻¹¹ M (K_{d} value). In some embodiments, the ligand of the present application binds to a cell surface protein or marker with an affinity of at least 10⁻⁷, at least 10⁻⁸ and at least 10⁻⁹ M (K_{d} value). In some embodiments, the ligand of the present application binds to a cell surface protein or marker with an affinity of less than 10⁻⁶, less than 10⁻⁷ and less than 10⁻⁸ M (K_{d} value). In some embodiments, the ligand of the present application binds to a cell surface protein or marker with a certain affinity, wherein the certain affinity refers to the affinity of the ligand to a target cell surface protein or marker which is at least two, three, four, five, six, eight, ten, twenty, fifty, one hundred or more times higher than that to a non-target cell surface protein or marker. In some embodiments, the expression of the cell surface protein or marker of the present application in target cells (e.g. cancer cells) is significantly higher than that in normal cells. The term "significantly" as used herein refers to statistically significant differences, or significant differences that can be recognized by a person skilled in the art.

In some embodiments, the expression level of the cell surface protein or marker of the present application in target cells (e.g. cancer cells) are 2 to 1,000,000 times higher than that in normal cells; for example, the expression level in target cells (e.g. cancer cells) are 2 to 10, 2 to 100, 2 to 1,000, 2 to 10,000, 2 to 100,000 or 2 to 1,000,000 (which can be equal to any value within the above numerical range, and the end values of this range included) times higher than that in normal cells. In some embodiments, the expression level of the cell surface receptor in target cells (e.g. cancer cells) is at least 10 times higher, or 100 times higher, or 1,000 times higher, or 10,000 times higher, or 100,000 times higher than that in normal cells. In some embodiments, compared with the level of the cell surface protein or marker on target cells (e.g. cancer cells), the level of the cell surface receptor on normal cells is reduced by at least 50%, 60%, 70%, 80%, 90%, 95%, or 99%. In some embodiments, the cell surface protein or marker described in the present application is undetectable in normal cells.

In some embodiments, the cell surface protein or marker of the present application is a cell surface receptor.

In some embodiments, the cell surface receptor of the present application is selected from the group consisting of a transferrin receptor (TFR), a low-density lipoprotein receptor (LDLR), a folate receptor (FR), a growth hormone-inhibiting hormone receptor, a uric acid kinase receptor, a tumour necrosis factor receptor (TNFR), an integrin receptor (LFA-1), an SST-14 receptor (SSTR2), a GNRH receptor (GNRHR), a TRPV6 and an integrin α receptor.

In some embodiments, the cell surface protein or marker of the present application is a cell surface antigen.

In some embodiments, the cell surface antigen of the present application is selected from the group consisting of a prostate-specific membrane antigen, a MUC1 mucin, an acute lymphoblast common antigen, a Thy-1 cell surface antigen, a Melan-A protein, a squamous cell carcinoma antigen, a galectin 3 and a human leukocyte antigen.

In some embodiments, the cell-interacting molecule of the present application can bind to a molecule selected from the group consisting of FOLR1, TRPV6, FOLH1 (PMSA), GNRHR, Her2, Trop2, Her3, NECTIN4, LRP1, GLUT1, EGFR1, AXL, CA9, CD44, Claudin18.2, APN, DLL3, CEACAM5, FZD10, TFRC, MET, IGFR1, SSTR2, CCKBR, LFA1, ICAM, GPR87, GM-CSF, GM-CSFR, TIM3, TLR family, CD40, CD40L, OX40, OX40L, GITRL, GITR, 4-BBL, 4-1BB, CD70, CD27, ICOSL, ICOS, HHLA2, CD28, CD86/80, CD28, MHCII antigen, TCR, CTLA-4, CD155, CD122, CD113, IGIT, PD-L1, PD1, Galectin-9, TIM-3, HVEM, BTLA, CD160, VISTA, B7-H4, B7-H3, phosphatidylserine, HHLA2, LAG3, Galectin-3, LILRB4, SIGLEC15, NKG2A, NKG2D, SLAMF7, KIR2DL1, KIR2DL2, KIR2DL3, FGFR1, FGFR2, FGFR4, NeuGcGM3 and CXCR4.

In some embodiments, the conjugate compound or the pharmaceutically acceptable salt thereof of the present application comprises a prostate-specific membrane antigen ligand moiety and a synergistic molecule moiety, wherein the synergistic molecule moiety binds to a molecule selected from the group consisting of FOLR1, TRPV6, FOLH1 (PMSA), SSTR2 and GNRHR.

In some embodiments, the conjugate compound or the pharmaceutically acceptable salt thereof of the present application comprises a ligand moiety represented by formula (I) and a synergistic molecule moiety, wherein the synergistic molecule moiety binds to a molecule selected from the group consisting of FOLR1, TRPV6, SSTR2 and GNRHR.

In yet some embodiments, the conjugate compound or the pharmaceutically acceptable salt thereof of the present application comprises P10 and a synergistic molecule moiety, wherein the synergistic molecule binds to a molecule selected from the group consisting of FOLR1, TRPV6, FOLH1 (PMSA) and GNRHR.

In some embodiments, one of the synergistic molecules in the conjugate compound or the pharmaceutically acceptable salt thereof of the present application is an endocytosis molecule moiety capable of mediating endocytosis. The term "endocytosis" as used herein means that the conjugate compound or the pharmaceutically acceptable salt thereof interacts with a target cell and then is capable of mediating its own endocytosis, internalization or uptake by the target cell. The term "endocytosis molecule" as used herein refers to a molecule that interacts with a target cell and then is capable of mediating the endocytosis, internalization, or uptake of the conjugate compound or the pharmaceutically acceptable salt thereof of the present application by the target cell.

In some embodiments, the endocytosis molecule is selected from the group consisting of folate and anyanalogue thereof, a peptide capable of mediating endocytosis, and a cell-penetrating peptide.

In some embodiments, the endocytosis molecule of the present application is folate or any analogue thereof.

Folate is beneficial for forming a chemical bond with other groups due to its small molecule weight, non-immunogenicity, and good stability. Folate can be associated with a folate receptor expressed on a cell surface with a high affinity to mediate a cellular uptake of the folate. Although expressed at a very low level in most normal cells, a folate receptor is expressed at a high level in numerous cancer cells to meet the high folate demand of rapidly dividing cells under a low folate condition (see Kelemen LE, Int J Cancer, 2006; 119:243-50; Kane MA, et al., J Clin Invest. 1988; 81: 1398-406; Matsue H, et al., Proc Natl Acad Sci USA. 1992; 89: 6006-9; Zhao R, et al., Annu Rev Nutr. 2011; 31: 177-201). Folate is capable of specifically binding to a folate receptor on a cell surface, and is also capable of mediating endocytosis of a conjugate compound or a pharmaceutically acceptable salt thereof into target cells.

In some embodiments, the analogue of folate is selected from the group consisting of 5-methyltetrahydrofolate, 5-formyltetrahydrofolate, methotrexate, and 5,10-methylenetetrahydrofolate.

In some embodiments, the endocytosis molecule is a peptide capable of mediating endocytosis.

In some embodiments, the peptide capable of mediating endocytosis comprises an amino acid sequence selected from the group consisting of SEQ ID NO: 16, SEQ ID NO: 17, SEQ ID NO: 18 and Arg-Gly-Asp (named as RGD), and homologous peptides having at least 70%, at least 80%, at least 85%, at least 90%, at least 91%, at least 92%, at least 93%, at least 94%, at least 95%, at least 96%, at least 97%, at least 98%, at least 99% amino acid sequence homology to any of SEQ ID NOs: 16-18, wherein the homologous peptides are functional equivalents of the peptides as shown in SEQ ID NOs: 16-18, respectively.

In some embodiments, the peptide capable of mediating endocytosis as described in the present application has a conservative substitution of an amino acid at only one amino acid site compared to the sequences of SEQ ID NOs: 16-20 and RGD. In some embodiments, the peptide capable of mediating endocytosis as described in the present application has a conservative substitution of an amino acid at 2, 3, 4, 5, 6, 7, 8, 9, or 10 amino acid sites compared to the sequences of SEQ ID NOs: 16-20.

Under the premise of not affecting its biological activity, the peptide capable of mediating endocytosis as described in the present application may also contain non-naturally occurring amino acids, including, for example, β-fluoroalanine, 1-methyl-histidine, γ-methylene-glutamic acid, α-methyl-leucine, 4,5-dehydro-lysine, hydroxyproline, 3-fluoro-phenylalanine, 3-amino-tyrosine, 4-methyl-tryptophan, and the like.

The percentage of homology can be determined by various well-known methods in the art. For example, the comparison of sequences can be achieved by the following publically available tools: BLASTp software (available from the website of National Centre for Biotechnology Information (NCBI): http://blast.ncbi.nlm.nih.gov/Blast.cgi; also see: Altschul S.F. et al., J. Mol. Biol., 215:403-410 (1990); Stephen F. et al, Nucleic Acids Res., 25:3389-3402 (1997)), ClustalW2 (available from the website of European Bioinformatics Institute: http://www.ebi.ac.uk/Tools/msa/clustalw2/; also see: Higgins D.G. et al., Methods in Enzymology, 266:383-402 (1996); Larkin M.A. et al., Bioinformatics (Oxford, England), 23(21): 2947-8 (2007)), and Tcoffee (available from the website of Sweden Bioinformatics Institute; also see: Poirot O. et al., Nucleic Acids Res., 31(13): 3503-6 (2003); Notredame C. et al., J. Mol. Boil., 302(1): 205-17 (2000)). If the alignment of sequences is performed using a software, the default parameters available in the software may be used, or otherwise the parameters may be customized to suit the alignment purpose. All of these are within the scope of knowledge of a person skilled in the art.

The term "functional equivalent" as used herein refers to a derivative peptide that retains a biological activity that is substantially similar to that of the original peptide sequence that the derivative peptide derives from. The functional equivalent may be a natural derivative or is prepared synthetically. Exemplary functional equivalents include amino acid sequences having substitutions, deletions, or additions of one or more amino acids, provided that the biological activity of a peptide is maintained. The amino acid used for substitution desirably has chemico-physical properties similar to the amino acid to be substituted. Desirable similar chemico-physical properties include, similarities in charges, bulkiness, hydrophobicity, hydrophilicity, and the like.

In some embodiments, the functional equivalents include a conservative substitution of an amino acid residue. The conservative substitution of an amino acid residue refers to a substitution between amino acids with similar properties, for example, a substitution between polar amino acids (such as a substitution between glutamine and asparagine), a substitution between hydrophobic amino acids (such as a substitution among leucine, isoleucine, methionine and valine), a substitution between amino acids with identical charges (such as a substitution among arginine, lysine and histidine, or a substitution between glutamic acid and aspartic acid), etc.

In some embodiments, the endocytosis molecule is a cell-penetrating peptide. Cell-penetrating peptides (CPPs), also known as protein transduction domains (PTDs), are short peptides (generally less than 40 amino acids), with the ability to gain access to the interior of cells in a receptor-independent manner. The cell-penetrating peptides, when conjugated with payloads, are capable of mediating the transmembrane transport of the payloads and have a protein transduction activity. In some embodiments, the cell-penetrating peptide described in the present application is selected from the group consisting of a tumour-homing peptide, a mitochondrial penetrating peptide, an activatable cell-penetrating peptide, and an antibacterial peptide. In some embodiments, the cell-penetrating peptide comprises an amino acid sequence selected from the group consisting of SEQ ID NO: 19 (RRRRRRRRR, named as R9) and SEQ ID NO: 20 (GRKKRRQRRRPPQ, which is a Tat peptide, i.e. a cell-penetrating peptide of the HIV transactivator of transcription protein).

In some embodiments, one targeting molecule in the conjugate compound or the pharmaceutically acceptable salt thereof of the present application is a prostate-specific membrane antigen ligand moiety.

The term "prostate-specific membrane antigen" as used herein refers to a type II transmembrane glycoprotein that exists in the membrane of prostate epithelial cells and consists of 750 amino acids, comprising 19 amino acids in the intracellular region, 24 amino acids in the transmembrane region and 707 amino acids in the extracellular region. The prostate-specific membrane antigen is expressed in normal prostate epithelial cells, but is expressed at a much higher level in prostate cancer cells. Compared with the traditional prostate-specific antigen used for clinical detection, the prostate-specific membrane antigen is a more sensitive and specific prostate cancer tumour marker, and especially, it is highly expressed in both hormone-refractory prostate cancer and prostate cancer metastatic lesions, and has a high sensitivity and specificity in distinguishing prostate cancer from other types of malignant tumours. Moreover, in a variety of nonprostate solid tumours (such as lung cancer, bladder cancer, gastric cancer, pancreatic cancer, kidney cancer and colorectal cancer), the prostate-specific membrane antigen is also highly and specifically expressed on tumour vascular endothelial cells.

The term "prostate-specific membrane antigen ligand" as used herein refers to an antibody, an aptamer and a small molecule that is capable of specifically recognizing and binding to a prostate-specific membrane antigen. The prostate-specific membrane antigen ligands of the present application include the prostate-specific membrane antigen ligands that are already in existence or will be produced later, as well as fragments of the aforementioned ligands, as long as these fragments still retain the ability to bind to a prostate-specific membrane antigen. Antibody ligands are the most common prostate-specific membrane antigen ligands, which include, but are not limited to monoclonal antibodies J591, J533, J415 and E99 (for example, see Liu H, Rajasekaran AK, Moy P et al. Constitutive and antibody-induced internalization of prostate-specific memberane antigen [J]. Cancer Res, 1998, 58 (18): 4055-4060). The aptamer is a single-stranded DNA or RNA that is obtained through technical screening by an exponential enrichment ligand system and can bind to prostate-specific membrane antigens with a high affinity and a high specificity. Such prostate-specific membrane antigen ligands include, but are not limited to an xPSM-A10 aptamer and a derivative thereof and an xPSM-A9 aptamer and a derivative thereof (for example, see Lupoid SE et al., Identification and Characterization of nuclease-stabilized RNA molecules that bind human prostate cancer cells via the prostate-specific membrane antigen, Cncer Res, 2002, 62(14):4029-4033). Compared with antibody ligands and aptamer ligands, the prostate-specific membrane antigen small molecule ligands have the advantages of small molecular weight, high permeability, low immunogenicity, and ease of synthesis, and include but are not limited to glutamine urea small molecule ligands and phosphoramidate small molecule ligands.

In some embodiments, the prostate-specific membrane antigen small mole cule ligands of the present application can be selected from the group consist ing of 2-[[methylhydroxyphosphinyl]methyl]glutaric acid; 2-[[ethylhydroxyphos phinyl]methyl]glutaric acid; 2-[[propylhydroxyphosphinyl]methyl]glutaric acid; 2-[[butylhydroxyphosphinyl]methyl]glutaric acid; 2- [[cyclohexylhydroxyphosphin yl]methyl]glutaric acid; 2-[[phenylhydroxyphosphinyl]methyl]glutaric acid; 2-[[2 -(tetrahydrofuranyl)hydroxyphosphinyl]methyl]glutaric acid; 2-[[(2-tetrahydropyra nyl)hydroxyphosphinyl]methyl]glutaric acid; 2-[[((4-pyridyl)methyl)hydroxyphos phinyl]methyl]glutaric acid; 2-[[((2-pyridyl)methyl)hydroxyphosphinyl]methyl]gl utaric acid; 2-[[(phenylmethyl)hydroxyphosphinyl]methyl]glutaric acid; 2-[[((2-p henylethyl)methyl)hydroxyphosphinyl]methyl]glutaric acid; 2-[[((3-phenylpropyl) methyl)hydroxyphosphinyl]methyl]glutaric acid; 2-[[((3-phenylbutyl)methyl)hydr oxyphosphinyl]methyl]glutaric acid; 2-[[((2-phenylbutyl)methyl)hydroxyphosphin yl]methyl]glutaric acid; 2-[[(4-phenylbutyl)hydroxyphosphinyl]methyl]glutaric ac id; and 2-[[(aminomethyl)hydroxyphosphinyl]methyl]glutaric acid; 2-[[methyl h ydroxyphosphinyl]oxy]glutaric acid; 2-[[ethyl hydroxyphosphinyl]oxy]glutaric a cid; 2-[[propyl hydroxyphosphinyl]oxy]glutaric acid; 2-[[butyl hydroxyphosphin yl]oxy]glutaric acid; 2-[[phenyl hydroxyphosphinyl]oxy]glutaric acid; 2-[[((4-py ridyl)methyl)hydroxyphosphinyl]oxy]glutaric acid; 2-[[((2-pyridyl)methyl)hydrox yphosphinyl]oxy]glutaric acid; 2-[[(phenylmethyl)hydroxyphosphinyl]oxy]glutaric acid; and 2[[((2-phenylethyl)methyl)hydroxyphosphinyl]oxy]glutaric acid; 2-[[(n-hydroxyl)carbamoyl]methyl]glutaric acid; 2- [[(n-hydroxyl-n-methyl)carbamo yl]methyl]glutaric acid; 2-[[(n-butyl-n-hydroxyl)carbamoyl]methyl]glutaric acid; 2-[[(n-benzyl-n-hydroxyl)carbamoyl]methyl]glutaric acid; 2-[[(n-hydroxyl-n-phen yl)carbamoyl]methyl]glutaric acid; 2-[[(n-hydroxyl-n-2-phenylethyl)carbamoyl]m ethyl]glutaric acid; 2-[[(n-ethyl-n-hydroxyl)carbamoyl]methyl]glutaric acid; 2-[[(n-hydroxyl-n-propyl)carbamoyl]methyl]glutaric acid; 2-[[(n-hydroxyl-n-3-phen ylpropyl)carbamoyl]methyl]glutaric acid; 2- [[(n-hydroxyl-n-4-pyridyl)carbamoyl] methyl]glutaric acid; 2-[[(n-hydroxyl)amide]methyl]glutaric acid; 2-[[n-hydroxyl (methyl)amide]methyl] glutaric acid; 2-[[n-hydroxyl(benzyl)amide]methyl]glutaric acid; 2-[[n-hydroxyl(phenyl)amide]methyl]glutaric acid; 2-[[n-hydroxyl(2-pheny lethyl)amide]methyl]glutaric acid; 2-[[n-hydroxyl(ethyl)amide]methyl]glutaric aci d; 2-[[n-hydroxyl(propyl)amide]methyl]glutaric acid; 2-[[n-hydroxyl(3-phenylpro pyl)amide]methyl]glutaric acid; and 2-[[n-hydroxyl(4-pyridyl)amide]methyl]gluta ric acid; 2-[(thionyl)methyl]glutaric acid; 2-[(methylthionyl)methyl]glutaric acid; 2-[(ethylthionyl)methyl]glutaric acid; 2-[(propylthionyl)methyl]glutaric acid; 2-[(butylthionyl)methyl] glutaric acid; 2-[(phenylthionyl]methyl]glutaric acid; 2-[[(2-phenylethyl)thionyl]methyl]glutaric acid; 2-[[(3-phenylpropyl)thionyl]methyl] glutaric acid; 2-[[(4-pyridyl)thionyl]methyl]glutaric acid; 2-[(benzylthionyl)meth yl]glutaric acid; 2-[(sulfonyl)methyl]glutaric acid; 2-[(methylsulfonyl)methyl]glu taric acid; 2-[(ethylsulfonyl)methyl]glutaric acid; 2-[(propylsulfonyl)methyl]gluta ric acid; 2-[(butylsulfonyl)methyl]glutaric acid; 2-[(phenylsulfonyl]methyl]glutar ic acid; 2-[[(2-phenylethyl)sulfonyl]methyl]glutaric acid; 2-[[(3-phenylpropyl)sul fonyl]methyl]glutaric acid; 2-[[(4-pyridyl)sulfonyl]methyl]glutaric acid; 2-[(benz ylsulfonyl)methyl]glutaric acid; 2-[(sulfoximinyl)methyl]glutaric acid; 2-[(methy lsulfoximinyl)methyl]glutaric acid; 2-[(ethylsulfoximinyl)methyl]glutaric acid; 2-[(propylsulfoximinyl)methyl]glutaric acid; 2-[(butylsulfoximinyl)methyl]glutaric acid; 2-[(phenylsulfoximinyl]methyl]glutaric acid; 2-[[(2-phenylethyl)sulfoximiny l]methyl]glutaric acid; 2-[[(3-phenylpropyl)sulfoximinyl]methyl]glutaric acid; 2-[[(4-pyridyl)sulfoximinyl]methyl]glutaric acid; and 2-[(benzylsulfoximinyl)methy l]glutaric acid; n-[methyl hydroxyphosphinyl]glutamic acid; n-[ethyl hydroxyph osphinyl]glutamic acid; n-[propyl hydroxyphosphinyl]glutamic acid; n-[butyl hy droxyphosphinyl]glutamic acid; n-[phenyl hydroxyphosphinyl]glutamic acid; n-[(phenylmethyl)hydroxyphosphinyl]glutamic acid; n- [((2-phenylethyl)methyl)hydr oxyphosphinyl]glutamic acid; and N-methyl-N-[phenylhydroxyphosphinyl]gluta mic acid. The prostate-specific membrane antigen ligands of the present appli cation also include all prostate-specific membrane antigen small molecule liga nds disclosed in PCT applications WO 2010/108125 and WO 2006/093991, t he above-mentioned two patent applications are incorporated herein in their e ntirety.

In some embodiments, the prostate-specific membrane antigen small mole cule ligand of the present application is a glutaric acid derivative. In some e mbodiments, the prostate-specific membrane antigen small molecule ligand of the present application is an aminocarbonyl derivative of glutaric acid.

In some embodiments, the prostate-specific membrane antigen small molecule ligand of the present application has the following structure:

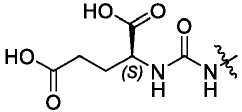

In some embodiments, the prostate-specific membrane antigen ligand comprised in the conjugate compound or the pharmaceutically acceptable salt thereof has the following structure:

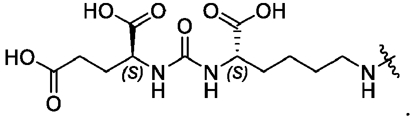

In some embodiments, the prostate-specific membrane antigen ligand comprised in the conjugate compound or the pharmaceutically acceptable salt thereof has the following structure:

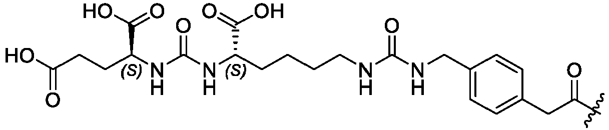

In some embodiments, the prostate-specific membrane antigen ligand comprised in the conjugate compound or the pharmaceutically acceptable salt thereof has the following structure:

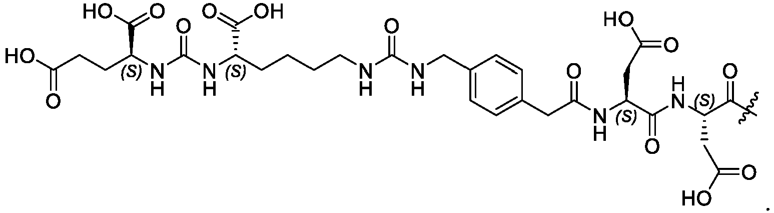

In some embodiments, the prostate-specific membrane antigen ligand comprised in the conjugate compound or the pharmaceutically acceptable salt thereof has the following structure:

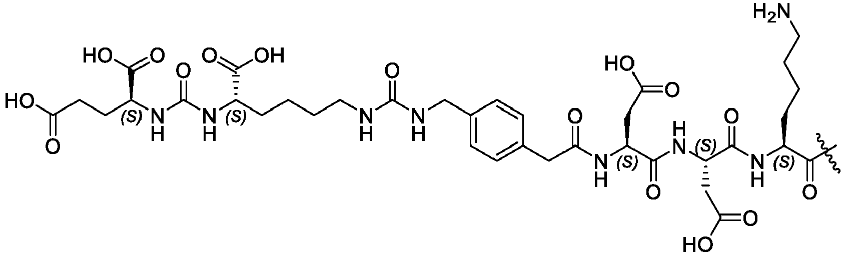

In some embodiments, one targeting molecule in the conjugate compound or the pharmaceutically acceptable salt thereof of the present application is a ligand moiety represented by formula (I): 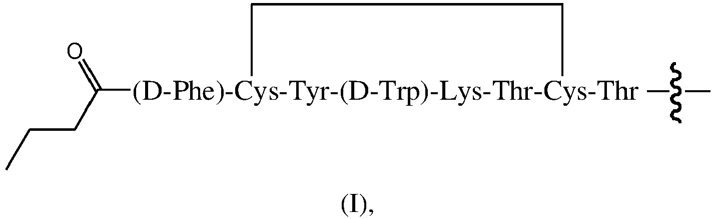 or a ligand moiety having at least 70%, at least 80%, at least 85% or at least 90% amino acid sequence homology thereto or having at most 3, 2 or 1 amino acid substitutions (for example, conservative substitutions) therewith.

In some embodiments, the one targeting molecule in the conjugate compound or the pharmaceutically acceptable salt thereof of the present application is P10 or a ligand moiety having at least 70%, at least 80%, at least 85%, at least 90%, at least 91%, at least 92% or at least 93% amino acid sequence homology thereto or having at most 3, 2 or 1 amino acid substitutions (for example, conservative substitutions) therewith.

The term "P10" as used herein refers to a peptide having an amino acid sequence Cys-Lys-Glu-Phe-Leu-His-Pro-Ser-Lys-Val-Asp-Leu-Pro-Arg.

In some embodiments, the conjugate compound of the present application has targeting molecules selected from the group consisting of (1) a folate ligand and a prostate-specific membrane antigen ligand; (2) a TRPV6 ligand and a prostate-specific membrane antigen ligand; (3) a GNRHR ligand and a prostate-specific membrane antigen ligand; (4) an SSTR2 ligand and a prostate-specific membrane antigen ligand; (5) a folate ligand and an SSTR2 ligand; or (6) a TRPV6 ligand and a folate ligand.

In some embodiments, the two targeting molecules in the conjugate compound or the pharmaceutically acceptable salt thereof provided in the present application are a synergistic molecule moiety and a prostate-specific membrane antigen ligand moiety, respectively. In some embodiments, the synergistic molecule is capable of mediating endocytosis. In some embodiments, the two targeting molecules in the conjugate compound or the pharmaceutically acceptable salt thereof provided in the present application are a folate or an analogue thereof and a prostate-specific membrane antigen ligand moiety, respectively. Without wishing to be limited by the theory, a specific folate or an analogue thereof and a prostate-specific membrane antigen ligand moiety that have a better stability than the ligand combinations in the prior art are selected.

In some embodiments, the two targeting molecules in the conjugate compound or the pharmaceutically acceptable salt thereof provided in the present application are a synergistic molecule moiety and a ligand moiety represented by formula (I), respectively. In some embodiments, the synergistic molecule is capable of mediating endocytosis. In some embodiments, the two targeting molecules in the conjugate compound or the pharmaceutically acceptable salt thereof provided in the present application are a folate or an analogue thereof and a ligand moiety represented by formula (I), respectively.

In some embodiments, the two targeting molecules of the conjugate compound or the pharmaceutically acceptable salt thereof provided in the present application are a synergistic molecule moiety and P10, respectively. In some embodiments, the synergistic molecule is capable of mediating endocytosis. In some embodiments, the two targeting molecules of the conjugate compound or the pharmaceutically acceptable salt thereof provided in the present application are a folate or an analogue thereof and P10, respectively.

In some embodiments, the conjugate compound provided in the present application only comprises a single payload conjugated with the two targeting molecules. In some embodiments, the conjugate compound provided in the present application comprises multiple payloads conjugated with the two targeting molecules.

The term "conjugated" as used herein refer to the linking through a covalent bond of two chemical groups, either directly forming a covalent bond between the two chemical groups, or indirectly linking the two chemical groups via a linker.

In some embodiments, the conjugate compound or the pharmaceutically acceptable salt thereof comprises a payload(s) (for example, 1 payload) and two targeting molecules, wherein the payload is directly covalently linked to at least one of the targeting molecules. In some embodiments, the payload is directly covalently linked to the two targeting molecules.

In some embodiments, the conjugate compound or the pharmaceutically acceptable salt thereof comprises a payload(s) (for example, 1 payload) and two targeting molecules, wherein the payload is covalently linked to at least one of the targeting molecules via a linker. In some embodiments, the payload is covalently linked to the two targeting molecules via a linker.

The term "linker" as used herein refers to a molecule or moiety that covalently links a payload to a targeting molecule. The linkers include a functional group for linking a payload to at least one targeting molecule. In some embodiments, the functional group may comprise two reactive moieties, one for linking to a payload and the other for linking to a targeting molecule. In some embodiments, the functional groups are different from each other. In some embodiments, the functional groups include a group containing a thiol-reacting moiety and an amine-reacting moiety. In some embodiments, the functional groups are identical to each other. In some embodiments, the functional groups are maleimide groups. In some embodiments, the linker contains an amino acid. In some embodiments, the carboxylic acid in the amino acid contained in the linker is amidated. In some embodiments, the linker contains a short chain polyethylene glycol (for example, comprising 2-10, 2-8, 3-8, 4-8, 4-7, 4-6, or 5 repeating units).

In some embodiments, the linker of the present application is a multivalent linker capable of binding to at least one (for example, 1, 2, 3, 4, 5, 6, 7, 8, 9, 10 or more) payload and at least one targeting molecule. The payloads bound to the multivalent linker may be identical or different, and the targeting molecules bound to the multivalent linker may be identical or different.

In one aspect, the linker shall be sufficiently stable to avoid from unintended release of payloads during a blood circulation to increase an effective amount of payloads delivered to target cells or tissues and avoid toxicity. In another aspect, the linker shall be capable of releasing the payloads around or within target cells to efficiently kill target cells or block functions of target cells. In some embodiments, the linker comprises at least one cleavable functional group. Preferably, the cleavable functional group is sufficiently stable outside a target cell, but upon entry into the target cell, is cleaved to release a payload(s). In some embodiments, the cleavable functional group is cleaved at least 10, 20, 30, 50, 100 times or more efficiently in target cells than in the blood or serum.

The cleavable linker may be cleaved by a hydrolysis, an enzymatic reaction, or a reduction reaction, or by a pH change. In some embodiments, the linker is cleavable under a certain physiological environment (for example, under an appropriate pH environment). In some embodiments, the linker is cleavable in an acidic environment with a pH of about 6.5 or lower, or by reagents such as enzymes. In some embodiments, the linker is susceptible to cleavage agents, for example, pH, redox potential or the presence of degradative molecules.

In some embodiments, the linker is non-cleavable. Non-cleavable linkers as used herein refer to linkers which remain basically intact during intracellular metabolism.

In some embodiments, the linker is a peptide linker consisting of a straight or branched chain amino acids linked by peptide bonds. In some embodiments, the peptide linker is cleavable by a protease that is highly or specifically expressed around or in target cells, for example, cathepsin B in the lysosome or endosome. The peptide linker as used herein can be of varying lengths. Typically, the peptide linker of the present application is from 1 to 50 amino acids in length. In some embodiments, the peptide liker is from 1 to 45, from 1 to 40, from 1 to 35, from 1 to 30, from 1 to 25, from 1 to 20, from 1 to 15, from 1 to 10, from 1 to 9, from 1 to 8, from 1 to 7, from 1 to 6, from 1 to 5, from 1 to 4, from 1 to 3, from 1 to 2 or 1 amino acids in length. In some embodiments, the peptide liker is from 2 to 45, from 2 to 40, from 2 to 35, from 2 to 30, from 2 to 25, from 2 to 20, from 2 to 15, from 2 to 10, from 2 to 9, from 2 to 8, from 2 to 7, from 2 to 6, from 2 to 5, from 2 to 4, from 2 to 3 or 2 amino acids in length. The number of amino acids of the peptide linker as described in the present application can be equal to any integer value within the above numerical range, including the end values of this range. In some embodiments, the peptide linker is preferred to be 1, 2, 3, 4 or 5 amino acids in length. In some embodiments, the peptide linker is cysteine, lysine, lysine-lysine, valine-citrulline, phenylalanine-lysine, valine-lysine, cysteine-lysine, cysteine-glutamic acid, aspartic acid-aspartic acid, and aspartic acid-aspartic acid-lysine, and optionally, the carboxylic acid in the above-mentioned amino acids is amidated.

In some embodiments, the linker is a disulphide linker containing a disulphide bond. The disulphide bond may be cleaved under an intracellular reductive environment, while remains stable in a circular system. The disulphide linker of the present application may be DSDM, DMDS, MDS, or NDMDS. The structures of these disulphide linkers are shown in Table 1 below.

**Table 1: Structures of DSDM, DMDS, MDS and NDMDS**

| **Name** | **Structure** |
|---|---|
| DSDM | 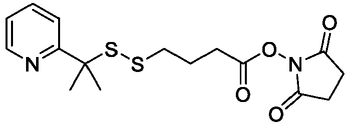 |
| DMDS | 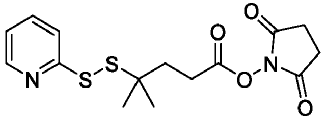 |
| MDS | 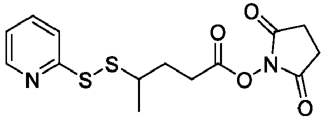 |
| NDMDS | 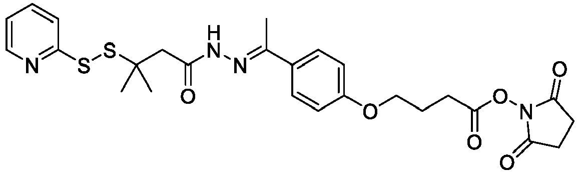 |

In some embodiments, the linker is a pH-dependent linker. The pH-dependent linker as described in the present application is cleavable under a certain pH environment. In some embodiments, the pH-dependent linker may be stable under an alkaline condition, while cleavable under an acidic condition, for example, under a pH value of 6.5 or lower. In some embodiments, the pH-dependent linker is a cis-aconitic anhydride.

In some embodiments, the linker of the conjugate compound or the pharmaceutically acceptable salt thereof is 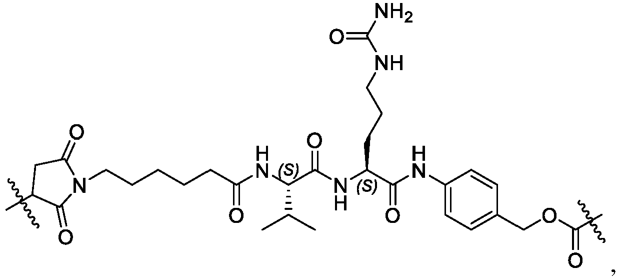 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 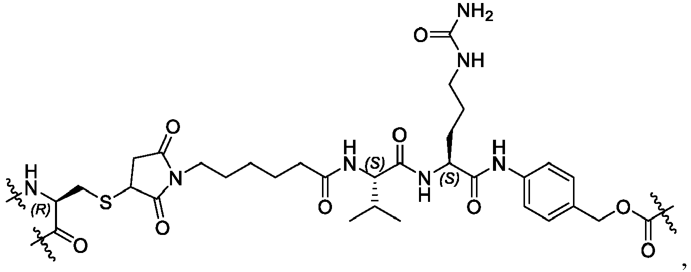 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 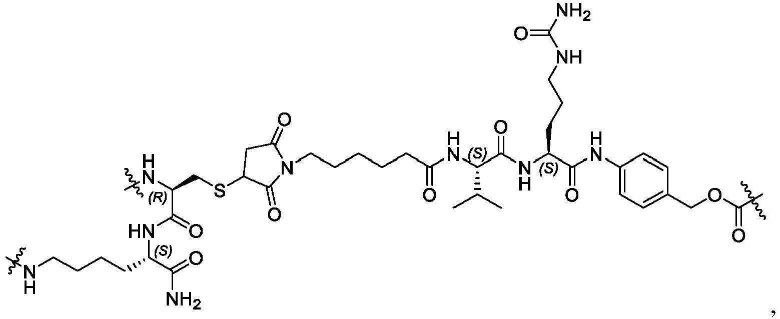 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 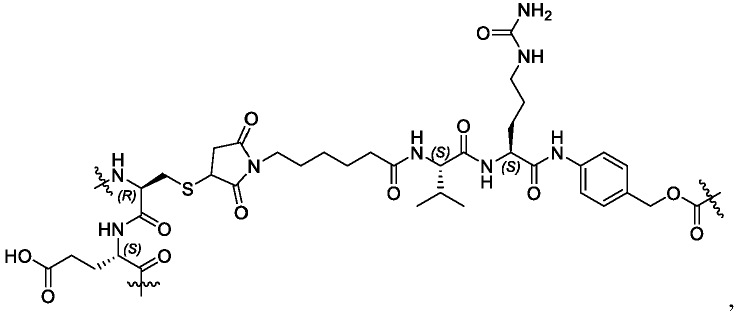 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 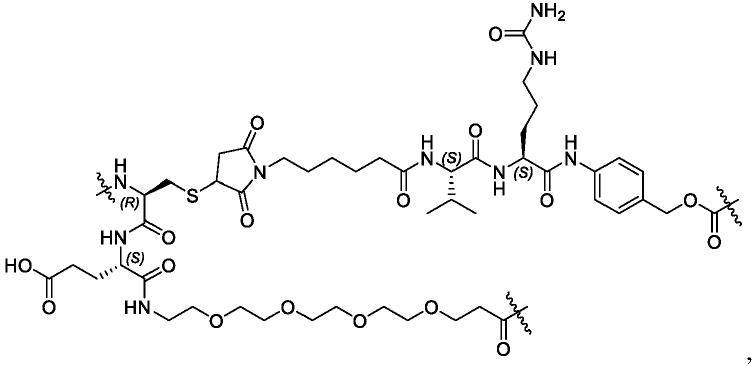 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 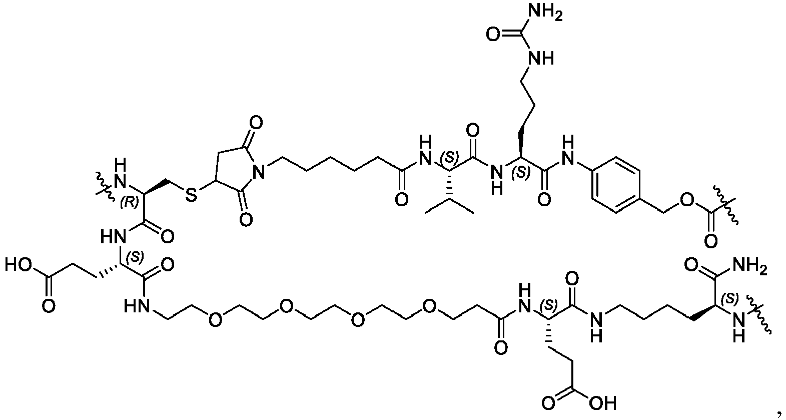 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 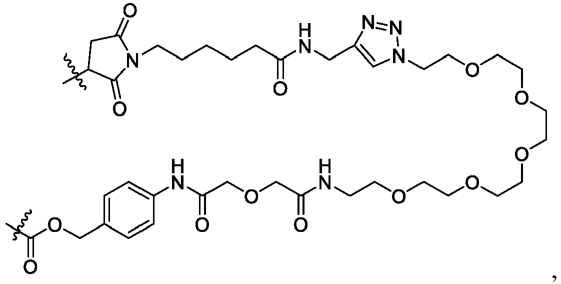 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 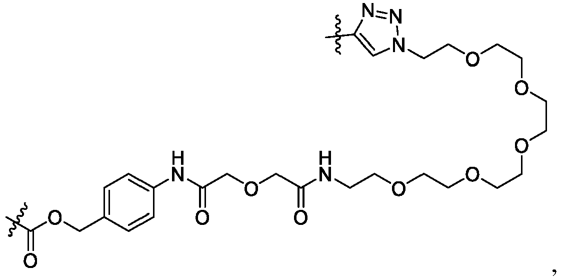 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 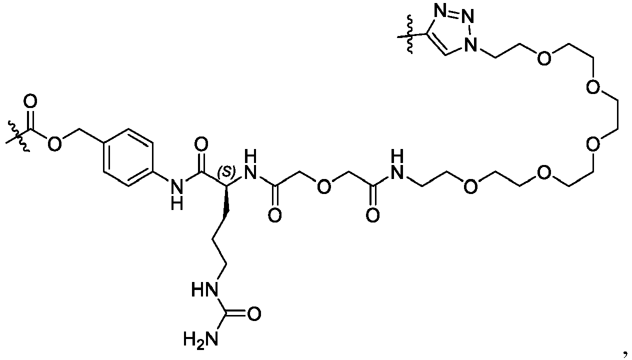 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 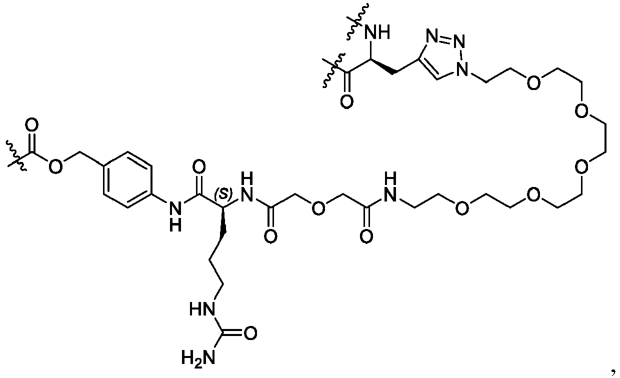 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 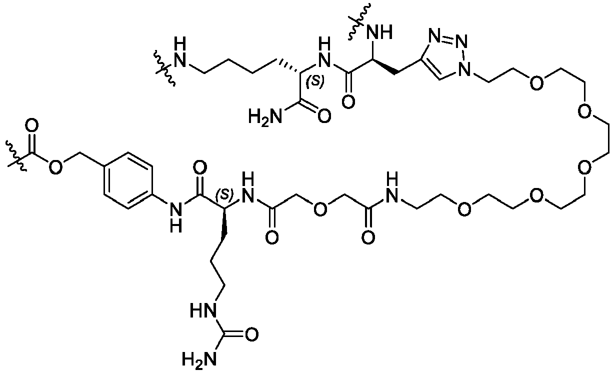 or a combination of the above-mentioned structure and a peptide linker (for example, binding to a targeting molecule through a peptide linker containing 1-3 amino acids).

In some embodiments, the linker of the conjugate compound or the pharmaceutically acceptable salt thereof has the following structure: 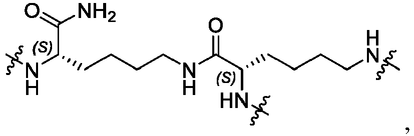 or a combination of the above-mentioned structure and a peptide linker (for example, binding to a targeting molecule through a peptide linker containing 1-3 amino acids).

In some embodiments, the linker of the present application may comprise any one of or a combination of the linkers as described above.

In some embodiments, the payload is conjugated with a first targeting molecule directly or indirectly, and the first targeting molecule is conjugated with a second targeting molecule directly or indirectly. In some embodiments, the payload is conjugated with each of the first and the second targeting molecule directly. In some embodiments, the payload is conjugated with each of the first and the second targeting molecule indirectly. In some embodiments, the payload is conjugated with the first targeting molecule indirectly, e.g. via a linker, and the first targeting molecule is conjugated with the second targeting molecule directly or indirectly. In some embodiments, the payload is conjugated with the first targeting molecule via a first linker, and the payload is conjugated with the second targeting molecule via a second linker. In some embodiments, the linker is a multivalent linker that binds to at least one (for example, 1, 2, 3, 4, 5, 6, 7, 8, 9, 10 or more) payload and two targeting molecules.

In some embodiments, the two targeting molecules are linked to each other via a spacer. In some embodiments, the spacer is cleavable by a protease that is specifically expressed in target cells or to be expressed in target cells. Such proteases include, for example, the proteases as listed in Table 2 below. In some embodiments, the spacer comprises the amino acid sequence selected from any one of the amino acid sequences as listed in Table 2 below.

**Table 2: List of Enzymatically Cleavable Sequences**

| **Protease** | **Amino acid sequence of recognition site** | **SEQ ID NO.** |
|---|---|---|
| Cathepsin B | RR | - |
| Legumain | ASN | - |
| Matripase | KSRAEDE | SEQ ID NO: 1 |
| MMP-2 | PLGLAG | SEQ ID NO: 2 |
| Prostate-specific antigen | SSLY | SEQ ID NO: 3 |
| Stromelysin-3 | AAA | - |
| TMPRSS2 | LLRSLIG | SEQ ID NO: 4 |
| Urokinase-type plasminogen activator | SSR | - |
| Activated protein C | LVKR | SEQ ID NO: 5 |
| Factor Ixa | LVVR | SEQ ID NO: 6 |
| Factor VIIa | QLTR | SEQ ID NO: 7 |
| Factor Xa | LEGR | SEQ ID NO: 8 |
| Thrombin | PR | - |
| Calpain-a | PLFAEP | SEQ ID NO: 9 |
| Calpain-2 | GLGSEP | SEQ ID NO: 10 |
| Enteropeptidase | DDDDK | SEQ ID NO: 11 |
| MMP-8 | GPSG | SEQ ID NO: 12 |
| Cathepsin L | PLG | - |
| Proprotein convertase 5 | RSKR | SEQ ID NO: 13 |
| Calpain-3 | VGVF | SEQ ID NO: 14 |

The terms "cleavable" or "cleaved" as used herein refer to a metabolic process or reaction process on the conjugate compound provided in the present application, whereby a linker between a payload and a targeting molecule, or a spacer between targeting molecules are broken to release free payload or targeting molecule. The linker and spacer is either cleaved by a protease or cleaved under a certain physiological environment, e.g. a pH environment.

In some embodiments, the conjugate compound has a structure of formula I, II, III, or IV shown as follows, wherein n, m, p and q are independently 0 or 1, which represent that the linker and spacer are present or absent independently. The "molecule" in the following formula is an abbreviation for "targeting molecule".

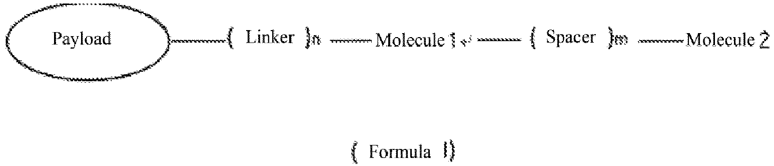

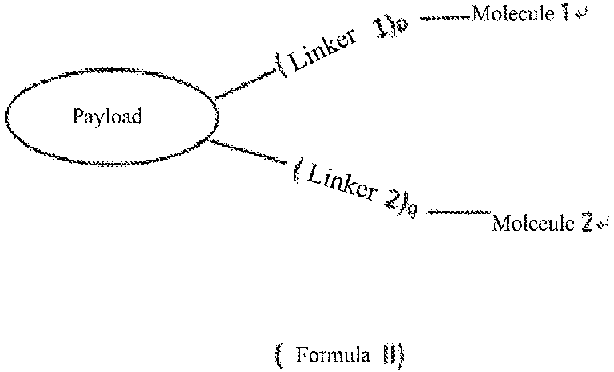

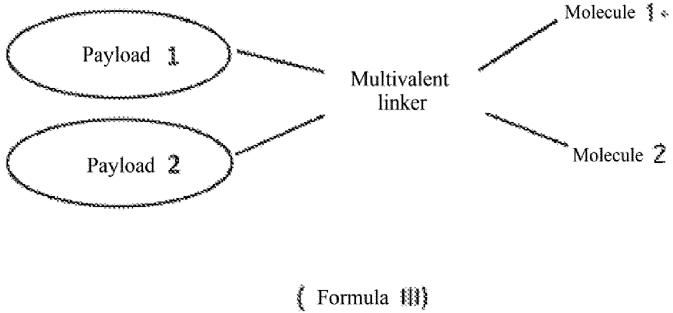

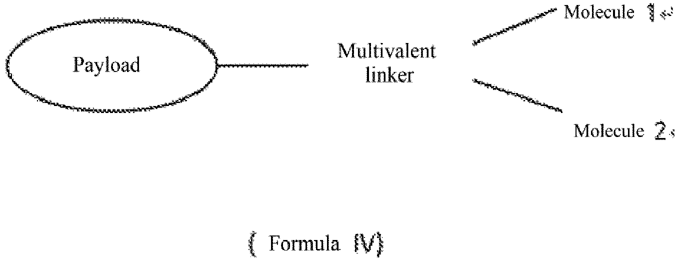

In some embodiments, the conjugate compound or the pharmaceutically acceptable salt thereof provided in the present application comprises at least one (for example, 1, 2, 3, 4, 5, 6, 7, 8, 9, 10 or more) payload as provided in the present application, two targeting molecules as provided in the present application and optionally a linker or spacer as provided in the present application. In some embodiments, the conjugate compound or the pharmaceutically acceptable salt thereof provided in the present application comprises one payload as provided in the present application, one ligand that specifically binds to a cell surface protein or marker as provided in the present application, one synergistic molecule as provided in the present application, and a linker or spacer as provided in the present application.

In some embodiments, the conjugate compound has a structure of formula V, VI, VII, or VIII shown as follows, wherein n, m, p, q and s are independently 0 or 1, which represent that the linker, multivalent linker and spacer are present or absent independently.

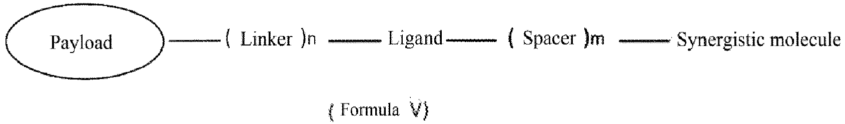

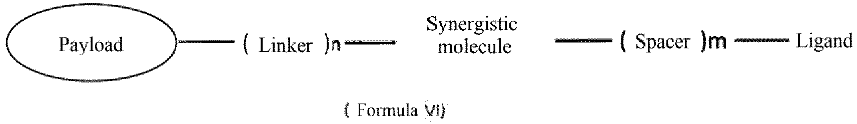

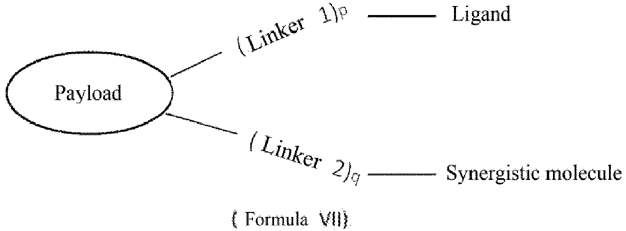

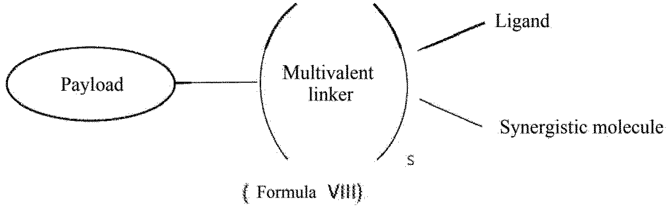

In some embodiments, the conjugate compound or the pharmaceutically acceptable salt thereof provided in the present application comprises a payload and two targeting molecules, wherein the two targeting molecules are a synergistic molecule moiety and a prostate-specific membrane antigen ligand moiety, respectively, for example, CB-20B, CB-20BK, CB-60S, CB-60SK, CB-20C, CB-1020, CB-1320, CB-1820, CR19428, 20R-SM09 and CB-20R.

In some embodiments, the conjugate compound or the pharmaceutically acceptable salt thereof provided in the present application comprises one or more payloads and two targeting molecules, wherein the two targeting molecules are a synergistic molecule moiety and a ligand moiety represented by formula (I), respectively, for example, CB-18G, CB-1820 and CR19426.

In some embodiments, the conjugate compound or the pharmaceutically acceptable salt thereof provided in the present application comprises one payload and two targeting molecules, wherein the two targeting molecules are a synergistic molecule moiety and P10, respectively, and the payload is camptothecin or any derivative thereof, such as CB-10S, CR19425 and CB-50S.

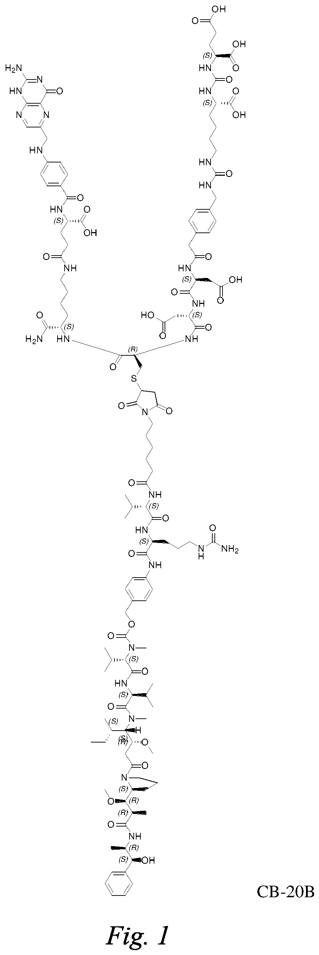

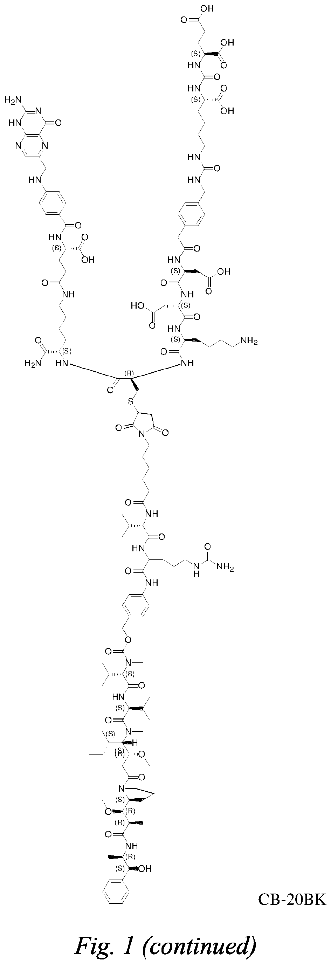

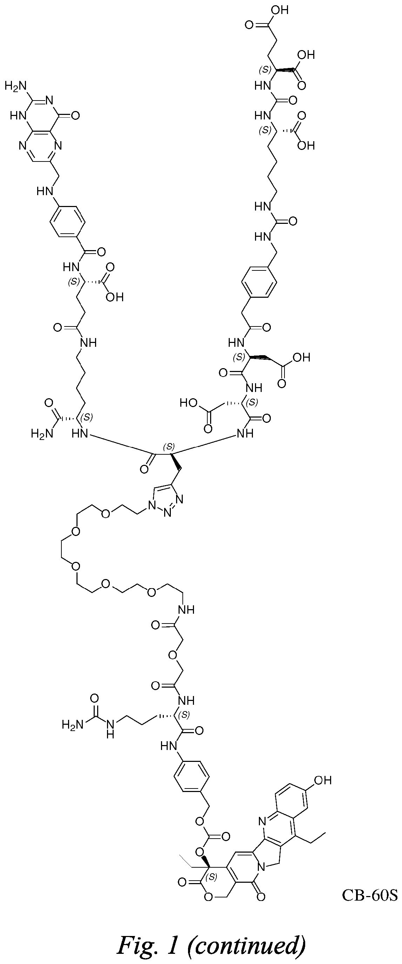

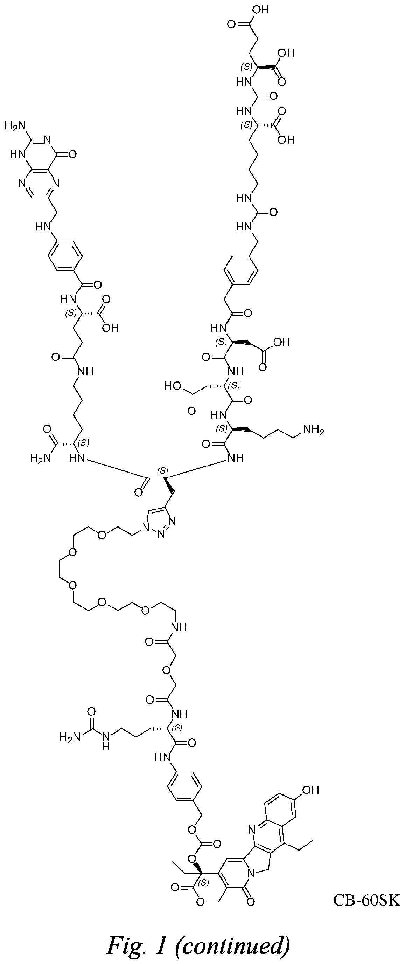

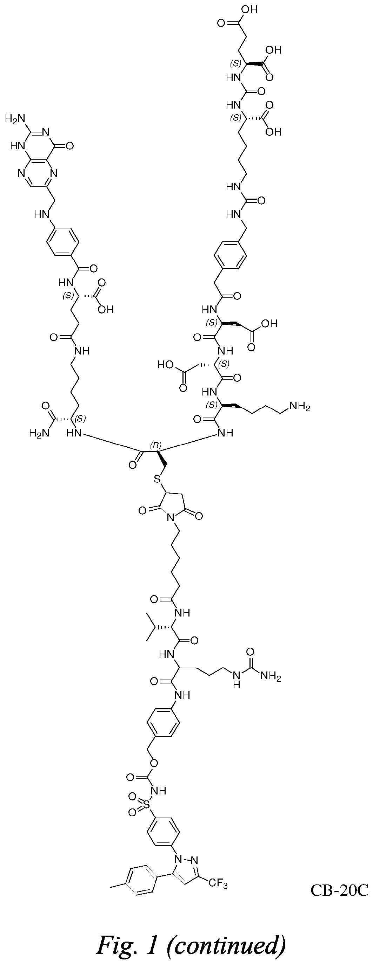

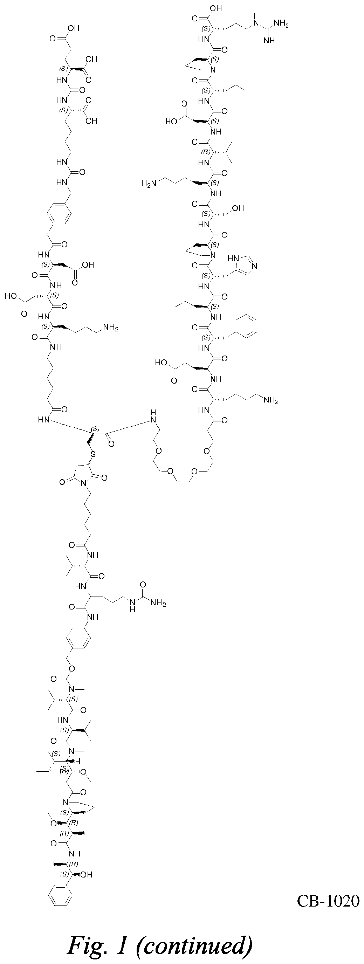

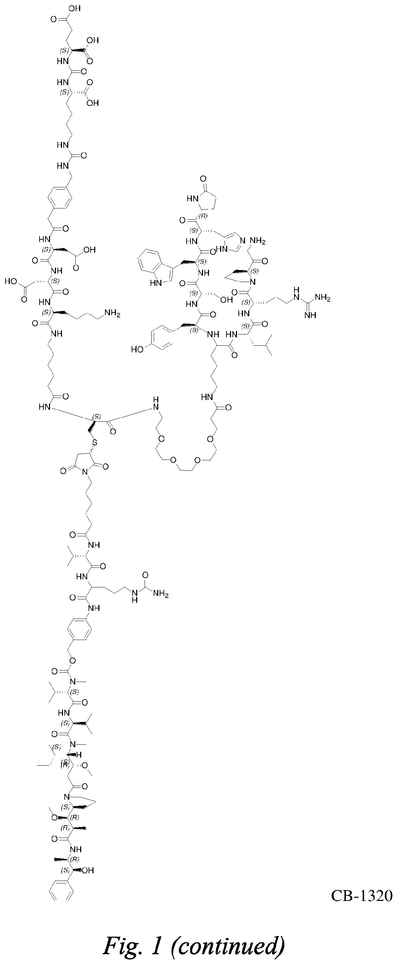

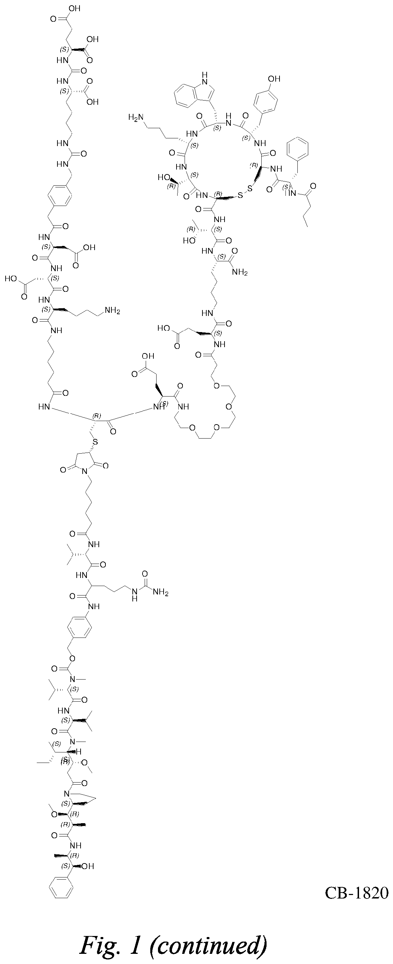

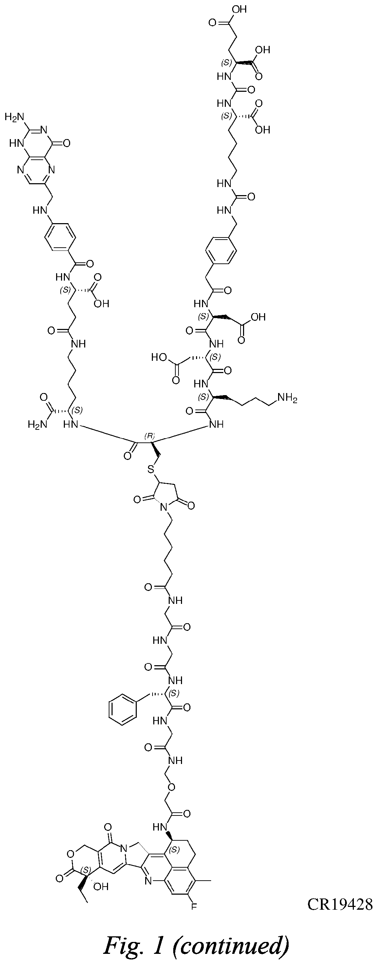

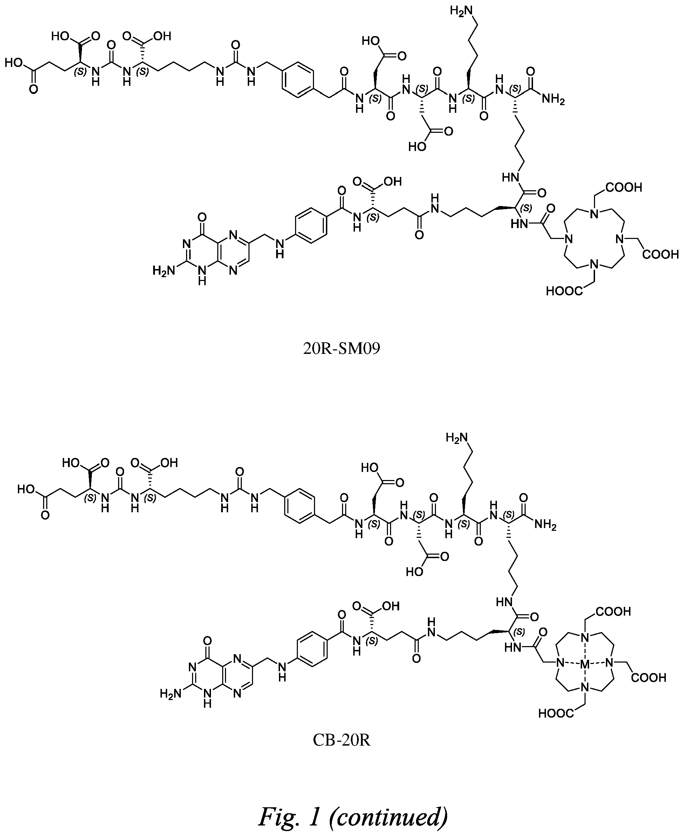

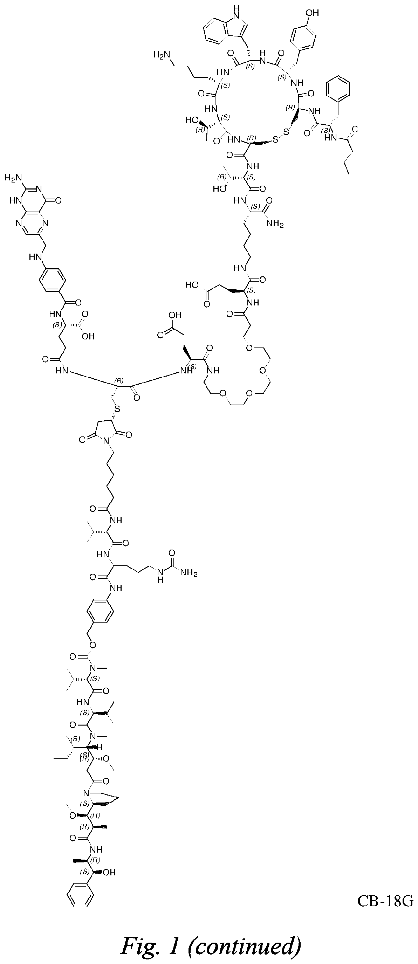

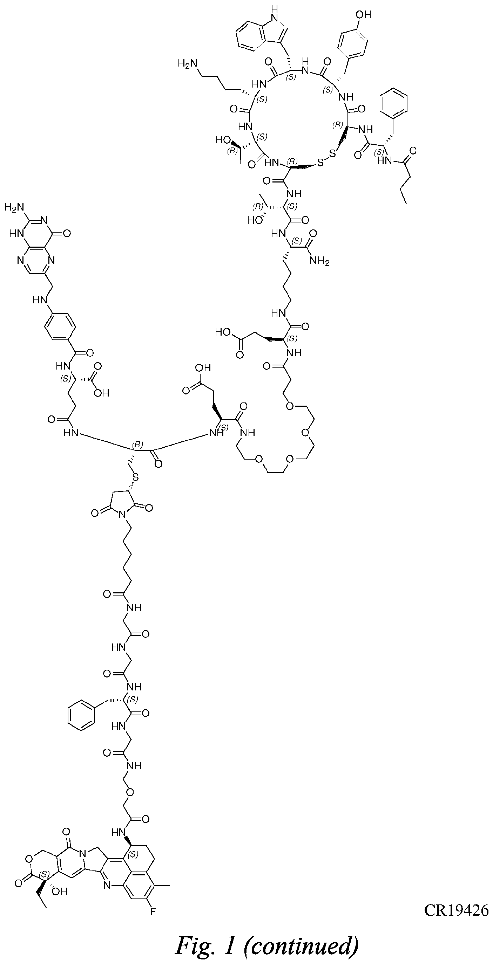

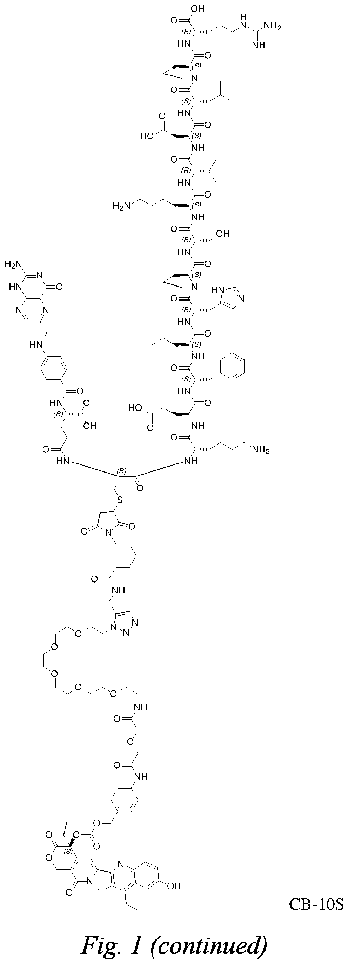

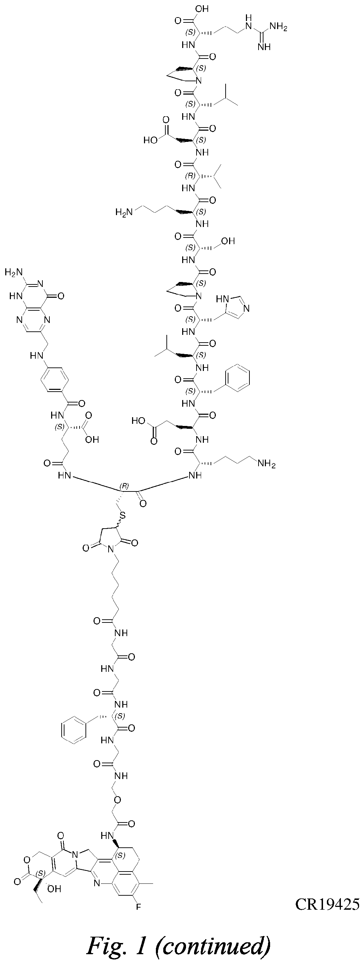

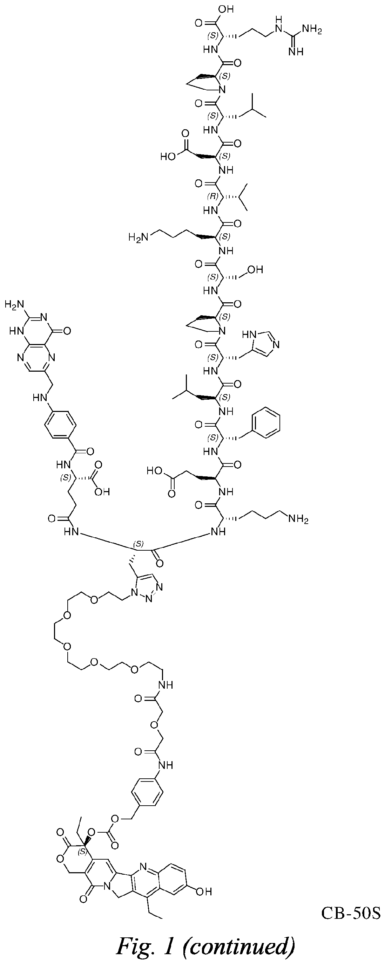

In some embodiments, the conjugate compound of the present application is selected from the group consisting of the following compounds: CB-20B, CB-20BK, CB-60S, CB-60SK, CB-20C, CB-1020, CB-1320, CB-1820, CR19428, 20R-SM09, CB-20R, CB-18G, CR19426, CB-10S, CR19425 and CB-50S (the specific structure of each conjugate compound is shown in Fig. 1). In some embodiments, the conjugate compound of the present application is formed by linking a linker-drug moiety to a ligand moiety via a covalent bond. The linker-drug moiety of the present application comprises a payload and a linker, and the ligand moiety of the present application comprises two targeting molecules and an optional spacer or linker, wherein the two moieties form the conjugate compound of the present application by reacting and forming a covalent bond, and the covalent bond can be formed between the linker in the linker-drug moiety and the ligand molecule in the ligand moiety, or can be formed between the linker in the linker-drug moiety and the spacer or linker in the ligand moiety.

The conjugate compound of the present application, CB-20B, is formed by linking a linker-drug moiety LT1002 to a ligand moiety 20B-SM09 via a covalent bond. The conjugate compound of the present application, CB-20BK, is formed by linking a linker-drug moiety LT1002 to a ligand moiety 20BK-SM09 via a covalent bond. The conjugate compound of the present application, CB-60S, is formed by linking a linker-drug moiety LT2000C to a ligand moiety 60S-SM09 via a covalent bond. The conjugate compound of the present application, CB-60SK, is formed by linking a linker-drug moiety LT2000C to a ligand moiety 60SK-SM09 via a covalent bond. The conjugate compound of the present application, CB-20C, is formed by linking a linker-drug moiety LD1001 to a ligand moiety 20BK-SM09 via a covalent bond. The conjugate compound of the present application, CB-1020, is formed by linking a linker-drug moiety LT1002 to a ligand moiety 1020BK-SM09 via a covalent bond. The conjugate compound of the present application, CB-1320, is formed by linking a linker-drug moiety LT1002 to a ligand moiety 1320BK-SM09 via a covalent bond. The conjugate compound of the present application, CB-1820, is formed by linking a linker-drug moiety LT1002 to a ligand moiety 1820BK-SM09 via a covalent bond. The conjugate compound of the present application, CR19428, is formed by linking a linker-drug moiety CR19423 to a ligand moiety 20BK-SM09 via a covalent bond. The conjugate compound of the present application. CB-20R. is formed by complexing 20R-SM09 with a radionuclide ion M. The conjugate compound of the present application, CB-18G, is formed by linking a linker-drug moiety LT1002 to a ligand moiety 18G-SM09 via a covalent bond. The conjugate compound of the present application, CR19426, is formed by linking a linker-drug moiety CR19423 to a ligand moiety 18G-SM09 via a covalent bond. The conjugate compound of the present application, CB-10S, is formed by linking a linker-drug moiety LT1000 to a ligand moiety CBSM09 via a covalent bond. The conjugate compound of the present application, CR19425, is formed by linking a linker-drug moiety CR19423 to a ligand moiety CBSM09 via a covalent bond. The conjugate compound of the present application, CB-50S, is formed by linking a linker-drug moiety LT1000N3 to a ligand moiety 50S-SM09 via a covalent bond. Each structure is shown in Table 3 below.

**Table 3: Structures of Linker-drug Moiety and Ligand Moiety**

| **Abbreviation** | **Structure** |
|---|---|
| LD1001 | 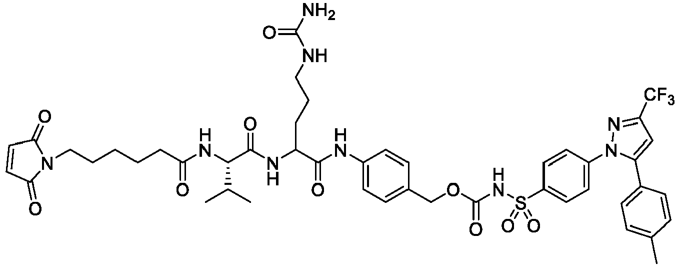 |
| LT1000 | 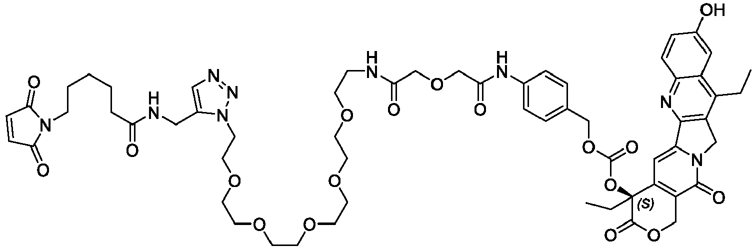 |
| LT1000N3 | 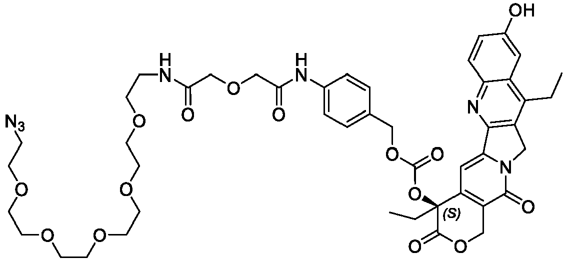 |
| LT1002 | 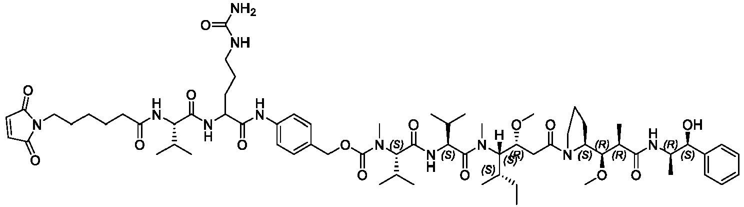 |
| LT2000C | 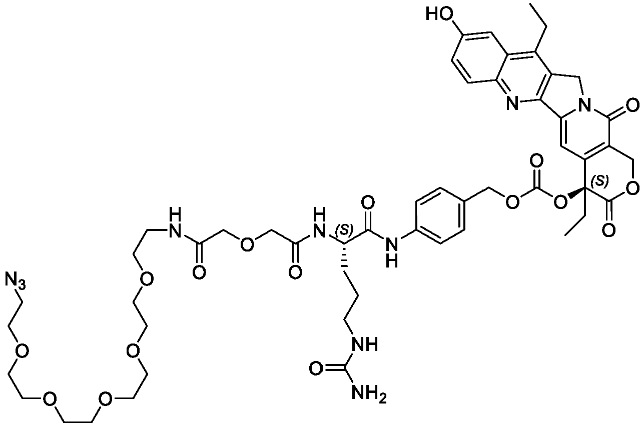 |
| CBSM09 | 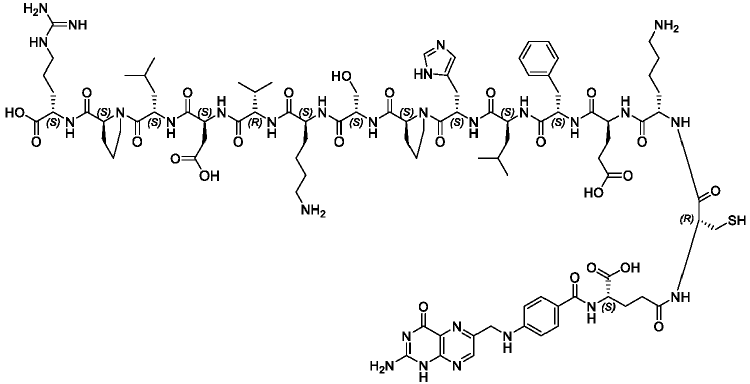 |
| 18G-SM09 | 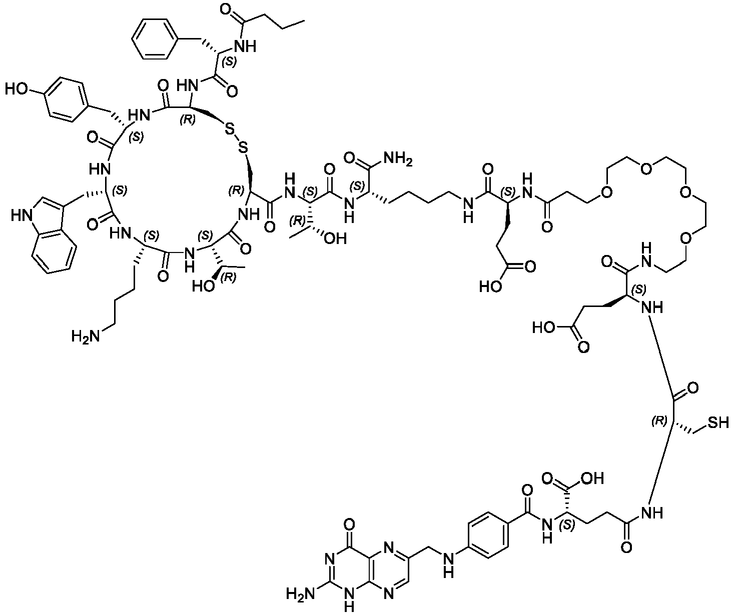 |
| 20B-SM09 | 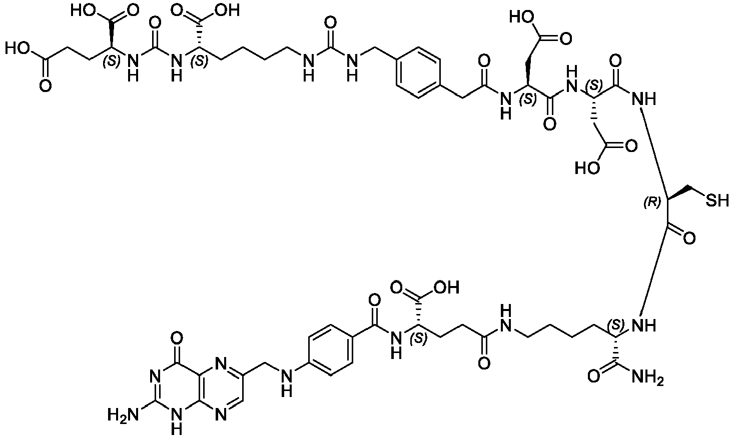 |
| 20BK-SM09 | 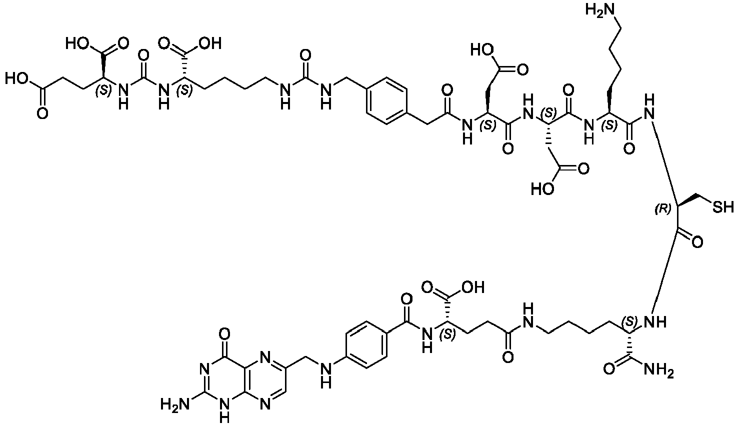 |
| 20R-SM09 | 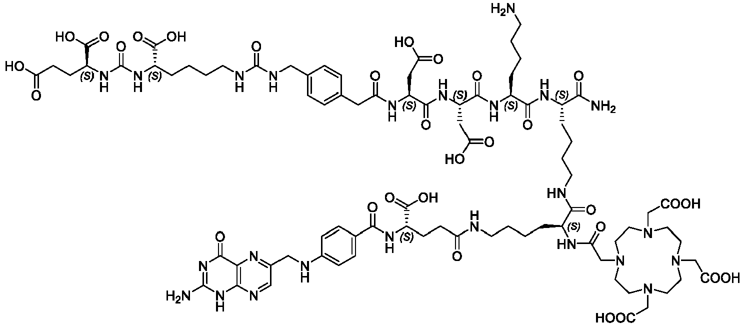 |
| 50S-SM09 | 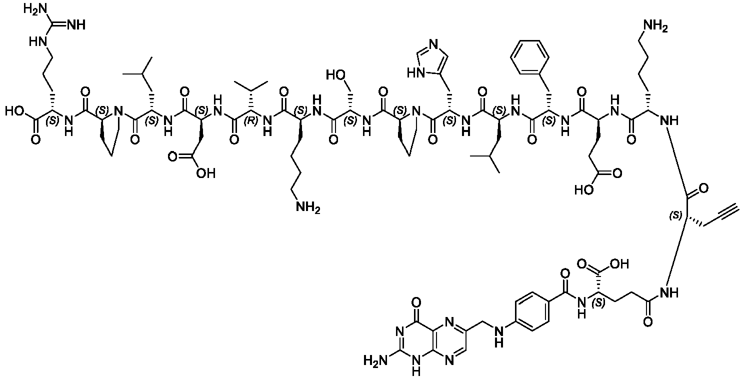 |
| 60S-SM09 | 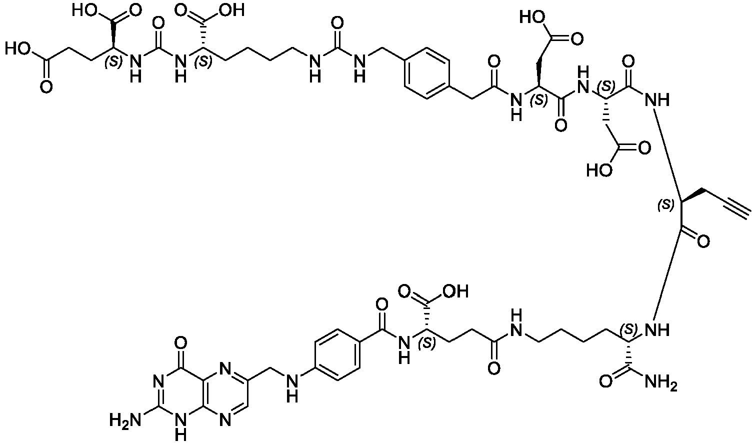 |
| 60SK-SM09 | 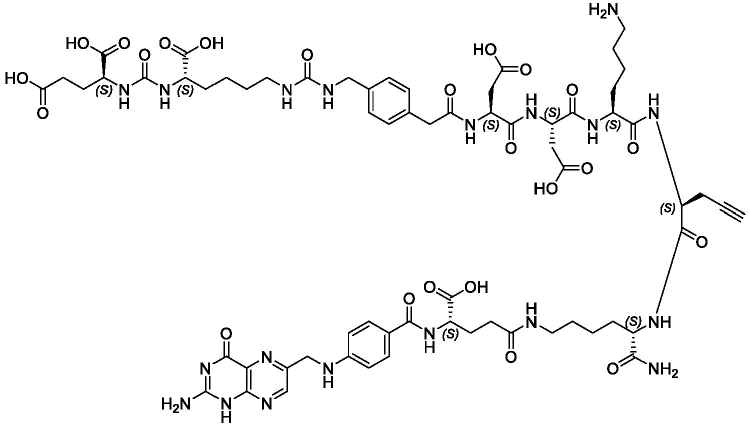 |
| 1020BK-SM09 | 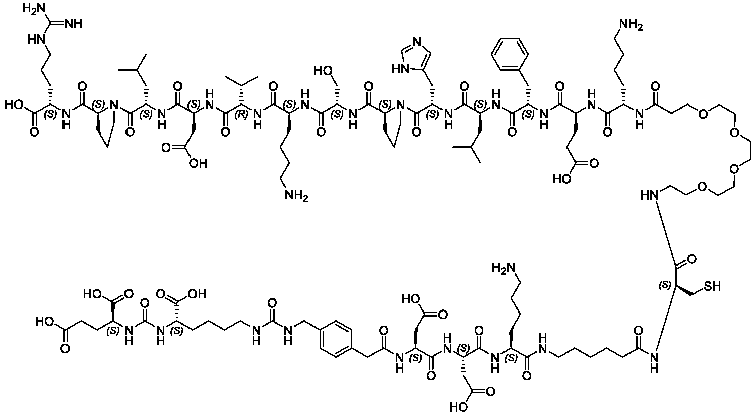 |
| 1320BK-SM09 | 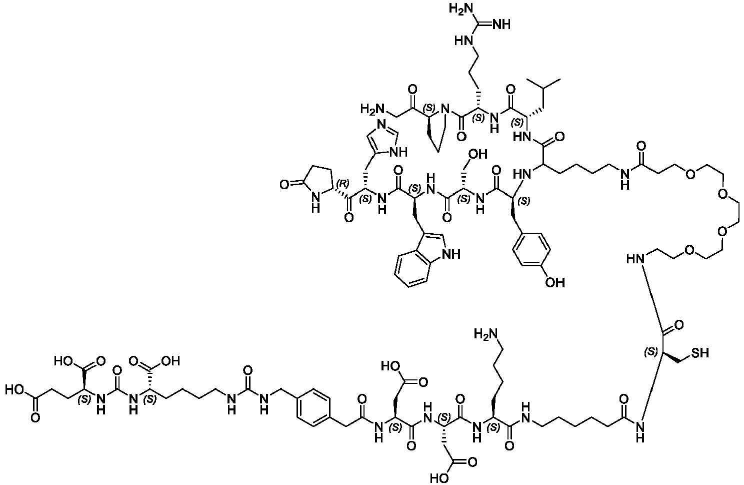 |
| 1820BK-SM09 | 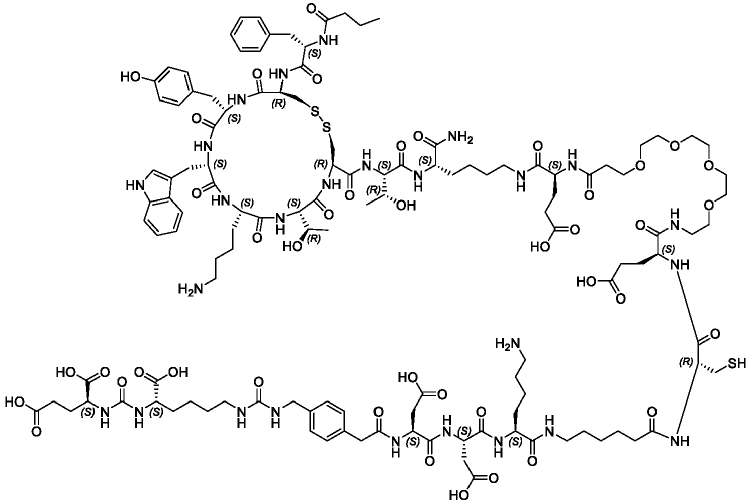 |
| CR19423 | 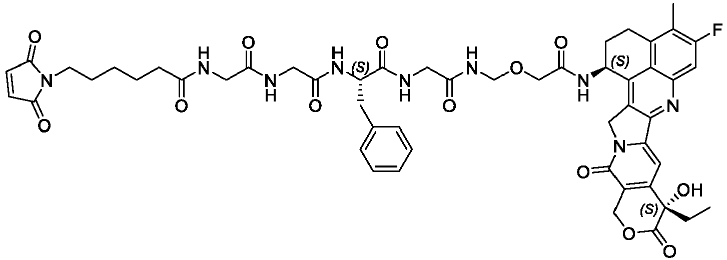 |

In some embodiments, the conjugate compound or the pharmaceutically acceptable salt thereof provided in the present application enters the blood circulation and the outside of cells (in an intercellular substance). Since the linker is very stable in an extracellular environment and drug molecules cannot be released, the toxicity of the drug molecules is blocked. The conjugate is a drug with no cytotoxicity or with a low toxicity and will not have a toxic effect on normal cells.

In some embodiments, the conjugate compound or the pharmaceutically acceptable salt thereof provided in the present application binds to multiple receptors or antigens and other acting molecules that are simultaneously highly expressed on diseased cells, and the synergistic effect thereof greatly increases the affinity of the conjugate compound to target cells, reducing the possibility of binding to normal cells. Therefore, a highly effective toxin drug such as MMAE/Dxd/SN38/a radionuclide complex can be carried to enhance the efficacy of drugs, broaden a therapeutic window and avoid drug side effects.

In some embodiments, the conjugate compound or the pharmaceutically acceptable salt thereof provided in the present application enters the interior of targeted cells, and then the linker can be cleaved through changes of the internal environment of the cells (by a specific enzyme digestion, a pH change, a disulphide bond reduction, etc.) to release drug molecules (equivalent to removing modifying groups of drug molecules), which has a therapeutic effect on tumour cells.

In some embodiments, the conjugate compound or the pharmaceutically acceptable salt thereof of the present application can be used to specifically deliver a payload to target cells in a target tissue environment. Generally, the two targeting molecules of the conjugate compound or the pharmaceutically acceptable salt thereof have three advantages. Firstly, the two targeting molecules can act in multiple modes (often synergistically), resulting in improved therapeutic effects while reducing side effects. Secondly, the binding of the two targeting molecules increases the affinity and avidity of the conjugate compound or the pharmaceutically acceptable salt thereof to target receptors or target cells, thereby enhancing its specificity and avoiding off-target toxicity. Finally, when properly designed, a combination of the two targeting molecules can fulfil multi-functional properties required for a drug conjugate.

The conjugate compound or the pharmaceutically acceptable salt thereof of the present application achieves unexpected technical effects, including but not limited to: (1) a combination of a ligand capable of binding to cell surface receptors and a endocytosis molecule capable of mediating endocytosis enable the conjugate compound to specifically enter target cells; (2) the conjugate compound or the pharmaceutically acceptable salt thereof enhances an affinity and targeting specificity of drug compounds, thereby delivering highly effective chemotherapeutic agents such as MMAE to a patient, broadening the therapeutic window of such agents and avoiding side effects; (3) a linker can prevent the release of a payload outside of target cells (for example, in a blood circulation system, intercellular substance, etc.), which ensures the stability of the conjugate compound during the blood circulation, and reduces the toxicity of the drug; after entering target cells, the linker is cleaved to release the payload and exert the effect of the drug, and meanwhile multiple drug resistance (MDR) can be avoided; and (4) a wide variety of drugs may be delivered in a form of the conjugate compound of the present application, and therefore, the application scopes of relevant drugs are widened. Therefore, the conjugate compound or the pharmaceutically acceptable salt thereof of the present application not only broadens the targeting scope and therapeutic window of LDC-based drugs, but also reduces toxicity and side effects of some drugs.

The terms "polypeptide", "protein" and "peptide" as used herein can be a single amino acid or a polymer of amino acids. The polypeptide, protein or peptide as described in the present application may contain naturally-occurring amino acids and non-naturally-occurring amino acids, or analogues and mimetics thereof. The polypeptide, protein or peptide can be obtained by any method well known in the art, for example, but not limited to, by an isolation and a purification from natural materials, a recombinant expression, a chemical synthesis, etc.

In another aspect, the present application discloses a pharmaceutical composition comprising the conjugate compound or the pharmaceutically acceptable salt thereof provided in the present application, and a pharmaceutically acceptable carrier.

The term "pharmaceutically acceptable" as used herein means, within the scope of sound medical judgment, being suitable for contact with cells of human beings and other animals without undue toxicity, irritation, allergic response, etc., and being commensurate with a reasonable benefit/risk ratio.

The term "pharmaceutically acceptable salt" as used herein refers to a relatively non-toxic, inorganic and organic acid addition salt and base addition salt, of the conjugate compound of the present application. Representative acid addition salts include hydrobromides, hydrochlorides, sulphates, bisulphates, phosphates, nitrates, acetates, oxalates, valerates, oleates, palmitates, stearates, laurates, borates, benzoates, lactates, phosphates, tosylates, citrates, maleates, fumarates, succinates, tartrates, naphthylates, mesylates, glucoheptonates, lactiobionates, sulphamates, malonates, salicylates, propionates, methylene-bis-*b*-hydroxynaphthoates, gentisates, isethionates, di-p-toluoyltartrates, methanesulphonates, ethanesulphonates, benzenesulphonates, p-toluenesulphonates, cyclohexylsulphamates, quinateslaurylsulphonate salts, and the like. Base addition salts include pharmaceutically acceptable metal and amine salts. Suitable metal salts include sodium, potassium, calcium, barium, zinc, magnesium, and aluminium salts. In some embodiments, sodium and potassium salts are preferred. Suitable inorganic base addition salts are prepared from metal bases which include, for example, sodium hydride, sodium hydroxide, potassium hydroxide, calcium hydroxide, aluminium hydroxide, lithium hydroxide, magnesium hydroxide, and zinc hydroxide. Suitable amine base addition salts are prepared from amines which have sufficient basicity to form a stable salt, and preferably include the following amines which are frequently used in medicinal chemistry because of their low toxicity and acceptability for medical use: ammonia, ethylenediamine, N-methyl-glucamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)-aminomethane, tetramethylammonium hydroxide, triethylamine, dibenzylamine, ephenamine, dehydroabietylamine, N-ethylpiperidine, benzylamine, tetramethylammonium, tetraethylammonium, methylamine, dimethylamine, trimethylamine, ethylamine, basic amino acids, e.g., lysine and arginine, dicyclohexylamine, and the like.

The term "pharmaceutically acceptable carrier" as used herein refers to a pharmaceutically acceptable solvent, suspension or any other pharmacologically inert vehicle for delivering the conjugate compound provided in the present application to a subject, without interfering with the structures and properties of the conjugate compound. Such carriers enable the conjugate compound to be formulated as, for example, tablets, pills, capsules, liquids, gels, syrups, slurries, suspensions and pastilles, for oral ingestion by a subject. Such carriers enable the conjugate compound to be formulated as injections, infusions or preparations for local administration.

The pharmaceutically acceptable carriers for use in the pharmaceutical composition provided in the present application may include, but are not limited to, for example, pharmaceutically acceptable liquids, gels, or solid carriers, aqueous vehicles (such as sodium chloride injection, Ringer's injection, isotonic dextrose injection, sterile water injection, or dextrose and lactated Ringer's injection), nonaqueous vehicles (such as fixed oils derived from vegetables, cottonseed oil, corn oil, sesame oil, or peanut oil), antimicrobial agents, isotonic agents (such as sodium chloride or dextrose), buffers (such as phosphate or citrate buffers), antioxidants (such as sodium bisulphate), anaesthetics (such as procaine hydrochloride), suspensions/dispersions (such as sodium carboxymethylcellulose, hydroxypropyl methylcellulose, or polyvinylpyrrolidone), chelating agents (such as EDTA (ethylenediamine tetraacetic acid) or EGTA (ethylene glycol tetraacetic acid)), emulsifying agents (such as polysorbate 80 (Tween-80)), diluents, adjuvants, excipients or non-toxic auxiliary substances, other components well known in the art, or various combinations thereof. Suitable components may include, for example, fillers, binders, buffers, preservatives, lubricants, flavouring agents, thickening agents, colouring agents, or emulsifying agents.

In some embodiments, the pharmaceutical composition is an injection preparation. The injection preparations include sterile water solutions or dispersions, suspensions or emulsions. In all cases, the injection preparations should be sterile and be a fluid for easy injection. It should be stable under the conditions of manufacture and storage and must be preserved against the contaminating action of microorganisms such as bacteria and fungi. The carriers can be solvents or dispersion mediums containing, for example, water, ethanol, polyol (for example, glycerol, propylene glycol, liquid polyethylene glycol, and the like), and suitable mixtures thereof and/or vegetable oils. The injection preparations should maintain appropriate fluidity. The appropriate fluidity can be maintained, for example, by the use of coatings such as lecithin, by the use of surfactants, and the like. Prevention of the action of microorganisms can be achieved by various antibacterial and antifungal agents, for example, parabens, chlorobutanol, phenol, sorbic acid, thimerosal, and the like.

In some embodiments, the pharmaceutical composition is an oral preparation. The oral preparations include, but are not limited to, capsules, cachets, pills, tablets, lozenges (using a flavoured basis, usually sucrose and acacia or tragacanth), powders, granules, or as solutions or suspensions in aqueous or non-aqueous liquids, or as oil-in-water or water-in-oil liquid emulsions, or as elixirs or syrups, or as pastilles (using an insert base, such as gelatin and glycerine, or sucrose and acacia) and/or as mouth washes.

In solid dosage forms for oral administration (e.g., capsules, tablets, pills, dragees, pulvis, granules and the like), the conjugate compound is mixed with one or more pharmaceutically acceptable carriers, such as sodium citrate or dicalcium phosphate, and/or any of the followings: (1) fillers or extenders, such as starches, lactose, sucrose, glucose, mannitol, and/or silicic acid; (2) binders, such as carboxymethylcellulose, alginates, gelatins, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as acetyl alcohol and glycerol monostearate; (8) absorbents, such as kaolin and bentonite clay; (9) lubricants, such as talc, calcium stearate, magnesium stearate, solid polyethylene glycol, sodium lauryl sulphate, and mixtures thereof; and (10) colouring agents.

In liquid dosage forms for oral administration, the conjugate compound is mixed with any of the followings: pharmaceutically acceptable emulsions, microemulsions, solutions, suspensions, syrups and elixirs. In addition to the conjugate compound, the liquid dosage forms may contain inert diluents commonly used in the art, such as, water or other solvents, solubilizing agents and emulsifying agents, such as ethyl alcohol, isopropyl alcohol, ethyl carbonate, ethyl acetate, benzyl alcohol, benzyl benzoate, isopropanol, 1,3-butylene glycol, oils (in particular, cottonseed oil, peanut oil, corn oil, olive oil, castor oil and sesame oil), glycerol, tetrahydrofurfuryl alcohol, polyethylene glycol and fatty acid esters of sorbitan, and mixtures thereof. Besides inert diluents, an oral composition can also include adjuvants such as wetting agents, emulsifying agents and suspensions, sweetening agents, flavouring agents, colouring agents, perfuming agents and preservatives.

In some embodiments, the pharmaceutical composition is a mouth spray preparation or a nasal spray preparation. The spray preparations include, but are not limited to, aqueous aerosols, nonaqueous suspensions, lipidosome preparations or solid granular preparations. Aqueous aerosols are prepared by mixing aqueous solutions or suspensions of agents with conventional pharmaceutically acceptable carriers and stabilizers. The carriers and stabilizers vary according to the requirements of specific compounds, but in general, they include nonionic surfactants (Tweens or polyethylene glycol), oleic acid, lecithin, amino acids such as glycine, buffer solution, salts, sugar or sugar alcohol. Aerosols are generally prepared from isotonic solutions, and can be delivered by sprayers.

In some embodiments, the pharmaceutical composition can be used by mixing with one or more other drugs. In some embodiments, the pharmaceutical composition comprises at least one other drug. In some embodiments, the other drugs are antineoplastic drugs, cardiovascular drugs, anti-inflammatory drugs, antiviral drugs, digestive system drugs, nervous system drugs, respiratory system drugs, immune system drugs, dermatologic drugs, metabolic drugs, and the like.

In some embodiments, the pharmaceutical compositions can be administered to a subject in need thereof by appropriate routes, including without limitation, oral, injection (such as intravenous, intramuscular, subcutaneous, intracutaneous, intracardiac, intrathecal, intrapleural and intraperitoneal injection), mucosal (such as nasal and intraoral administration), sublingual, rectal, percutaneous, intraocular, and pulmonary administration. In some embodiments, the pharmaceutical composition can be administered intravenously, subcutaneously, orally, intramuscularly or intraventricularly.

Due to the properties of some payloads, such as high toxicity and high hydrophilicity, it is desired to deliver the payloads more specifically and more efficiently to a subject in need thereof. For example, in cancer treatment, it is desired to specifically deliver chemotherapeutic agents to cancer cells without toxicity to normal cells. Therefore, in another aspect, the present application discloses a method for delivering a payload to a subject in need thereof, comprising administering to the subject a therapeutically effective amount of the conjugate compound or the pharmaceutically acceptable salt thereof provided in the present application, or the pharmaceutical composition provided in the present application. The payloads described in the present application may be any pharmaceutical agent that elicits biological or medicinal responses in a tissue, system, animal, individual or human that is being sought by a researcher, veterinarian, medical doctor or other clinicians in preventing, inhibiting, ameliorating or treating a disease.

The term "object" as used herein refers to human and non-human animals. Non-human animals include all vertebrates, for example, mammals and non-mammals. The subject may also be a livestock animal such as cattle, swine, sheep, poultry and horse, or a domestic animal such as dog and cat. The subject may be male (e.g. man) or female (e.g. woman), may be elderly, and may be an adult, adolescent, child, or infant. A human subject may be Caucasian, African, Asian, Semitic, or human with other racial backgrounds or a mixture of such racial backgrounds.

The term "therapeutically effective amount" as used herein refers to an amount of the conjugate compound or the pharmaceutically acceptable salt thereof, or the pharmaceutical composition which relieves to some extent one or more symptoms of a disease or disorder in a subject, returns to normal either partially or completely one or more physiological or biochemical parameters associated with or causative of the disease or disorder, and/or reduces the likelihood of the onset of the disease or disorder. Such amounts generally vary according to a number of factors which can be determined and explained, according to the scope of the specification provided in the present application, by those of ordinary skill in the art. These factors include, without limitation: the particular subject and the age, weight, height, general physical condition, and medical history thereof, the particular compound used, the carrier of the preparation and the administration route selected, and the nature and severity of the condition being treated.

In some embodiments, the amount of the conjugate compound, or the pharmaceutically acceptable salt thereof, or the pharmaceutical composition is sufficient to inhibit a disease or disorder in a subject, or prophylactically inhibit or prevent the onset of a disease or disorder in a subject. Although the therapeutically effective amount may vary in different subjects, it is generally ranged from 0.01 to 100 mg/kg, for example, 0.01 to 90 mg/kg, 0.01 to 80 mg/kg, 0.01 to 70 mg/kg, 0.01 to 60 mg/kg, 0.01 to 50 mg/kg, 0.01 to 40 mg/kg, 0.01 to 30 mg/kg, 0.01 to 20 mg/kg, 0.01 to 10 mg/kg, 0.01 to 5 mg/kg, 0.01 to 4 mg/kg, 0.01 to 3 mg/kg, 0.01 to 2 mg/kg, 0.01 to 1 mg/kg, and 0.01 to 0.1 mg/kg. The therapeutically effective amount as described in the present application can be equal to any value within the above numerical range, including the end values of this range.

In another aspect, the present application discloses a method for delivering a payload to a subject in need thereof, comprising administering to the subject a therapeutically effective amount of the conjugate compound or the pharmaceutically acceptable salt thereof provided in the present application, or the pharmaceutical composition provided in the present application.

In another aspect, the present application discloses a method for treating a disease in a subject, comprising administering to the subject a therapeutically effective amount of the conjugate compound or the pharmaceutically acceptable salt thereof provided in the present application, or the pharmaceutical composition provided in the present application.

In some embodiments, the disease is a cancer, including but not limited to, prostatic cancer, breast cancer, lung cancer, renal cancer, leukaemia, ovarian cancer, gastric cancer, uterine cancer, endometrial carcinoma, liver cancer, thyroid cancer, pancreatic cancer, colon cancer, colorectal cancer, oesophageal cancer, skin cancer, lymphoma, and multiple myeloma.

In some embodiments, cancer cells of the cancers have an expression of the cell surface receptors or antigens mentioned in the present application. In some embodiments, cancer cells of the cancers have a high expression (for example, according to data from Depmap (see: https://depmap.org/portal/), the corresponding gene expression is at least 0, 0.01, 0.05, 0.1, 0.5, 1, 2, 3, 4, 5, 6, 7, 8, 9, 10 or more than 10) of cell surface receptors or antigens mentioned in the present application. In some embodiments, cancer cells of the cancers have a high expression of FOLR1 and FOLH1, TRPV6 and FOLH1, GNRHR and FOLH1, SSTR2 and FOLH1, FOLR1 and SSTR2, or TRPV6 and FOLR1. In some embodiments, the disease is an immunological disease, for example, an autoimmune disease, including but not limited to, connective tissue disease, systemic sclerosis, rheumatoid arthritis, and systemic lupus erythematosus.

In some embodiments, the disease is a cardiovascular disease, including but not limited to, angina, myocardial infarction, stroke, heart attack, hypertensive heart disease, rheumatic heart disease, cardiomyopathy, arrhythmia, and congenital heart disease.

In some embodiments, the disease is a metabolic disease, including but not limited to, diabetes, gout, obesity, hypoglycaemia, hyperglycaemia, and dyslipidaemia.

In some embodiments, the disease is a neurological disease, including but not limited to, Alzheimer's disease, Parkinson's disease, Huntington's disease, head injury, multiple sclerosis, vertigo, coma, and epilepsy.

In some embodiments, the method provided in the present application further comprises administering one or more therapeutic agents in combination with the conjugate compound or the pharmaceutically acceptable salt thereof, or the pharmaceutical composition. In some embodiments, the therapeutic agents target an anti-cancer therapeutic target, induce or boost an immune response against cancer, or are chemotherapeutic agents.

The present application will be described in greater detail by way of specific examples. The following examples are offered for illustrative purposes only, and are not intended to limit the invention in any manner. A person skilled in the art will readily recognize a variety of noncritical parameters which can be changed or modified to yield essentially the same results.

### EXAMPLES

The following examples are intended to further illustrate the present application. The advantages and features of the present application will become clear with the descriptions. However, these illustrations are merely exemplary, and should not be constructed as limitations to the scope of the present application.

### Example 1: Preparation of Conjugate Compounds

### Synthesis of conjugate compounds CB-20BK, CB-18G, CB-20B, CB-10S, CB-20C, FA-MMAE, CB-20AK, CB-1020, CB-1320 and CB-1820

1. 10 g of Rink amide-am resin (hereafter referred to as "Rink Resin", from Sunresin New Materials Co. Ltd., Cat#: 183599-10-2) with a degree of substitution of 0.45 mmol/g was weighed and loaded onto a solid phase reaction column; DCM was added, and nitrogen gas was bubbled through the solvent to swell the resin for 30 minutes; the solvent was pumped off; and Fmoc protecting groups on the resin were removed by DBLK, and then the resin was washed 5 times with DMF. 4.79 g (9 mmol) of Fmoc-Lys(Dde)-OH and 1.47 g (10.8 mmol) of HOBt were weighed and dissolved in DMF. To the above-mentioned solution at 0°C in an ice-water bath, 1.67 ml (10.8 mmol) of DIC was added and mixed to activate for 5 minutes. The solution was added to the above-mentioned reaction column and reacted for 3 hours, and then the solvent was pumped off; and the resin in the reaction column was washed 3 times. Then Fmoc protecting groups were removed by DBLK.
2. The above-mentioned operations were repeated, and Fmoc-Cys(Trt)-OH, Fmoc-Lys(Boc)-OH, Fmoc-Asp(OtBu)-OH, Fmoc-Asp(OtBu)-OH and intermediate 108 were successively conjugated according to the structures. 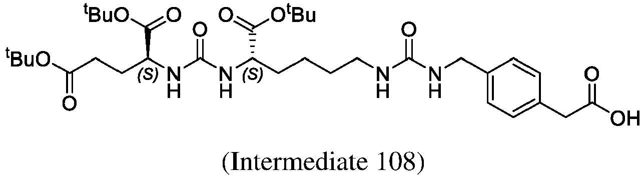
3. Dde protecting groups were removed twice with 2% hydrazine hydrate/DMF, for 10 minutes each time, and then the resin was washed 5 times with DMF. Fmoc-Glu-OtBu and pteroic acid were conjugated successively. Finally, the resin was condensed twice with methanol, and the solvent was pumped off to obtain 17.4 g of a protected peptide resin.
4. 17.4 g of the peptide resin obtained in the previous step was added to a 250 ml single-necked flask; 139 ml of lysis buffer (TFA : H₂O : TIS = 95 : 3 : 2 (volume ratio)) was previously prepared, and 2.1 g of DTT was weighed and added to the lysis buffer. The lysis buffer was added to the above-mentioned flask, and the mixture was reacted at room temperature for 2.5 hours and filtered; the resin was continued to be washed with 30 ml of TFA; the above-mentioned filtrate was combined, and the mixture was added to 834 ml of absolute ether, with a yellow solid being precipitated, and centrifuged to obtain a solid, which was washed with absolute ether and dried in vacuo to obtain 6.4 g of a crude product as a yellow solid and with a yield of 93.4% and an HPLC purity of 82.3%. The product was separated by prep-HPLC (condition: C18 column, mobile phase A: 0.1% trifluoroacetic acid in water, B: acetonitrile, elution gradient: (15-25)%B, elution time: 60 minutes; fractions were collected); and the fraction containing the qualified product was lyophilized to obtain 4.73 g of 20BK-SM09, with a purity of 98.8%.
5. 4.09 g (3.11 mmol) of Mc-Val-Cit-PAB-MMAE (LT1002) was weighed and added to a 1000 ml single-necked flask, and 500 ml of phosphate buffer and 100 ml of acetonitrile were added; the solution was stirred and maintained at pH = 7.2 until a clear solution was obtained; and 4.73 g (3.11 mmol) of intermediate 20BK-SM09 was added, and the mixture was reacted at room temperature for 2 hours, during which the reaction was monitored by HPLC. After the reaction was completed, the mixture was filtered, and the filtrate was separated by prep-HPLC (condition: C18 column, mobile phase A: ammonium bicarbonate solution (pH = 7.2), B: acetonitrile, elution gradient: (25-35)%B, elution time: 60 minutes; fractions were collected); the fraction containing the qualified product was lyophilized to obtain 6.96 g of CB-20BK product, with a purity of 98.8% and a yield of 78.8%.

In the same way, conjugate compounds CB-18G, CB-20B, CB-10S, CB-20C, FA-MMAE (with a structure shown as follows), CB-20AK (with a structure shown as follows), CB-1020, CB-1320 and CB-1820 can be obtained through similar steps in the above-mentioned methods.

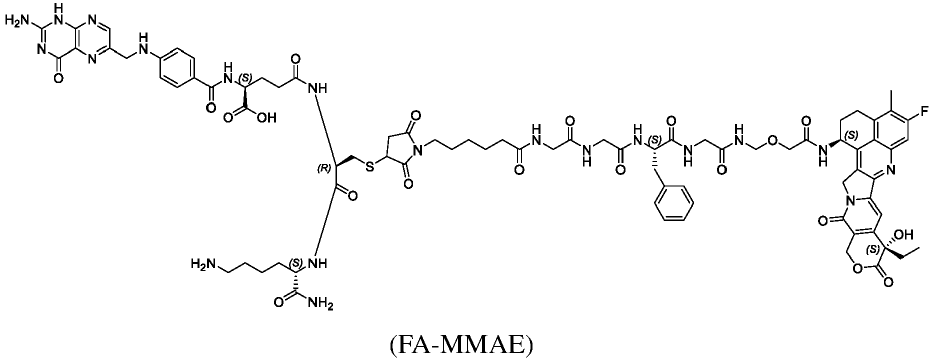

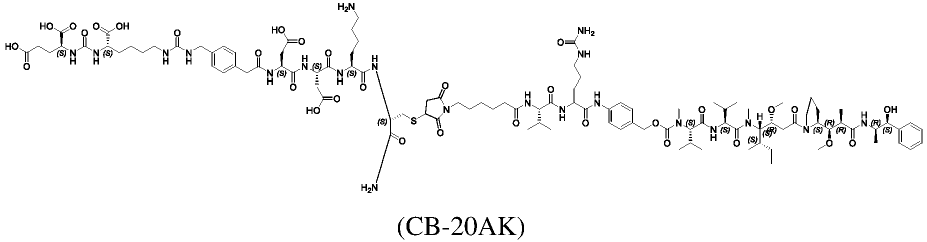

### Synthesis of conjugate compounds CB-50S, CB-60S and CB-60SK

1. 10 g of Wang Resin (from Sunresin New Materials Co. Ltd., Cat#: 1365700-43-1) with a degree of substitution of 1.1 mmol/g was weighed and loaded onto a solid phase reaction column; DMF was added, and nitrogen gas was bubbled through the solvent to swell the resin for 30 minutes; 14.3 g (22 mmol) of Fmoc-Arg(pbf)-OH, 3.56 g (26.4 mmol) of HOBt, and 0.27 g (2.2 mmol) of DMAP were weighed and dissolved in DMF; at 0°C in an ice-water bath, 4.1 ml (26.4 mmol) of DIC was added and mixed to activate for 5 minutes; the solution was added to the reaction column and reacted for 3 hours, and then the solvent was pumped off; and the resin was washed 3 times.
2. 10.4 ml of acetic oxide and 8.9 ml of pyridine were dissolved in 50 ml of DMF and mixed, and the resin after washing in the above steps was added; the mixture was blocked at room temperature for 5 hours and washed three times with DMF; and the resin was condensed with methanol, and then the solvent was pumped off to obtain Fmoc-Arg(pbf)-Wang Resin, which has a degree of substitution determined to be 0.53 mmol/g.
3. 3.8 g (2 mmol) of Fmoc-Arg(pbf)-Wang Resin (Sub = 0.53 mmol/g) was weighed and loaded onto a reaction column; the resin was washed 3 times with DMF and then was swelled for 30 minutes by means of adding DMF. Then Fmoc protecting groups were removed by DBLK, and the resin was washed 6 times with DMF. 2.0 g (6 mmol) of Fmoc-Pro-OH and 0.97 g (7.2 mmol) of HOBt were weighed and dissolved in DMF; at 0°C in an ice-water bath, 1.1 ml (7.2 mmol) of DIC was added and mixed to activate for 5 minutes; the mixture was added to the reaction column and reacted for 2 hours; and then Fmoc protecting groups were removed by DBLK.
4. The above-mentioned operations were repeated, and Fmoc-Leu-OH, Fmoc-Asp(OtBu)-OH, Fmoc-Val-OH, Fmoc-Lys(Boc)-OH, Fmoc-Ser(tBu)-OH, Fmoc-Pro-OH, Fmoc-His(Trt)-OH, Fmoc-Leu-OH, Fmoc-Phe-OH, Fmoc-Glu(OtBu)-OH, Fmoc-Lys(Boc)-OH, Fmoc-propargyl-Gly-OH, Fmoc-Glu-OtBu and pteroic acid were conjugated successively according to the structures. The resin was condensed twice with methanol, and the solvent was pumped off to obtain 8.4 g of a peptide resin.
5. 8.4 g of the peptide resin obtained in the previous step was added to a 250 ml single-necked flask; 67 ml of lysis buffer (TFA : H₂O : TIS = 95 : 3 : 2 (volume ratio)) was previously prepared, and 0.92 g of DTT was weighed and added to the lysis buffer. The lysis buffer was added to the flask, and the mixture was reacted at room temperature for 2.5 hours; the resin was filtered and washed with 20 ml of TFA; the filtrate was combined, and the mixture was added to 402 ml of absolute ether, with a yellow solid being precipitated, and centrifuged to obtain a solid, which was washed with absolute ether and dried in vacuo to obtain 4.06 g of a crude product as a yellow solid and with a yield of 97.3% and an HPLC purity of 84.6%. The product was separated by prep-HPLC (condition: C18 column, mobile phase A: 0.1% trifluoroacetic acid in water, B: acetonitrile, elution gradient: (20-29)%B, elution time: 60 minutes; fractions were collected); and the fraction containing the qualified product was lyophilized to obtain 2.86 g of 50S-SM09, with a purity of 97.6%.
6. 1.29 g (1.37 mmol) of LT1000N3 was weighed and added to a 500 ml single-necked flask, and 270 ml of a mixed solvent (CAN : H₂O = 1 : 4) and 393 mg (2.74 mmol) of CuBr were added and stirred. 2.86 g (1.37 mmol) of intermediate 50S-SM09 was added, and the mixture was reacted at room temperature for 2-3 hours, during which the reaction was monitored by HPLC. After the reaction was completed, the mixture was filtered, and the filtrate was separated by prep-HPLC (condition: C18 column, mobile phase A: 0.1% trifluoroacetic acid in water, B: acetonitrile, elution gradient: (22-40)%B, elution time: 60 minutes; fractions were collected); and the fraction containing the qualified product was lyophilized to obtain 3.17 g of CB-50S, with a purity of 98.6% and a yield of 76.4%.

In the same way, conjugate compounds CB-60S and CB-60SK can be obtained through similar steps in the above-mentioned methods.

### Synthesis of CB-20R

1. 5 g of Rink Resin with a degree of substitution of 0.45 mmol/g was weighed and loaded onto a solid phase reaction column; DCM was added, and nitrogen gas was bubbled through the solvent to swell the resin for 30 minutes; the solvent was pumped off; Fmoc protecting groups on the resin were removed by DBLK; and then the resin was washed 5 times with DMF. 2.4 g (4.5 mmol) of Fmoc-Lys(Dde)-OH and 0.74 g (5.4 mmol) of HOBt were weighed and dissolved in DMF; at 0°C in an ice-water bath, 0.84 ml (5.4 mmol) of DIC was added and mixed to activate for 5 minutes; the mixture was added to the reaction column and reacted for 3 hours; and the solvent was pumped off, and the resin was washed 3 times. Then Fmoc protecting groups were removed by DBLK.
2. The above-mentioned operations were repeated, and Fmoc-Cys(Trt)-OH, Fmoc-Lys(Boc)-OH, Fmoc-Asp(OtBu)-OH, Fmoc-Asp(OtBu)-OH and intermediate 108 were successively conjugated according to the structures.
3. Dde protecting groups were removed twice with 2% hydrazine hydrate/DMF, for 10 minutes each time, and then the resin was washed 5 times with DMF. DOTA-tris(tBu) ester was conjugated. Then Dde protecting groups were removed twice with 2% hydrazine hydrate/DMF, for 10 minutes each time, and then the resin was washed 5 times with DMF. Fmoc-Glu-OtBu and pteroic acid were conjugated successively, and finally, the resin was condensed twice with methanol and the solvent was pumped off to obtain 9.2 g of a protected peptide resin.
4. 9.2 g of the peptide resin obtained in the previous step was added to a 250 ml single-necked flask; 74 ml of lysis buffer (TFA : H₂O : TIS = 95 : 3 : 2 (volume ratio)) was previously prepared, and 1.05 g of DTT was weighed and added to the lysis buffer. The lysis buffer was added to the flask, and the mixture was reacted at room temperature for 2.5 hours; the resin was filtered and washed with 20 ml of TFA; the filtrate was combined, and the mixture was added to 560 ml of absolute ether, with a yellow solid being precipitated, and centrifuged to obtain a solid, which was washed with absolute ether and dried in vacuo to obtain 3.8 g of a crude product as a yellow solid and with a yield of 87.4% and an HPLC purity of 81.2%. The product was separated by prep-HPLC (condition: C18 column, mobile phase A: 0.1% trifluoroacetic acid in water, B: acetonitrile, elution gradient: (15-25)%B, elution time: 60 minutes; fractions were collected); and the fraction containing a synthetic product was lyophilized to obtain 2.9 g of 20R-SM09, with a purity of 97.8%.
5. 20R-SM09 was complexed with radionuclide ion M to obtain CB-20R. Specifically, the radioactive label ¹⁷⁷Lu (about 50 MBq) was mixed with 100 µl of 0.5 M sodium acetate buffer (pH = 5). 40 µl of aqueous solution of 1 mM CB-20R dissolved in 10% DMSO, 2 µl of saturated ascorbic acid solution and 100 µl of solution containing ¹⁷⁷Lu were mixed, and the mixture was heated to 95°C for 10 minutes. The label was detected by radio-HPLC (within 5 minutes; 0%-100% ACN in water; C18 column).

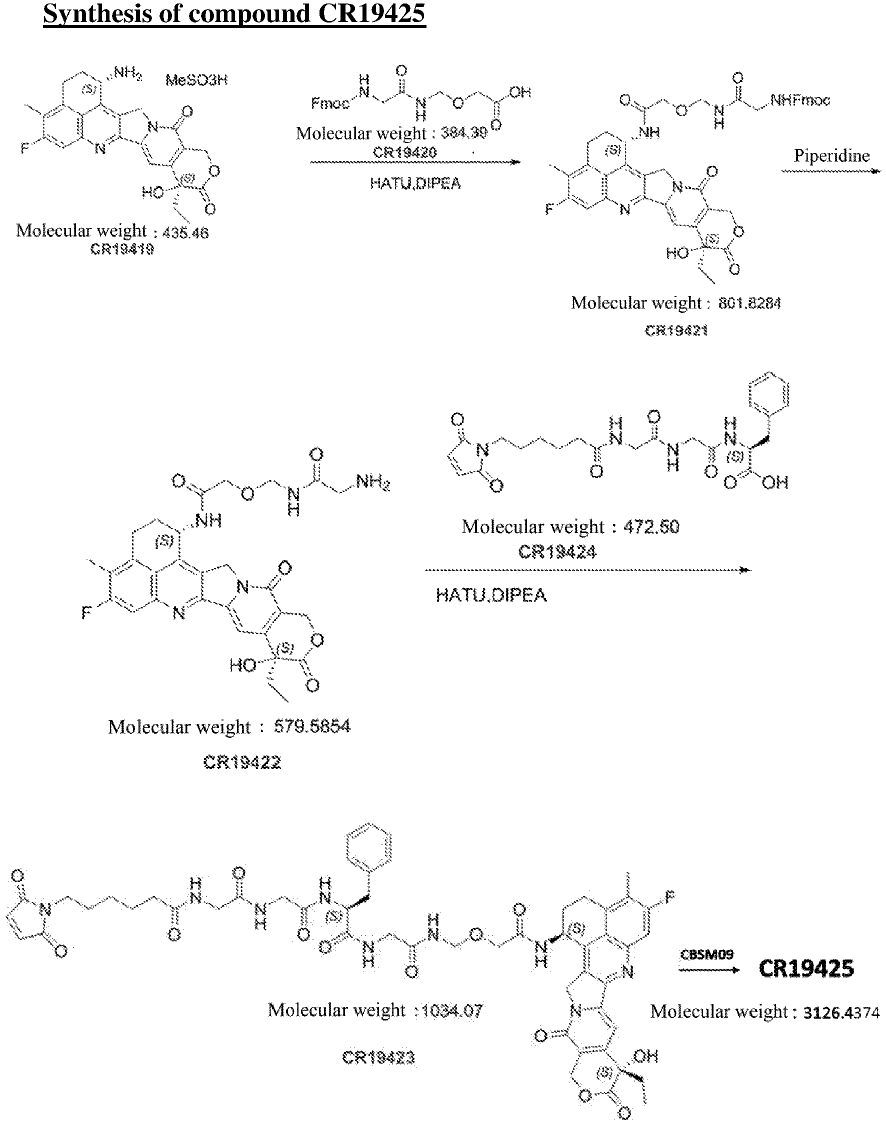

1. Under N₂ protection, to a reaction flask, 441.4 mg of CR19420 (with a structure as shown in the above reaction step) and 8.0 mL of DMF were added, stirred, dissolved and cooled in an ice bath; then 459.2 mg of HATU and 380 µL of DIPEA were added and stirred for half an hour; and then 500.0 mg of CR19419 (with a structure as shown in the above reaction step) and 190 µL of DIPEA were added, and the mixture was reacted at room temperature until the reaction was completed. After the reaction was completed, the reaction solution was poured into an acetic acid aqueous solution; a solid was precipitated and filtered, and the filter cake was washed with an acetic acid aqueous solution and water, and dried in vacuo to obtain 835.7 mg of CR19421 (with a structure as shown in the above reaction step) as a brown powder and with an HPLC purity of 90.0% and a yield of 90.6%.
2. Under N₂ protection, to a reaction flask, 835.7 mg of CR19421 and 16 mL of 10% piperidine DMF solution were added and reacted at room temperature for half an hour. After the reaction was completed, the reaction solution was poured into TFA/MTBE; a solid was precipitated and filtered, and the filter cake was washed with MTBE and dried in vacuo to obtain 599.7 mg of CR19422 (with a structure as shown in the above reaction step) as a field gray powder and with an HPLC purity of 84.7% and a yield of 83.0%.
3. Under N₂ protection, to a reaction flask, 599.7 mg of CR19422, 586.7 mg of CR19424 (with a structure as shown in the above reaction step) and 15 mL of DMF were added, stirred, dissolved and cooled in an ice bath; then 495.7 mg of HATU and 410 µL of DIPEA were added and reacted at room temperature. After the reaction was completed, the reaction solution was subjected to a preparative purification, and the acetonitrile was removed under reduced pressure from the pure product solution; the residue was extracted with a mixed solvent of dichloromethane and methanol, concentrated and dried to obtain 674.9 mg of CR19423 (with a structure as shown in the above reaction step) as a yellow powder and with an HPLC purity of 88.4% and a yield of 63.1%.
4. Under N₂ protection, to a reaction flask, 14.8 mg of CR19423, 3.0 mL of PBS buffer (pH = 6.6) and 3.0 mL of acetonitrile were added, stirred and dissolved; then 32.9 mg of CBSM09 was added, and the mixture was adjusted to pH 6.6-6.8 with Na₂HPO₄ and reacted for half an hour. After the reaction was completed, the reaction solution was subjected to a preparative purification, and the pure product was lyophilized to obtain 21.8 mg of CR19425 as a yellow powder and with an HPLC purity of 95.6% and a yield of 48.8%.

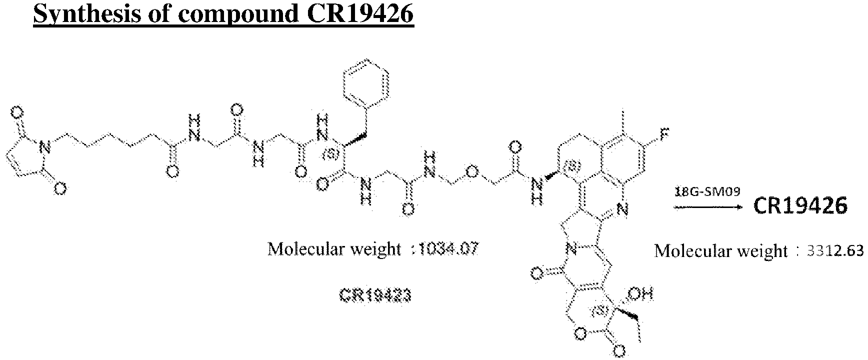

1. Under N₂ protection, to a reaction flask, 25.2 mg of CR19423, 3.0 mL of PBS buffer (pH = 6.6) and 3.0 mL of acetonitrile were added, stirred and dissolved; then 55.5 mg of 18G-SM09 was added, and the mixture was adjusted to pH 6.6-6.8 with Na₂HPO₄ and reacted for half an hour. After the reaction was completed, the reaction solution was subjected to a preparative purification, and the pure product was lyophilized to obtain 44.6 mg of CR19426 as a yellow powder and with an HPLC purity of 95.4% and a yield of 55.2%.

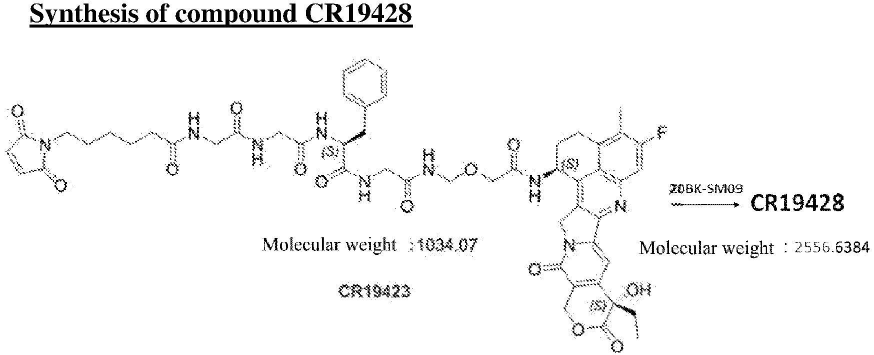

1. Under N₂ protection, to a reaction flask, 486 mg of CR19423, 3.0 mL of PBS buffer (pH = 6.6) and 3.0 mL of acetonitrile were added, stirred and dissolved; then 712 mg of 20BK-SM09 was added, and the mixture was adjusted to pH 6.6-6.8 with Na₂HPO₄ and reacted for half an hour. After the reaction was completed, the reaction solution was subjected to a preparative purification, and the pure product was lyophilized to obtain 508 mg of CR19428 as a yellow powder and with an HPLC purity of 96.7% and a yield of 42.3%.

### Example 2: Determination of Affinity of Conjugate Compounds to Target Proteins

### 1. Determination of binding affinity of CB-20BK to protein FOLR1

Experimental instruments, materials and reagents:
BIAcore T200 (GE)
CM5 chip (GE, Cat#: 29104988)
Buffer: HBS-EP+ buffer 10X (GE, Cat#: BR100669), diluted 10 folds with deionized water before use.
Amino coupling kit (GE, Cat#: BR100050)
Regeneration reagents: 10 mM Glycine 2.0 (GE, Cat#: BR100355)
10 mM Glycine 3.0 (GE, Cat#: BR100357)

### Experimental steps

The experiment was carried out according to BIAcore T200 (GE) instruction manual to determine the affinity of analytes CB-20BK, CB-20AK, folate (FA) and FA-MMAE to FOLR1. In the experiment, CM5 chip was conjugated with ligand FOLR1 (R&D System, Cat#: 5646-FR). The experimental results are as shown in Table 4.

**Table 4. Binding Affinity of CB-20BK and Related Compounds to FOLR1**

| | **ka** | **kd** | **KD** |
|---|---|---|---|
| **FA (folate)** | 3.34 × 10⁶ M⁻¹s⁻¹ | 2.26 × 10⁻⁴s⁻¹ | 6.77 × 10⁻¹¹ M |
| **FA-MMAE** | 1.79 × 10⁶ M⁻¹s⁻¹ | 1.15 × 10⁻⁴s⁻¹ | 6.43 × 10⁻¹¹ M |
| **CB-20AK** | N/D | N/D | N/D |
| **CB-20BK** | 1.03 × 10⁶ M⁻¹s⁻¹ | 1.31 × 10⁻⁴s⁻¹ | 1.27 × 10⁻¹⁰ M |

| | | | |
|---|---|---|---|
| "N/D" means that no specific binding was detected. | | | |

Table 4 shows that CB-20BK specifically binds to FOLR1 with a good affinity. The binding affinity of CB-20BK to FOLR1 is slightly weaker than the binding affinity of FA or FA-MMAE to FOLR1. CB-20AK does not comprise the folate moiety of CB-20BK and has not been detected to specifically bind to FOLR1 in the experiment.

### 2. Determination of binding affinity of CB-20BK to protein FOLH1

Experimental instruments, materials and reagents:
Gator™ (Probe Life)
SA probe (Probe Life, Cat#: 1906018)
Buffer: Q buffer (Probe Life), 10 mM, pH = 7.4

### Experimental steps

### (1) Synthesis steps of analyte Biotin-CB-20BK

1) 5.1 g of Rink Resin with a degree of substitution of 0.45 mmol/g was weighed and loaded onto a solid phase reaction column; DCM was added, and nitrogen gas was bubbled to swell the resin for 30 minutes; the solvent was pumped off; Fmoc protecting groups were removed by DBLK; and then the resin was washed 5 times with DMF. 2.45 g of Fmoc-Lys(Dde)-OH and 0.75 g of HOBt were weighed and dissolved in DMF; at 0°C in an ice-water bath, 0.83 ml of DIC was added to activate for 5 minutes; the mixture was added to the reaction column and reacted for 3 hours; and the solvent was pumped off, and the resin was washed 3 times. Then Fmoc protecting groups were removed by DBLK.
2) The above-mentioned operations were repeated, and Fmoc-Cys(Trt)-OH, Fmoc-Lys(Boc)-OH, Fmoc-Asp(OtBu)-OH, Fmoc-Asp(OtBu)-OH and intermediate 108 were successively conjugated according to the structures.
3) Dde protecting groups were removed twice with 2% hydrazine hydrate/DMF, for 10 minutes each time, and then the resin was washed 5 times with DMF. Fmoc-Lys(Biotin)-OH, Fmoc-Glu-OtBu and pteroic acid were conjugated successively, and after pteroic acid was conjugate, the resin was washed twice with DMF; and finally, the resin was condensed with methanol, and the solvent was pumped off to obtain 9.7 g of a protected peptide resin.
4) 9.7 g of the peptide resin obtained in the previous step was added to a 250 ml single-necked flask; 77.6 ml of lysis buffer (TFA : H2O : TIS = 95 : 3 : 2 (volume ratio)) was previously prepared, and 1.1 g of DTT was weighed and added to the lysis buffer. The lysis buffer was added to the flask, and the mixture was reacted at room temperature for 2.5 hours; the resin was filtered and washed with 20 ml of TFA; the filtrate was combined, and the mixture was added to 466 ml of methyl tert-butylether, with a yellow solid being precipitated, and centrifuged to obtain a solid, which was washed with methyl tert-butylether and dried in vacuo to obtain 4.6 g of a yellow solid with an HPLC purity of 83.2%. The product was separated by prep-HPLC and lyophilized to obtain 2.1 g of Biotin-20BK-SM09 with a purity of no less than 95%.
5) 500 mg of Mc-Val-Cit-PAB-MMAE (LT1002) was weighed and added to a 250 ml single-necked flask, and 65 ml of phosphate buffer and 20 ml of acetonitrile were added; the solution was stirred and maintained at pH = 7.2 until a clear solution was obtained; and 785 mg of intermediate Biotin-20BK-SM09 was added, and the mixture was reacted at room temperature for 2 hours, during which the reaction was monitored by HPLC. After the reaction was completed, the mixture was filtered, separated by prep-HPLC, and lyophilized to obtain 723 mg of Biotin-CB-20BK, with a purity of 96.8% and a yield of 59.4%.

(2) The experiment was carried out according to Gator™ (Probe Life) instruction manual to determine the binding affinity of Biotin-CB-20BK to FOLH1. In the experiment, SA probe binds to ligand Biotin-CB-20BK, and the analyte is FOLH1 (Sino Biologicals, Cat#: 15877-H07H). The experimental results are as shown in Table 5.

**Table 5. Binding Affinity of CB-20BK to FOLH1**

| | **ka** | **kd** | **KD** |
|---|---|---|---|
| **FOLH1** | 1.19 × 10⁴ M⁻¹s⁻¹ | 1.70 × 10⁻⁴s⁻¹ | 6.98 × 10⁻⁹ M |

Table 5 shows that CB-20BK binds to FOLH1 with a good affinity.

### 3. FOLH1 binds to CB-20BK-FOLR1 complex

Experimental materials and reagents:
BIAcore T200 (GE)
CM5 chip (GE, Cat#: 29104988)
Buffer: HBS-EP+ buffer 10X (GE, Cat#: BR100669), diluted 10 folds with deionized water before use.
Amino coupling kit (GE, Cat#: BR100050)
Regeneration reagents: 10 mM Glycine 2.0 (GE, Cat#: BR100355)
10 mM Glycine 3.0 (GE, Cat#: BR100357)

### Experimental steps

CM5 chip was conjugated with a ligand: FOLR1 (R&D System, Cat#: 5646-FR)
Analyte: CB-20BK, FA-MMAE and FOLH1 (Sino Biologicals, Cat#: 15877-H07H)

According to BIAcore T200 (GE) operating manual, FOLR1 was conjugated to the CM5 chip; CB-20BK or FA-MMAE was loaded first, and then FOLH1 was loaded; and the binding of FOLH1 to the CB-20BK-FOLR1 complex was detected. The experimental results are as shown in Table 6.

**Table 6. Binding of CB-20BK-FOLR1 Complex to FOLH1 Solution at Different Concentrations**

| Concentration (µM) | FA-MMAE (RU) | CB-20BK (RU) |
|---|---|---|
| 0.5 | 9.8 | 23.9 |
| 0.25 | 8.4 | 14.1 |
| 0.125 | 4.2 | 4.6 |
| 0.0625 | -1 | -0.9 |

Table 6 shows that with regard to an FOLH1 solution at a high concentration, the amount of FOLH1 bound to a CB-20BK-FOLR1 complex is significantly higher than that bound to an FA-MMAE-FOLR1 complex, which shows a continuous rising trend. The binding of FOLH1 to an FA-MMAE-FOLR1 complex tends to saturate at a high concentration. This experiment has proved that CB-20BK can bind to both FOLR1 and FOLH1 receptors with a good affinity.

### Example 3: Study on Binding and Endocytosis of Ligand Conjugates to Target Cells

### 1. Cell binding and endocytosis experiment of conjugate compound CB-20BK

### Synthesis of labelled samples Cy5-pep-20BK, Cy5-FA and Cy5-pep-20AK

(1) 2 g of Rink Resin with a degree of substitution of 0.45 mmol/g was weighed and loaded onto a solid phase reaction column; DCM was added, and nitrogen gas was bubbled through the solvent to swell the resin for 30 minutes; the solvent was pumped off; Fmoc protecting groups were removed by DBLK; and then the resin was washed 5 times with DMF. 0.96 g (1.8 mmol) of Fmoc-Lys(Dde)-OH and 0.3 g (2.2 mmol) of HOBt were weighed and dissolved in DMF; at 0°C in an ice-water bath, 0.33 ml (2.2 mmol) of DIC was added and mixed to activate for 5 minutes; the mixture was added to the reaction column and reacted for 3 hours; and the solvent was pumped off, and the resin was washed 3 times. Then Fmoc protecting groups were removed by DBLK.
(2) The above-mentioned operations were repeated, and Fmoc-Lys(Boc)-OH, Fmoc-Asp(OtBu)-OH, Fmoc-Asp(OtBu)-OH and intermediate 108 were conjugated successively according to the structures:
(3) Dde protecting groups were removed twice with 2% hydrazine hydrate/DMF, for 10 minutes each time, and then the resin was washed 5 times with DMF. Fmoc-Lys(Dde)-OH, Fmoc-Glu-OtBu and pteroic acid were conjugated successively.
(4) Dde protecting groups were removed twice with 2% hydrazine hydrate/DMF, for 10 minutes each time, and then the resin was washed 5 times with DMF; the resin was conjugated with Cy5-COOH and then washed twice with DMF; and finally, the resin was condensed twice with methanol and the solvent was pumped off to obtain 3.6 g of a protected peptide resin.
(5) 3.6 g of the peptide resin obtained in the previous step was added to a 50 ml single-necked flask; 29 ml of lysis buffer (TFA : H₂O : TIS = 95 : 3 : 2 (volume ratio)) was previously prepared, and 0.4 g of DTT was weighed and added to the lysis buffer. The lysis buffer was added to the flask, and the mixture was reacted at room temperature for 2.5 hours; the resin was filtered and washed with 8 ml of TFA; the filtrate was combined, and the mixture was added to 173 ml of absolute ether, with a yellow solid being precipitated, and centrifuged to obtain a solid, which was washed with absolute ether and dried in vacuo to obtain 1.9 g of a crude product as a blue solid and with a yield of 89.6% and an HPLC purity of 76.3%. The product was separated by prep-HPLC (condition: C18 column, mobile phase A: 0.1% trifluoroacetic acid in water, B: acetonitrile, elution gradient: (20-28)%B, elution time: 50 minutes; fractions were collected); and the fraction containing the qualified product was lyophilized to obtain 736 mg of Cy5-pep-20BK, with a purity of 93.4%.

In the same way, compounds Cy5-FA and Cy5-pep-20AK can be obtained through similar steps in the above-mentioned methods.

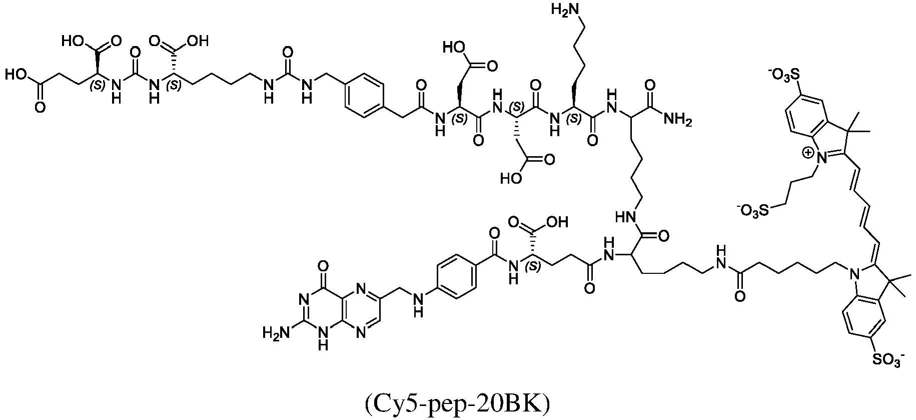

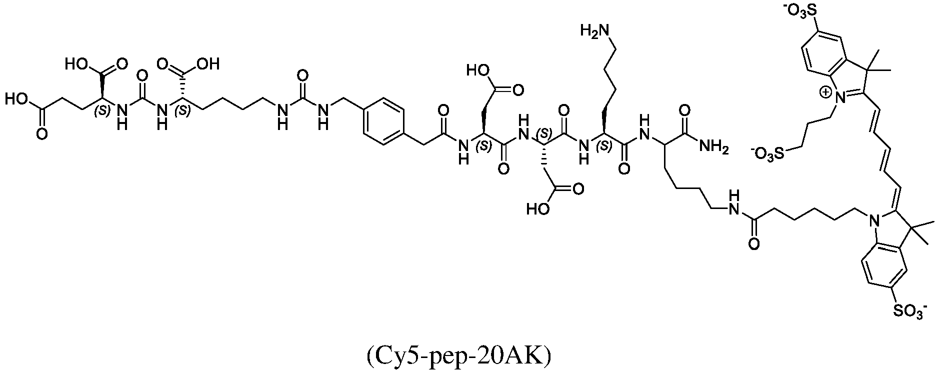

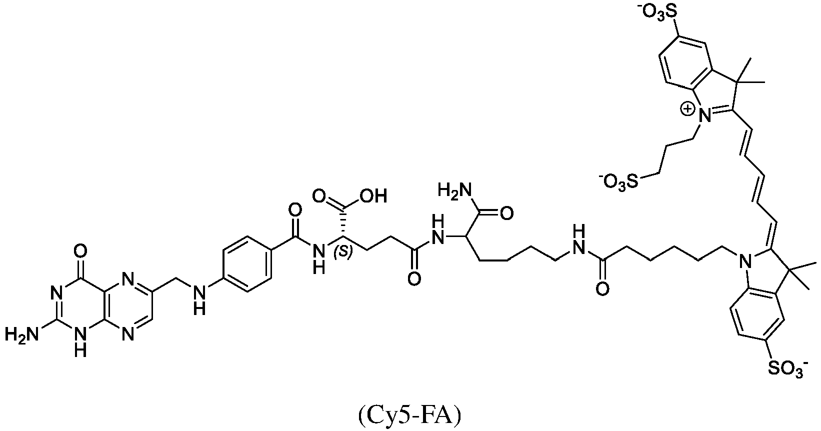

### Experiment for detecting cell binding and endocytosis of samples Cy5-pep-20AK/Cy5-pep-20BK by flow cytometry method

Sample information: Cy5-pep-20AK and Cy5-pep-20BK
Cell lines: LNCaP human prostate cancer cells, Du145 human prostate cancer cells, SKOV3 human ovarian cancer cells and NCI-H460 human lung cancer cells
Main reagents: IMDM medium, fetal bovine serum, penicillin-streptomycin solution, L-glutamine and PBS

Experimental operations:
(1) Cell culture: the cells were digested with trypsin, collected and counted, and then the cells were added to several culture flasks containing a complete medium; the culture flasks were placed in an incubator at 37°C, 5% CO₂ for culturing the cells, and finally about 5 × 10⁶ cells were collected into a centrifuge tube.
(2) Sample incubation: Cy5-pep-20AK/Cy5-pep-20BK samples were diluted to 8 nmol/L with PBS; the cell suspension was centrifuged at 1000 rpm for 5 minutes; the supernatant was removed, and the residue was washed once with PBS; the cells were suspended uniformly and divided equally into different centrifuge tubes according to the experimental scheme; PBS was removed by centrifugation; 200 µl of a sample working solution was added to each tube and incubated at 37°C for 15, 30, 60, and 90 minutes, respectively, and 1 tube to which only PBS was added was used as a blank control; all operations were performed avoiding light exposure.
(3) Washing and loading: the working solution was removed by centrifugation at 1000 rpm for 5 minutes, and the cells were washed 3 times with PBS, and an appropriate amount of PBS was added to suspend the cells to 1 × 10⁶ cells/ml; Beckman CytoFLEX flow cytometer was turned on in advance to complete the startup cleaning process; and the cell samples were loaded successively, and the fluorescence of 10,000 living cells was read in the APC channel; all operations were performed avoiding light exposure.
(4) Data analysis: the absolute MFI (mean fluorescence intensity) of the APC fluorescence of each cell sample and the relative MFI of the relative blank control were obtained, and a data line graph was completed according to the relative MFI.

### Results and analysis

**Table 7. Expression Levels of FOLR1 and FOLH1 in Different Cell Lines (with reference to data from Depmap, https://depmap.org/portal/)**

| RNA-Seq | LNCaP | SKOV3 | Du145 | NCI-H460 |
|---|---|---|---|---|
| FOLR1 | +/- | 6+ | +/- | +/- |
| FOLH1 | 10+ | + | +/- | +/- |

According to data from Depmap, the data being 0 or negative is expressed as "-", the data being 0.001-0.499 is expressed as "+/-", the data being 0.500-1.499 is expressed as "+", the data being 1.500-2.499 is expressed as "2+", and so on.

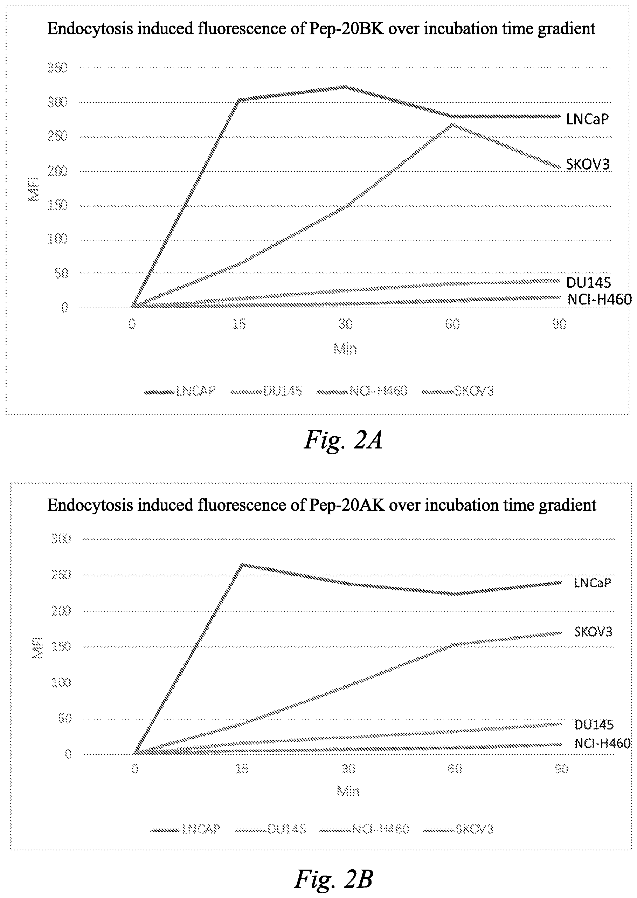

The fluorescence intensity of cells was respectively detected at 15 minutes, 30 minutes, 60 minutes, and 90 minutes of incubation, and the results were plotted and shown in Figs. 2A and 2B. The binding and endocytosis of the sample Cy5-pep-20BK cells can be seen from the figures. Compared with a DU145 cell line with a relatively low expression of FOLR1 and a NCI-H460 cell line with a relatively low expression of FOLH1, an LNCaP cell line with a relatively high expression of FOLR1 and an SKOV3 cell line with a relatively high expression of FOLH1 have a significantly higher fluorescence intensity of CB-20BK that was bond and endocytosed. Since Cy5-pep-20AK only comprises an FOLH1 ligand and does not comprise the FOLR1 ligand, FA, Cy5-pep-20AK shows a high level of binding and endocytosis in LNCaP cells with a high expression of FOLH1, and shows a moderate level of binding and endocytosis in SKOV3 cells with a moderate expression of FOLH1; in the two cell lines, the binding and endocytosis level of Cy5-pep-20AK was significantly lower than that of Cy5-pep-CB-20BK. The degree of binding and internalization of a ligand conjugate to cells is positively correlated with expression levels of cell-related receptors.

### 2. Binding and endocytosis experiment of single-ligand conjugate Cy5-FA to cells

Sample information: Cy5-FA
Cell lines: Hela cervical cancer cells, SKOV3 human ovarian cancer cells and A549 human lung cancer cells
Main reagents: IMDM medium, fetal bovine serum, penicillin-streptomycin solution, L-glutamine, PBS, and anti-fluorescence quenching mounting medium containing DAPI

Experimental operations:
(1) Cells growing on coverslips: coverslips were placed in a 24-well plate; the cells were digested with trypsin, collected and counted, and then the cells were diluted with a complete cell culture medium to about 2 × 10⁵ cells/ml; 500 µl of the diluted cell solution was added to each well of the 24-well plate; and the plate was placed in an incubator at 37°C, 5% CO₂ for culturing 48 hours.
(2) Sample incubation: the Cy5-FA sample was diluted in an IMDM medium to 73 nmol/L; the culture medium in the 24-well plate was discarded; 200 µl of the sample working solution was added to the cell coverslip in each well and incubated at 37°C for 15, 30, and 60 minutes, respectively, and 1 well to which only an IMDM medium was added was used as a blank control; all operations were performed avoiding light exposure.
(3) Washing and mounting: the working solution in the 24-well plate was discarded, and the plate was washed 3 times with preheated PBS at 37°C; the cell coverslips were taken out, and 5 µl of anti-fluorescence quenching mounting medium containing DAPI was added dropwise onto the slides and then covered with the cell coverslips to complete the mounting; all operations were performed avoiding light exposure.
(4) Image interpretation for samples: Leica fluorescence microscope DM2500 was turned on to preheat the fluorescence exciter for 15 minutes; at the same position, cell nucleus and Cy5 fluorescein were photographed at the corresponding fluorescence channel, and the image fusion was completed in the software.

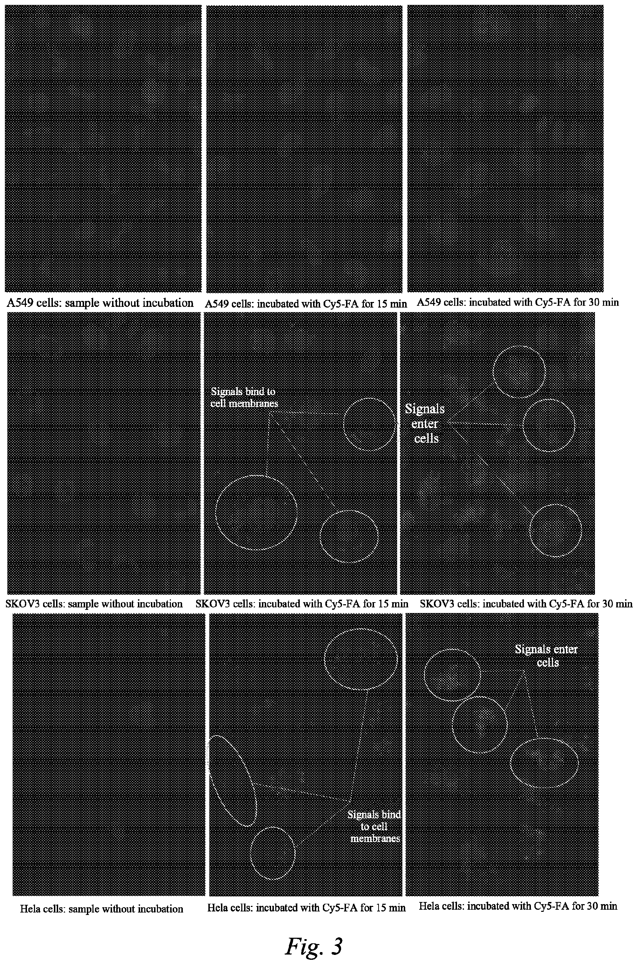

The binding and endocytosis of the sample Cy5-FA to cells at 15 minutes and 30 minutes can be seen from Fig. 3, and the fluorescence intensity in cell lines Hela and SKOV-3 with a high expression of FOLR1 is significantly higher than that in a cell line A549 with a relatively low expression of FOLR1. The degree of binding and internalization of a ligand conjugate to cells is positively correlated with expression levels of cell-related receptors.

### Example 4: Experiment of Inhibiting Cell Expansion in Conjugate Compounds 1. Experiment of inhibiting tumour cell expansion in CB-20BK

Sample information: CB-20BK
Cell lines: KB human oral epidermoid carcinoma cells, T-47D human breast cancer cells, NCI-H460 human lung cancer cells, CALU-3 human lung adenocarcinoma cells, HuH-7 human liver cancer cells and LNCaP human prostate cancer cells
Main reagents: IMDM medium, fetal bovine serum, penicillin-streptomycin solution, L-glutamine and CCK8

### Experimental operations:

### 1) Cell plating

The cells were prepared in advance, digested with trypsin, collected and counted; with a complete cell culture medium, KB cells were diluted to 2.5 × 10⁴ cells/ml, T-47D cells were diluted to 1.3 × 10⁴ cells/ml, NCI-H460 cells were diluted to 3.5 × 10⁴ cells/ml, CALU-3 cells were diluted to 4.0 × 10⁴ cells/ml, HuH-7 cells were diluted to 3.0 × 10⁴ cells/ml, and LNCaP cells were diluted to 8.0 × 10⁴ cells/ml; 100 µl of the diluted cell solution was added to each well of 96-well plates; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 8). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 8: Concentration of Sample (CB-20BK) After Dilution**

| Tube number | Concentration after dilution (µmol/L) |
|---|---|
| 1 | 201 |
| 2 | 50 |
| 3 | 13 |
| 4 | 3.1 |
| 5 | 0.8 |
| 6 | 0.2 |
| 7 | 0.05 |
| 8 | 0.01 |
| 9 | 0.003 |
| 10 | 0.0008 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4A, wherein C value corresponds to IC₅₀), CB-20BK has an inhibitory effect on the growth and expansion of KB, T-47D, NCI-H460, CALU-3, HuH-7, and LNCaP tumour cell lines. Since expression levels of the corresponding receptors of conjugate compounds in the cells are different, the inhibition levels are different. The IC₅₀ for cell lines T-47D, KB, LNCaP, HuH-7 and CALU-3 with a relatively high expression of the receptors is significantly lower than that for cell line NCI-H460 with a relatively low expression of the receptors. CB-20BK shows a correlation between tumour growth inhibition effects and expression levels of cell-related receptors.

### 2. Experiment of conjugate compound CB-20BK and related compounds inhibiting expansion of tumour cell lines LNCaP (human prostate cancer cells) and 22RV1 (human prostate cancer cells)

Since different cells have different sensitivity to a cell proliferation inhibitory toxicity of the payload in a ligand conjugate, the quantitative analysis may be interfered occasionally. A series of cell lines with known expression levels of receptors of different ligands were selected and tested for the response to inhibitory effects of the cell proliferation of a ligand conjugate and a single payload. The IC₅₀ ratio of a single payload to that of a ligand conjugate in same cell line were taken to remove such interference.

### Related samples: MMAE, CB-20BK, CB-20AK and FA-MMAE

Experimental operations: LNCaP and 22RV1 cells were prepared in advance, counted, and plated in 96-well cell culture plates at a cell density of 4 × 10⁴ cells/ml and 2.0 × 10⁴ cells/ml, respectively (100 µl/well); negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The calculated data is shown in Table 9.

**Table 9. Inhibitory Effects of MMAE, FA-MMAE, CB-20BK and CB-20AK on Proliferation of Cell Lines and Receptor Gene Expression Levels in Cell Lines (with reference to data from Depmap)**

| Cell line | FOL R1_ gene expr essio n level | FOL H1_ gene expr essio n level | IC₅₀ (MMAE) | IC₅₀ (FA-MMA E) | IC₅₀ (CB-2 0AK) | IC₅₀ (CB-20 BK) | IC₅₀ ratio (MM AE/F A-M MAE) | IC₅₀ ratio (MMA E/CB-2 0AK) | IC₅₀ ratio (MMA E/CB-2 0BK) |
|---|---|---|---|---|---|---|---|---|---|
| LNCaP | +/- | 10+ | 0.00255 | 0.264 | 0.102 | 0.0958 | 0.010 | 0.025 | 0.0267 |
| 22RV1 | +/- | 6+ | 0.00648 | 1.01 | 1.05 | 0.502 | 0.006 | 0.006 | 0.013 |

It can be seen from the above table that there is not much difference in the expression level of FOLR1 between LNCaP and 22RV1, and the expression level of FOLH1 in LNCaP is significantly higher than the expression level of FOLH1 in 22RV1. CB-20BK (with FOLH1 ligand and FOLR1 ligand) and CB-20AK (only with FOLH1 ligand) have inhibitory effects on the expansion of both LNCaP and 22RV1 tumour cell lines. The inhibitory effects of CB-20BK and CB-20AK on the cells with different expression levels of receptor FOLH1 are different. The IC₅₀ ratio in cell line LNCaP with a relatively high expression of receptor FOLH1 is 2-4 times higher than the IC₅₀ ratio in cell line 22RV1 with a relatively low expression of the receptor. FA-MMAE also has an inhibitory effect on the expansion of LNCaP and 22RV1 tumour cell lines. Since the expression level of FOLR1 in LNCaP and 22RV1 is very low, the expression in LNCaP cells (FOLR1 gene expression level is 0.3219, with reference to data from Depmap) is slightly higher than that in 22RV1 (FOLR1 gene expression level is 0.0704, with reference to data from Depmap), and the IC₅₀ ratio of FA-MMAE between LNCaP and 22RV1 only differs by a factor of 1.5. The above data indicates that inhibitory effects on cell proliferation is positively correlated with expression levels of cell expression receptors FOLH1 and FOLR1.

### 3. Experiment of conjugate compound CB-20BK and related compounds inhibiting expansion of tumour cell lines PANC-1 (human pancreatic cancer cells) and CFPAC-1 (human pancreatic cancer cells)

### Related samples: MMAE and CB-20BK

Experimental operations: PANC-1 and CFPAC-1 cells were prepared in advance, counted, and plated in 96-well cell culture plates at a cell density of 4 × 10⁴ cells/ml (100 µl/well); negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The calculated data is shown in the following table:

**Table 10. Inhibitory Effects of CB-20BK on Proliferation of Cell Lines and Receptor Gene Expression Levels in Cell Lines (with reference to data from Depmap)**

| **Cell line** | **FOLR1 gene expression level** | **FOLH1 gene expression level** | **IC₅₀ (MMAE)** | **IC₅₀ (CB-20BK)** | **IC₅₀ (MMAE)/** |
|---|---|---|---|---|---|
| | | | | | **IC₅₀ (CB-20BK)** |
| CFPAC-1 | 4+ | +/- | 0.015 | 0.557 | 0.027 |
| PANC-1 | +/- | +/- | 0.002 | 0.126 | 0.020 |

It can be seen from Table 10 that the expression level of FOLH1 in CFPAC-1 is very low, and the expression level of FOLR1 in CFPAC-1 is significantly higher than that in PANC1 cell lines. CB-20BK has an inhibitory effect on the expansion of PANC-1 and CFPAC-1 tumour cell lines. The inhibitory effects on the cells with different expression levels of receptor FOLR1 are different. The IC₅₀ ratio in cell line CFPAC-1 with a relatively high expression of receptor FOLR1 is significantly higher than the IC₅₀ ratio in the PANC-1 with a relatively low expression of receptor FOLR1, and the inhibitory effects on cell proliferation is positively correlated with expression levels of cell-related receptor FOLR1.

Experiments 2 and 3 prove that the two ligands in CB-20BK and the receptors thereof (FOLR1 and FOLH1) expressed on the cell surface play an important role in the compound inhibiting tumour cell proliferation.

### 4. Experiment of inhibiting tumour cell expansion in CB-20B

Sample information: CB-20B
Cell lines: A549 human lung cancer cells, HuH-7 human liver cancer cells, KB human oral epidermoid carcinoma cells, LNCaP human prostate cancer cells, DU145 human prostate cancer cells and T-47D human breast cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; diluted with a complete cell culture medium, A549 cells, HuH-7 cells, KB cells and DU145 cells were plated at a density of 2 × 10⁴ cells/ml, T-47D cells were plated at a density of 1 × 10⁵ cells/ml, and LNCaP cells were plated at a density of 6 × 10⁴ cells/ml in 96-well plates; 100 µl of the diluted cell solution was added to each well; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 11). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 11: Concentration of Sample (CB-20B) After Dilution**

| Tube number | Concentration after dilution (µmοl/L) |
|---|---|
| 1 | 200 |
| 2 | 66.7 |
| 3 | 22.2 |
| 4 | 7.4 |
| 5 | 2.5 |
| 6 | 0.8 |
| 7 | 0.3 |
| 8 | 0.09 |
| 9 | 0.03 |
| 10 | 0.01 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4B, wherein C value corresponds to IC₅₀), CB-20B has an inhibitory effect on the growth and expansion of A549, HuH-7, KB, LNcap, DU145 and T-47D tumour cell lines. Since expression levels of the corresponding receptors of conjugate compounds in the cells are different, the inhibition levels are different. The IC₅₀ for cell lines T-47D, LNcap, KB, HuH-7 and DU145 with a relatively high expression of the receptors is significantly lower than that for cell line A549 with a relatively low expression of the receptors. CB-20B shows a correlation between tumour growth inhibition effects and expression levels of cell-related receptors.

### 5. Experiment of inhibiting tumour cell expansion in CB-10S

Sample information: CB-10S
Cell lines: KB human oral epidermoid carcinoma cells, NCI-H460 human lung cancer cells, RT4 human bladder cancer cells, T-47D human breast cancer cells and LNcap human prostate cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; with a complete cell culture medium, KB cells were diluted to 3.5 × 10⁴ cells/ml, LNCaP cells were diluted to 8.0 × 10⁴ cells/ml, T-47D cells were diluted to 1.2 × 10⁴ cells/ml, NCI-H460 cells were diluted to 2.5 × 10⁴ cells/ml, and RT4 cells were diluted to 1.2 × 10⁴ cells/ml; to each well of 96-well plates, 100 µl of the diluted cell solution was added; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 12). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 12: Concentration of Sample (CB-10S) After Dilution**

| Tube number | Concentration after dilution (µmοl/L) |
|---|---|
| 1 | 302 |
| 2 | 43 |
| 3 | 6 |
| 4 | 0.9 |
| 5 | 0.1 |
| 6 | 0.02 |
| 7 | 0.003 |
| 8 | 0.0004 |
| 9 | 0.00005 |
| 10 | 0.000007 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4C, wherein C value corresponds to IC₅₀), CB-10S has an inhibitory effect on the growth and expansion of KB, LNCaP, T-47D, RT4 and NCI-H460 tumour cell lines. Since expression levels of the corresponding receptors of conjugate compounds in the cells are different, the inhibition levels are different. The IC₅₀ for cell lines KB, LNcap, T-47D and RT4 with a relatively high expression of the receptors is significantly lower than that for cell line NCI-H460 with a relatively low expression of the receptors. CB-10S shows a correlation between tumour growth inhibition effects and expression levels of cell-related receptors.

### 6. Experiment of inhibiting tumour cell expansion in CB-60S

Sample information: CB-60S
Cell lines: KB human oral epidermoid carcinoma cells, T-47D human breast cancer cells, NCI-H460 human lung cancer cells, CALU-3 human lung adenocarcinoma cells, HuH-7 human liver cancer cells and LNCaP human prostate cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; with a complete cell culture medium, KB cells were diluted to 2.0 × 10⁴ cells/ml, T-47D cells were diluted to 1.5 × 10⁴ cells/ml, NCI-H460 cells were diluted to 2.0 × 10⁴ cells/ml, CALU-3 cells were diluted to 3.0 × 10⁴ cells/ml, HuH-7 cells were diluted to 4.0 × 10⁴ cells/ml, and LNCaP cells were diluted to 8.0 × 10⁴ cells/ml; 100 µl of the diluted cell solution was added to each well of 96-well plates; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 13). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 13: Concentration of Sample (CB-60S) After Dilution**

| Tube number | Concentration after dilution (µmοl/L) |
|---|---|
| 1 | 320 |
| 2 | 80 |
| 3 | 20 |
| 4 | 5 |
| 5 | 1.3 |
| 6 | 0.3 |
| 7 | 0.08 |
| 8 | 0.02 |
| 9 | 0.005 |
| 10 | 0.001 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4D, wherein C value corresponds to IC₅₀), CB-60S has an inhibitory effect on the growth and expansion of KB, T-47D, NCI-H460, CALU-3, HuH-7, and LNCaP tumour cell lines. Since expression levels of the corresponding receptors of conjugate compounds in the cells are different, the inhibition levels are different. The IC₅₀ for cell lines LNCaP and HuH-7 with a relatively high expression of the receptors is significantly lower than that for cell lines NCI-H460, KB, T-47D and CALU-3 with a relatively low expression of the receptors. CB-60S shows a correlation between tumour growth inhibition effects and expression levels of cell-related receptors.

### 7. Experiment of inhibiting tumour cell expansion in CB-60SK

Sample information: CB-60SK
Cell lines: KB human oral epidermoid carcinoma cells, T-47D human breast cancer cells, NCI-H460 human lung cancer cells, CALU-3 human lung adenocarcinoma cells, HuH-7 human liver cancer cells and LNCaP human prostate cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; with a complete cell culture medium, KB cells were diluted to 2.5 × 10⁴ cells/ml, T-47D cells were diluted to 1.3 × 10⁴ cells/ml, NCI-H460 cells were diluted to 3.5 × 10⁴ cells/ml, CALU-3 cells were diluted to 4.0 × 10⁴ cells/ml, HuH-7 cells were diluted to 3.0 × 10⁴ cells/ml, and LNCaP cells were diluted to 8.0 × 10⁴ cells/ml; 100 µl of the diluted cell solution was added to each well; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 14). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 14: Concentration of Sample (CB-60SK) After Dilution**

| Tube number | Concentration after dilution (µmol/L) |
|---|---|
| 1 | 289 |
| 2 | 72 |
| 3 | 18 |
| 4 | 4.5 |
| 5 | 1.1 |
| 6 | 0.3 |
| 7 | 0.07 |
| 8 | 0.02 |
| 9 | 0.004 |
| 10 | 0.001 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4E, wherein C value corresponds to IC₅₀), CB-60SK has an inhibitory effect on the growth and expansion of KB, T-47D, NCI-H460, CALU-3, HuH-7, and LNCaP tumour cell lines. Since expression levels of the corresponding receptors of conjugate compounds in the cells are different, the inhibition levels are different. The IC₅₀ for cell lines LNCaP and HuH-7 with a relatively high expression of the receptors is significantly lower than that for cell lines T-47D, NCI-H460, CALU-3 and KB with a relatively low expression of the receptors. CB-60SK shows a correlation between tumour growth inhibition effects and expression levels of cell-related receptors.

### 8. Experiment of inhibiting tumour cell expansion in CB-18G

Sample information: CB-18G
Cell lines: A549 human lung cancer cells, Hela human cervical cancer cells, SCLC-21H small cell lung cancer cells, U-2 OS human osteosarcoma cells, T-47D human breast cancer cells and NCI-H460 human lung cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; diluted with a complete cell culture medium, A549 cells, Hela cells, SCLC-21H cells and U-2 OS cells were plated at a density of 2 × 10⁴ cells/ml, and T-47D cells and NCI-H460 cells were plated at a density of 3 × 10⁴ cells/ml in 96-well plates; to each well of the 96-well plates, 100 µl of the diluted cell solution was added; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 15). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 15: Concentration of Sample (CB-18G) After Dilution**

| Tube number | Concentration after dilution (µmol/L) |
|---|---|
| 1 | 100 |
| 2 | 25 |
| 3 | 6.25 |
| 4 | 1.56 |
| 5 | 0.39 |
| 6 | 0.10 |
| 7 | 0.02 |
| 8 | 0.006 |
| 9 | 0.002 |
| 10 | 0.0004 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4F, wherein C value corresponds to IC₅₀), CB-18G has an inhibitory effect on the growth and expansion of A549, Hela, SCLC-21H, U-2 OS, T-47D and NCI-H460 tumour cell lines. Since expression levels of the corresponding receptors of conjugate compounds in the cells are different, the inhibition levels are different. The IC₅₀ for cell line Hela with a relatively high expression of the receptors is significantly lower than that for cell line NCI-H460 with a relatively low expression of the receptors. CB-18G shows a correlation between tumour growth inhibition effects and expression levels of cell-related receptors.

### 9. Experiment of inhibiting tumour cell expansion in CB-50S

Sample information: CB-50S
Cell lines: KB human oral epidermoid carcinoma cells, NCI-H460 human lung cancer cells, RT4 human bladder cancer cells, T-47D human breast cancer cells and LNCaP human prostate cancer cells
Main reagents: IMDM medium, fetal bovine serum, penicillin-streptomycin solution, L-glutamine and CCK8

Experimental operations:

### 1) Cell plating

The cells were prepared in advance, digested with trypsin, collected and counted; with a complete cell culture medium, KB cells were diluted to 3.5 × 10⁴ cells/ml, LNCaP cells were diluted to 8.0 × 10⁴ cells/ml, T-47D cells were diluted to 1.2 × 10⁴ cells/ml, NCI-H460 cells were diluted to 2.5 × 10⁴ cells/ml, and RT4 cells were diluted to 1.2 × 10⁵ cells/ml; to each well of 96-well plates, 100 µl of the diluted cell solution was added; and negative control and blank control wells were set on each plate. The 96-well plates added with the cells were placed in an incubator at 37°C, 5% CO₂ for culturing overnight.

### 2) Diluting and adding samples

The samples were diluted with a culture medium to desired concentrations (see Table 16). To each well of the 96-well plates that were cultured overnight, 50 µl of the diluted samples were added, with 3 replicate wells; negative controls and blank controls were set; and the plates were placed in an incubator at 37°C, 5% CO₂ for culturing 72 hours.

**Table 16: Concentration of Sample (CB-50S) After Dilution**

| Tube number | Concentration after dilution (µmol/L) |
|---|---|
| 1 | 314 |
| 2 | 45 |
| 3 | 6 |
| 4 | 0.9 |
| 5 | 0.1 |
| 6 | 0.02 |
| 7 | 0.003 |
| 8 | 0.0004 |
| 9 | 0.00005 |
| 10 | 0.000008 |

### 3) Colour development and reading plate

To each well, 15 µl (10% of the liquid volume in a well) of a colour developing solution from CCK-8 was added; the plates were incubated at 37°C for an appropriate time (try to keep the OD value in the range of 1.0-2.0); the covers of the 96-well plates were removed, and the plates were read at 450 nm on a microplate reader (Molecular Devices Spectra MAX Plus).

### 4) Data processing

The data was edited using SoftMax Pro and a four-parameter fitting curve was plotted.

### 5) Experimental results and analysis

According to the four-parameter fitting curve graph (Fig. 4G, wherein C value corresponds to IC₅₀), CB-50S has an inhibitory effect on the growth and expansion of KB, LNCaP, T-47D, RT4 and NCI-H460 tumour cell lines. Since expression levels of the corresponding receptors of conjugate compounds in the cells are different, the inhibition levels are different. The IC₅₀ for cell lines KB, LNcap, T-47D and RT4 with a relatively high expression of the receptors is significantly lower than that for cell line NCI-H460 with a relatively low expression of the receptors. CB-50S shows a correlation between tumour growth inhibition effects and expression levels of cell-related receptors.

### 10. Experiment of inhibiting tumour cell expansion in conjugate compound CB-1020

Experiment purpose: to test inhibitory effects of CB-1020 and related compounds on the expansion of tumour cell lines LNCaP (human prostate cancer cells), SK-BR-3 (human breast cancer cells), NCI-H226 (human lung cancer cells), CFPAC-1 (pancreatic cancer cells) and PANC -1 (pancreatic cancer cells).

### Related samples: MMAE and CB-1020

Experimental operations: LNCaP, SK-BR-3, NCI-H226, CFPAC-1 and PANC-1 cells were prepared in advance and counted; with a culture medium (IMDM + 10% FBS + IX L-Glutamine + IX P/S), the cells LNCaP, SK-BR-3 and CFPAC-1 were diluted to 4 × 10⁴ cells/ml, NCI-H226 was diluted to 2.0 × 10⁴ cells/ml and PANC-1 was diluted to 3.0 × 10⁴ cells/ml; the diluted cell solution was plated in 96-well plates at 100 µl/well; and negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The data is shown in the following table:

**Table 17. Inhibitory Effects of CB-1020 on Proliferation of Cell Lines and Receptor Gene Expression Levels in Cell Lines (with reference to data from Depmap)**

| **Cell line** | **TRPV6 gene expression level** | **FOLH1 gene expression level** | **IC₅₀ (MMAE)** | **IC₅₀ (CB-1020)** | **IC₅₀ (MMAE)/IC₅₀ (CB-1020)** |
|---|---|---|---|---|---|
| LNCap | 5+ | 10+ | 0.003 | 0.168 | 0.0179 |
| SK-BR-3 | 4+ | 2+ | 0.002 | 0.287 | 0.0070 |
| CFPAC-1 | + | +/- | 0.015 | 3.000 | 0.005 |
| PANC-1 | +/- | +/- | 0.002 | 0.458 | 0.0044 |
| NCI-H226 | +/- | - | 0.006 | 1.570 | 0.00384 |

It can be seen from the above table that both LNCaP and SK-BR-3 express TRPV6 and FOLRH1, wherein LNCap has a relatively higher expression level of the two receptors. NCI-H226, CFPAC-1 and PANC-1 express the two receptors at a low or weak level. CB-1020 has an inhibitory effect on the growth and expansion of LNCaP, SK-BR-3, NCI-H226, CFPAC-1 and PANC-1 tumour cell lines. The IC₅₀ ratio of CB-1020 for cell line LNCaP with a relatively high expression of the two receptors is higher than the IC₅₀ ratio for cell line SK-BR-3 with a medium expression of the receptors; the IC₅₀ ratio for SK-BR-3 is higher than the IC₅₀ ratios for CFPAC-1 with a relatively low expression of the receptors and cell lines NCI-H226 and PANC-1 with a weak expression of the two receptors. The inhibitory effect on cell proliferation is positively correlated with the expression level of the two receptors in the cells.

### 11. Experiment of inhibiting tumour cell expansion in conjugate compound CB-1320

Experiment purpose: to test inhibitory effects of CB-1320 and related compounds on the expansion of tumour cell lines LNCaP (human prostate cancer cells), SK-BR-3 (human breast cancer cells), MDA-MB-468 (human breast cancer cells) and CFPAC-1 (pancreatic cancer cells).

### Related samples: MMAE and CB-1320

Experimental operations: LNCaP, SK-BR-3, MDA-MB-468 and CFPAC-1 cells were prepared in advance and counted; with a culture medium (IMDM + 10% FBS + IX L-Glutamine + IX P/S), the cells LNCaP, SK-BR-3, MDA-MB-468 and CFPAC-1 were diluted to 4 × 10⁴ cells/ml; the diluted cell solution was plated in 96-well plates at 100 µl/well; and negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The specific data is shown in the following table:

**Table 18. Inhibitory Effects of CB-1320 on Proliferation of Cell Lines and Receptor Gene Expression Levels in Cell Lines (with reference to data from Depmap)**

| Cell line | GNRHR gene expression level | FOLH1 gene expression level | IC₅₀ (MMAE) | IC₅₀ (CB-1320) | IC₅₀ (MMAE)/IC₅₀ (CB-1320) |
|---|---|---|---|---|---|
| LNCaP | +/- | 10+ | 0.003 | 0.060 | 0.043 |
| SK-BR-3 | +/- | 2+ | 0.002 | 0.093 | 0.016 |
| CFPAC-1 | +/- | +/- | 0.015 | 1.090 | 0.014 |
| MDA-MB-468 | +/- | +/- | 0.002 | 0.212 | 0.011 |

It can be seen from the above table that the expression levels of GNRHR in the 4 cell lines are roughly equivalent, and the expression level of FOLH1 in LNCaP is significantly higher than that in other cell lines. CB-1320 has an inhibitory effect on the expansion of 4 tumour cell lines. The inhibitory effects of CB-1320 on the cells with different expression levels of receptor FOLH1 are different. The IC₅₀ ratio in cell line LNCap with a relatively high expression of receptor FOLH1 is significantly higher than the IC₅₀ ratio in other cell lines with a relatively low expression of the receptor.

### 12. Experiment of inhibiting tumour cell expansion in conjugate compound CB-1820

Experiment purpose: to test inhibitory effects of CB-1820 and related compounds on the expansion of tumour cell lines LNCaP (human prostate cancer cells), MDA-MB-468 (human breast cancer cells), CFPAC-1 (pancreatic cancer cells) and PANC-1 (pancreatic cancer cells).

### Related samples: MMAE and CB-1820

Experimental operations: LNCaP, MDA-MB-468, CFPAC-1 and PANC-1 cells were prepared in advance and counted; with a culture medium (IMDM + 10% FBS + IX L-Glutamine + IX P/S), the cells LNCaP, MDA-MB-468 and CFPAC-1 were diluted to 4 × 10⁴ cells/ml, and PANC-1 was diluted to 3.0 × 10⁴ cells/ml; the diluted cell solution was plated in 96-well plates at 100 µl/well; and negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The specific values are shown in the following table:

**Table 19. Inhibitory Effects of CB-1820 on Proliferation of Cell Lines and Receptor Gene Expression Levels in Cell Lines (with reference to data from Depmap)**

| **Cell line** | **SSTR2 gene expression level** | **FOLH1 gene expression level** | **IC₅₀ (MMAE)** | **IC₅₀ (CB-1820)** | **IC₅₀ (MMAE)/IC₅₀ (CB-1820)** |
|---|---|---|---|---|---|
| LNCap | +/- | 10+ | 0.003 | 0.021 | 0.1429 |
| MDA-MB-468 | +/- | +/- | 0.002 | 0.062 | 0.0323 |
| CFPAC-1 | +/- | +/- | 0.015 | 0.356 | 0.0421 |
| PANC-1 | +/- | +/- | 0.002 | 0.055 | 0.0364 |

It can be seen from the above table that the expression levels of SSTR2 in LNCap, MDA-MB-468, CFPAC-1 and PANC-1 are roughly equivalent, and the expression level of FOLH1 in LNCaP is significantly higher than the expression level of FOLH1 in other cell lines. CB-1820 has an inhibitory effect on the expansion of 4 tumour cell lines. The inhibitory effects of CB-1820 on the cells with different expression levels of receptor FOLH1 are different. The IC₅₀ ratio in cell line LNCaP with a relatively high expression of the receptor is significantly higher than the IC₅₀ ratio in other cell lines with a relatively low expression of the receptor, and the inhibitory effects on cell proliferation is positively correlated with expression levels of cell-related receptor FOLH1.

### 13. Experiment of inhibiting tumour cell expansion in conjugate compounds CR19425, CR19426 and CR19428

Experiment purpose: to test inhibitory effects of CR19428, CR19425, CR19426 and related compounds on the expansion of tumour cell lines NCI-H226 (human lung cancer cells), CFPAC-1 (human pancreatic cancer cells) and MDA-MB-468 (human breast cancer cells).

### Related samples: SN-38, Dxd0017, CR19428, CR19425 and CR19426

Experimental operations: NCI-H226, CFPAC-1 and MDA-MB-468 cells were prepared in advance and counted; MDA-MB-468 and CFPAC-1 cells were plated at a density of 2 × 10⁴ cells/ml (100 µl/well) and NCI-H226 cells were plated at a density of 1 × 10⁴ cells/ml (100 (µl/well) in 96-well plates; and negative control and blank control wells were set on each plate. After adherent cell culture overnight, the diluted samples to be tested were added at 50 µl/well. The plates were placed in an incubator at 37°C, 5% CO₂ and cultured for 68-72 hours. To each well, 15 µl (10% of the liquid volume in a well) of a colour developing solution from CCK8 was added; and the plates were incubated at 37°C for 45-70 minutes and each plate was read at 450 nm on a microplate reader. The data was edited using SoftMax Pro and a 4-P fitting curve was plotted. The specific data is shown in the following table:

**Table 20. Inhibitory Effects of CR19428, CR19425 and CR19426 on Proliferation of Cell Lines**

| **Cell line** | **IC₅₀ (SN-38)** | **IC₅₀ (Dxd0017)** | **IC₅₀ (CR19428)** | **IC₅₀ (CR19425)** | **IC₅₀ (CR19426)** |
|---|---|---|---|---|---|
| **CFPAC-1** | 0.0055 | 0.0064 | 0.9060 | 0.6210 | 1.6600 |
| **MDA-MB-468** | 0.0136 | 0.0061 | 1.0700 | 0.9510 | 2.3000 |
| **NCI-H226** | 0.0173 | 0.0080 | 1.6200 | 1.0100 | 4.3900 |

### Example 5: Pharmacodynamic Study of Conjugate Compounds in CDX Models

### 1. Pharmacodynamic study 1 of CB-20BK in CDX models

### 1) Sample preparation:

CB-20BK lyophilized powder was weighed, dissolved with PBS and prepared into a sample mother solution, and the mother solution was diluted with a physiological saline for injection to a sample solution with a working concentration for later use.

### 2) CDX model construction

Main cell lines: KB human oral epidermoid carcinoma cells, MIA paca-2 human pancreatic cancer cells, HCC1954 human breast cancer cells, CALU-3 human lung adenocarcinoma cells and DU145 human prostate cancer cells.

Model construction: The cells were resuscitated, cultured, collected, counted, and subcutaneously injected into the right flank of BALB/c-nude mice. When the tumour grew and expanded to 80-160 mm³, grouping and administration were performed, or rapidly expanded tumour masses were transferred and injected to mice for scale up.

### 3) Grouping, administration and observation

Grouping: When the tumour grew to about 80-160 mm³ on average, a grouping was performed, and a model control group and different doses of administration groups were set.

Administration: The mice were administrated via tail vein injection.

Experimental observation and measurement: After tumour inoculation, the effects of tumour growth and treatment on normal behaviours of animals, specifically including the activity of experimental animals, food intake and drinking status, weight gain or loss (measuring weight twice a week), and other abnormal conditions in eyes, hair, etc., were conventionally monitored.

The weight of mice and the long diameter (a) and short diameter (b) of tumour mass were measured twice a week. The formula for the calculation of tumour volume (TV) is: TV = 1/2 × a × b².

### 4) Experimental results and analysis

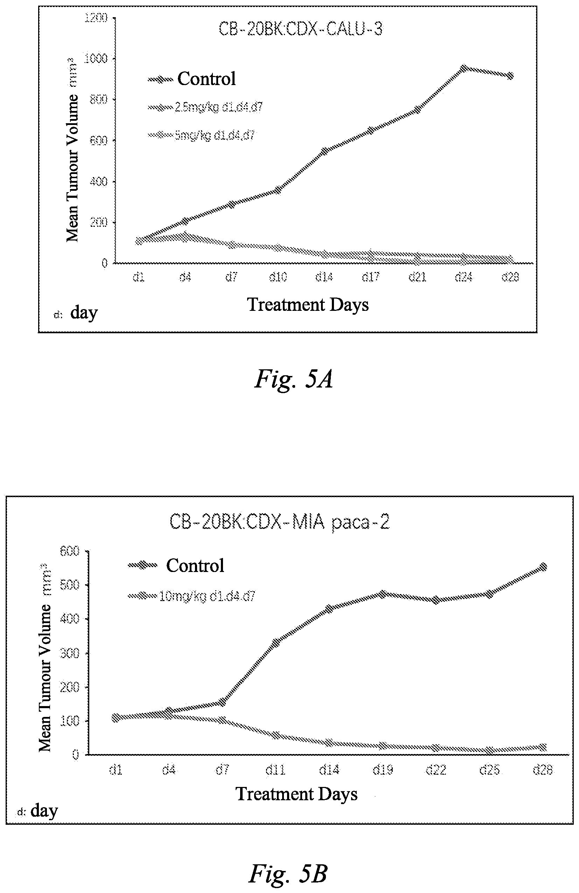

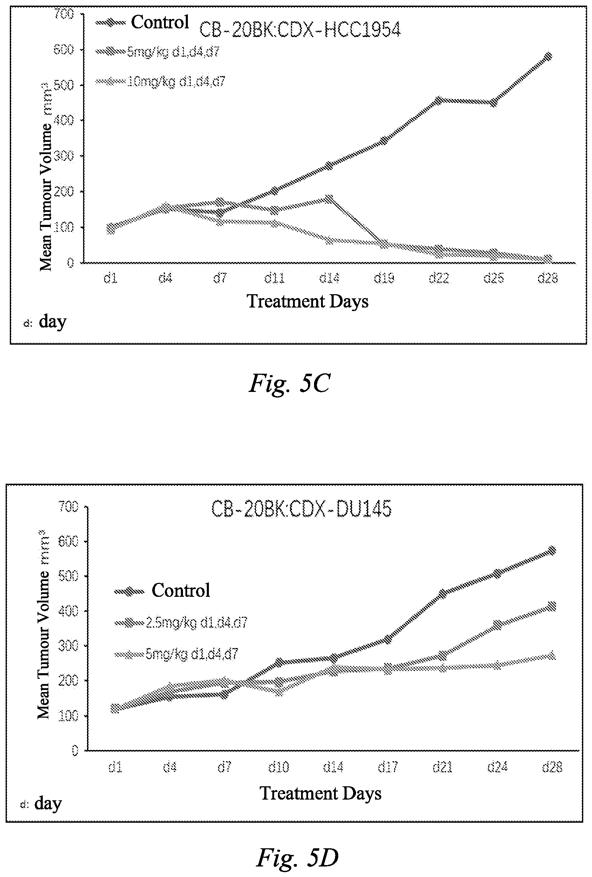

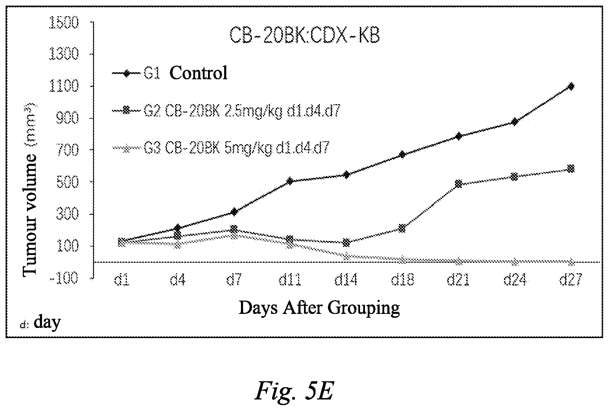

According to changes in tumour volumes of mice (Figs. 5A-5E), it can be seen that CB-20BK has a good inhibitory effect in CDX tumour models of KB, MIA aca-2, HCC1954, CALU-3 and DU145 cell lines in mice.

### 2. Pharmacodynamic study 2 of CB-20BK in CDX models

Experiment purpose: to perform a pharmacodynamic study of conjugate compound CB-20BK in a subcutaneous xenograft model (CDX) of humanized LNCaP, DU145 and NCI-H460 cell lines

Main CDX models: LNCaP, DU145 and NCI-H460

Experimental scheme:
The cells or tumour tissue mass were prepared and subcutaneously inoculated into the anterior right flank of BALB/c-nude mice. When the tumour volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumour growth and treatment on normal behaviours of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumour were measured with a vernier calliper, and the tumour volume, relative tumour proliferation rate, tumour volume inhibition rate and other indicators were calculated. The formula of tumour volume is TV = 0.5 a × b², wherein a is the long diameter of a tumour and b is the short diameter of a tumour.

**Table 21. Inhibitory Effects of CB-20BK on Growth in CDX models and Receptor Gene Expression Levels in Each Model (with reference to data from Crown Bioscience Inc.)**

| Model | FOLR1 | FOLH1 | Tumour growth inhibition rate (TGI) (%) | Administration dosage/time |
|---|---|---|---|---|
| LNCaP | 5+ | 10+ | 95.68 | 3 mg/kg D1, 8, 15 |
| DU145 | - | - | 52 | 5 mg/kg D1, 4, 7 |
| NCI-H460 | - | - | 32 | 10 mg/kg D1, 8, 15 |

It can be seen from the above table that the test sample CB-20BK shows different degrees of tumour growth inhibition effects with a regimen of administration via tail vein. For CDX models of humanized LNCaP, DU145 and NCI-H460 cell lines, the test sample has a certain anti-tumour growth effect compared with a negative control group. In the LNCaP model with dual expression of FOLR1 and FOLH1, the test sample was administered on day 1, day 8 and day 15 (D1, D8 and D15) at a dose of 3 mg/kg, has a TGI value of 95.68%, shows an excellent anti-tumour growth effect, which is significantly better than that in DU145 and NCI-H460 models with a relatively low expression of FOLR1 and FOLH1. The tumour growth inhibition effect has a certain correlation with the expression level of cell-related receptors.

### 3. Tumour growth inhibition experiment 1 of conjugate compound CB-20BK in HuPrime® xenograft PDX models

Experiment purpose: Pharmacodynamic study of conjugate compound CB-20BK in PDX models

Main models: LU1206, LU1380 and LU0367

Experimental scheme: The tumour tissue mass were prepared and subcutaneously inoculated into the anterior right flank of BALB/c-nude mice. When the tumour volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g and an administration dosage of 3 mg/kg. After grouping and administration, the effects of tumour growth and treatment on normal behaviours of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumour were measured with a vernier calliper, and the tumour volume, relative tumour proliferation rate, tumour volume inhibition rate and other indicators were calculated. The formula of tumour volume is TV = 0.5 a × b², wherein a is the long diameter of a tumour and b is the short diameter of a tumour.

**Table 22. Inhibitory Effects of CB-20BK on Growth in Lung Cancer PDX Models and Receptor Gene Expression Levels in Each Model (with reference to data from Crown Bioscience Inc.)**

| Model | FOLR1 gene expression level | FOLH1 gene expression level | TGI (%) | Dosage |
|---|---|---|---|---|
| LU1206 | 4+ | 2+ | 90.89 | 3 mg/kg |
| LU1380 | - | 4+ | 30.02 | 3 mg/kg |
| LU0367 | - | 5+ | 71.34 | 3 mg/kg |

It can be seen from the data in Table 22 that the test sample CB-20BK (3 mg/kg) shows a certain anti-tumour effect on LU1206, LU1380 and LU0367 humanized lung cancer PDX models. The TGI value in the LU1206 model with dual expression of FOLR1 and FOLH1 is 90.89%, and the TGI values in LU1380 and LU0367 models with a single expression are 30.02% and 71.34%, respectively. In the tumour growth inhibition experiment, the tumour growth inhibition effect has a certain correlation with the expression level of cell-related receptors.

### 4. Tumour growth inhibition experiment 2 of conjugate compound CB-20BK in HuPrime® xenograft PDX models

Experiment purpose: Pharmacodynamic study of conjugate compound CB-20BK in PDX models

Main models: BR1283 and BR0438

Experimental scheme: The tumour tissue mass were prepared and subcutaneously inoculated into the anterior right flank of BALB/c-nude mice. When the tumour volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g and an administration dosage of 3 mg/kg. After grouping and administration, the effects of tumour growth and treatment on normal behaviours of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumour were measured with a vernier calliper, and the tumour volume, relative tumour proliferation rate, tumour volume inhibition rate and other indicators were calculated. The formula of tumour volume is TV = 0.5 a × b², wherein a is the long diameter of a tumour and b is the short diameter of a tumour.

**Table 23. Inhibitory Effects of CB-20BK on Growth in Breast Cancer PDX Models and Receptor Gene Expression Levels in Each Model (with reference to data from Crown Bioscience Inc.)**

| Model | FOLR1 gene expression level | FOLH1 gene expression level | TGI (%) | Dosage |
|---|---|---|---|---|
| BR1283 | 7+ | 6+ | 96.49 | 3 mg/kg |
| BR0438 | 5+ | 4+ | 70.74 | 3 mg/kg |

It can be seen from the above table that the TGI values of the test sample CB-20BK at a dose of 3 mg/kg in BR1283 and BR0438 models with dual expression of FOLR1 and FOLH1 are 96.49% and 70.74%, respectively, showing an excellent anti-tumour growth effect. Moreover, the TGI of CB-20BK is higher in BR1283 with a higher expression of FOLR1 and FOLH1.

### 5. Pharmacodynamic study of CB-20B in CDX models

### 1) Sample preparation:

CB-20B lyophilized powder was weighed, dissolved with PBS and prepared into a sample mother solution, and the mother solution was diluted with a physiological saline for injection to a sample solution with a working concentration for later use.

### 2) CDX model construction

Main cell lines: KB human oral epidermoid carcinoma cells, PC-9 human lung cancer cells and DU145 human prostate cancer cells.

Model construction: The cells were resuscitated, cultured, collected, counted, and subcutaneously injected into the right flank of BALB/c-nude mice. When the tumour grew and expanded to 80-160 mm³, grouping and administration were performed, or rapidly expanded tumour masses were transferred and injected to mice for scale up.

### 3) Grouping, administration and observation

Grouping: When the tumour grew to about 80-160 mm³ on average, a grouping was performed, and a model control group and different doses of administration groups were set.

Administration: The mice were administrated via tail vein injection.

Experimental observation and measurement: After tumour inoculation, the effects of tumour growth and treatment on normal behaviours of animals, specifically including the activity of experimental animals, food intake and drinking status, weight gain or loss (measuring weight twice a week), and other abnormal conditions in eyes, hair, etc., were conventionally monitored.

The weight of mice and the long diameter (a) and short diameter (b) of tumour mass were measured twice a week. The formula for the calculation of tumour volume (TV) is: TV = 1/2 × a × b².

### 4) Experimental results and analysis

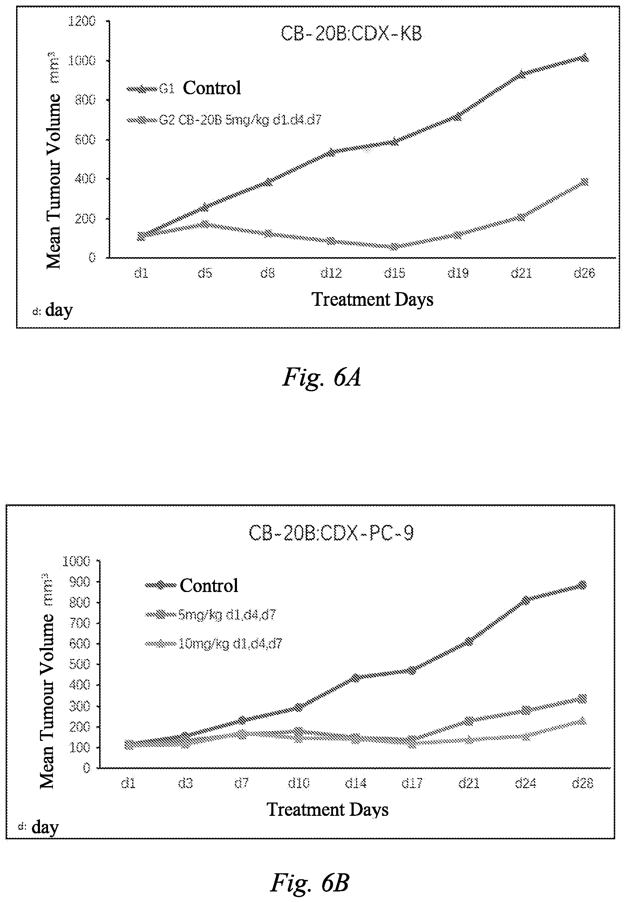

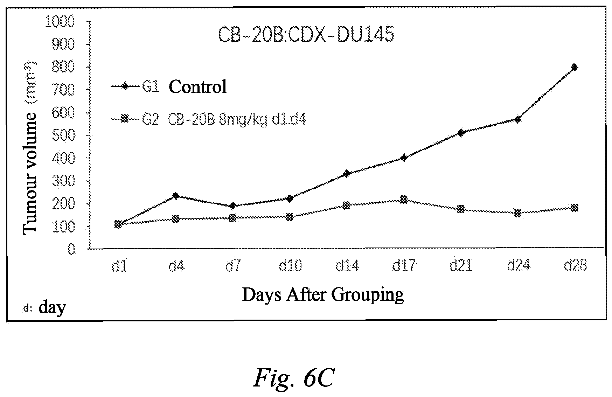

According to changes in tumour volume of mice (Figs. 6A-6C), it can be seen that CB-20B has a good inhibitory effect in CDX tumour models of KB, PC-9 and DU145 cell lines in mice.

### 6. Pharmacodynamic study of CB-18G in CDX models

### 1) Sample preparation:

CB-18G lyophilized powder was weighed, dissolved with PBS and prepared into a sample mother solution, and the mother solution was diluted with a physiological saline for injection to a sample solution with a working concentration for later use.

### 2) CDX model construction

Main cell lines: KB human oral epidermoid carcinoma cells, PC-9 human lung cancer cells, SPC-A1 human lung adenocarcinoma cells, CALU-3 human lung adenocarcinoma cells and DU145 human prostate cancer cells

Model construction: The cells were resuscitated, cultured, collected and counted, and the cell solution was subcutaneously injected into the right flank of BALB/c-nude mice. When the tumour grew and expanded to 80-160 mm³, grouping and administration were performed, or rapidly expanded tumour masses were transferred and injected to mice for scale up.

### 3) Grouping, administration and observation

Grouping: When the tumour grew to about 80-160 mm³ on average, a grouping was performed, and a model control group and different doses of administration groups were set.

Administration: The mice were administrated via tail vein injection.

Experimental observation and measurement: After tumour inoculation, the effects of tumour growth and treatment on normal behaviours of animals, specifically including the activity of experimental animals, food intake and drinking status, weight gain or loss (measuring weight twice a week), and other abnormal conditions in eyes, hair, etc., were conventionally monitored.

The weight of mice and the long diameter (a) and short diameter (b) of tumour mass were measured twice a week. The formula for the calculation of tumour volume (TV) is: TV = 1/2 × a × b².

### 4) Experimental results and analysis

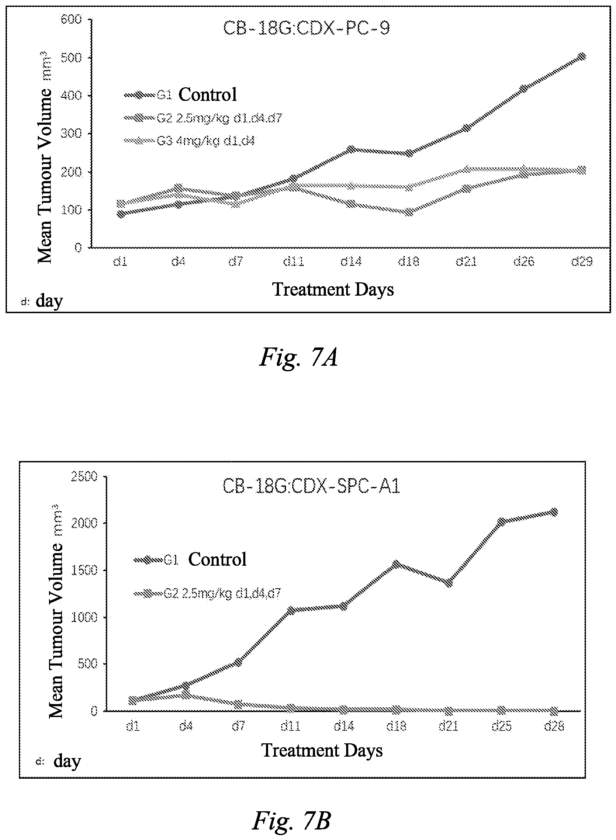

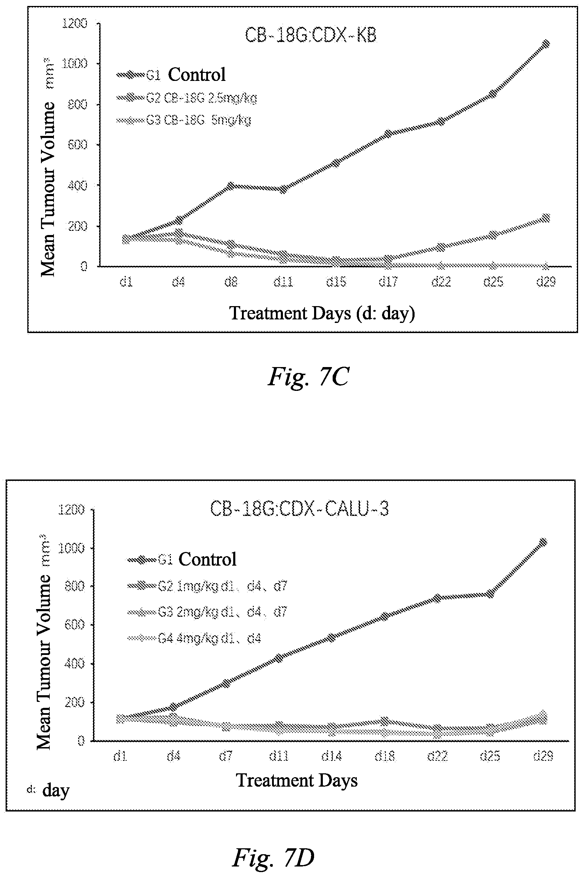

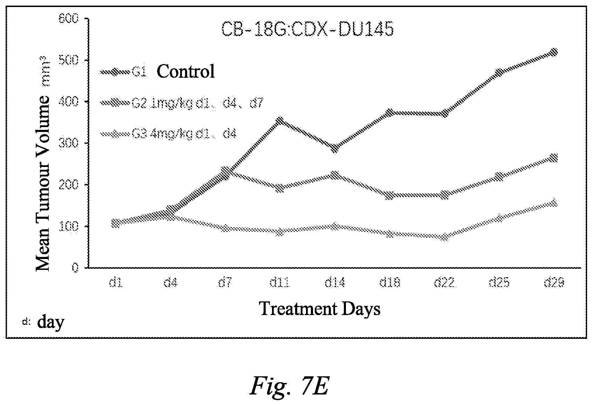

According to changes in tumour volumes of mice (Figs. 7A-7E), it can be seen that CB-18G has a good inhibitory effect in CDX tumour models of KB, PC-9, SPC-A1, CALU-3 and DU145 cell lines in mice.

### 7. Tumour growth inhibition experiment of conjugate compounds CB-1020, CB-1320 and CB-1820 in subcutaneous xenograft model (CDX) of humanized HPAF-II, NCI-H226 and SCLC-21H cell lines

Experiment purpose: Pharmacodynamic study of ligand conjugates CB-1020, CB-1320 and CB-1820 in CDX models

Main CDX models: HPAF-II (human pancreatic cancer cells), NCI-H226 (human lung cancer cells) and SCLC-21H (small cell lung cancer cells)

Experimental scheme:
The cells or tumour tissue mass were prepared and subcutaneously inoculated into the anterior right flank of BALB/c-nude mice. When the tumour volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 µl/g. After grouping and administration, the effects of tumour growth and treatment on normal behaviours of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumour were measured with a vernier calliper, and the tumour volume, relative tumour proliferation rate, tumour volume inhibition rate and other indicators were calculated. The formula of tumour volume is TV = 0.5 a × b², wherein a is the long diameter of a tumour and b is the short diameter of a tumour.

**Table 24. Inhibitory Effects of CB-1020, CB-1320 and CB-1820 on Growth in CDX Models and Receptor Gene Expression Levels in Each Model (with reference to data from Crown Bioscience Inc.)**

| Model | TRPV6 gene expression level | GNRHR gene expression level | SSTR2 gene expression level | FOLH1 gene expression level | TGI (%) (CB-1020) | TGI (%) (CB-1320) | TGI (%) (CB-1820) | P value | Dosage |
|---|---|---|---|---|---|---|---|---|---|
| HPAF-II | + | - | +/- | +/- | 0.283 | | | 0.461 | 3 mg/kg |
| | | | | | 0.445 | | | 0.153 | 5 mg/kg |
| | | | | | 0.899 | | | 0.023 | 10 mg/kg |
| NCIH226 | +/- | +/- | +/- | - | | | 0.823 | 0.011 | 3 mg/kg |
| | | | | | | 0.962 | | 0.006 | 3 mg/kg |
| | | | | | | 0.992 | | 0.005 | 10 mg/kg |
| SCLC-21H | 2+ | +/- | 3+ | - | | | 0.995 | 0.131 | 3 mg/kg |
| | | | | | | 0.361 | | 0.610 | 3 mg/kg |
| | | | | | | 0.985 | | 0.134 | 10 mg/kg |

CB-1020 has an obvious anti-tumour growth effect in subcutaneous xenograft models of humanized HPAF-II cell lines; CB-1320 has an obvious anti-tumour growth effect in subcutaneous xenograft models of NCI-H226 and SCLC-21H cell lines; and CB-1820 has an obvious anti-tumour growth effect in subcutaneous xenograft models of SCLC-21H cell lines.

### 8. Tumour growth inhibition experiment of conjugate compound CR19428 in subcutaneous xenograft model (CDX) of humanized CALU-3, SCLC-21H and SPC-A1 cell lines

Experimental scheme: The tumour tissue mass were prepared and subcutaneously inoculated into the anterior right flank of BALB/c-nude mice. When the tumour volume expanded to 80-160 mm³, a randomized grouping was performed, and a model control group and administration groups were set. The mice were administrated from the first day of grouping, wherein the administration dose was adjusted according to the latest weight measurement. The mice were administrated via tail vein injection at an administration volume of 10 (µl/g and twice a week for consecutive 3 weeks. After grouping and administration, the effects of tumour growth and treatment on normal behaviours of animals, specifically including the activity of experimental animals, food intake and drinking status, weight gain or loss status, and other abnormal conditions in eyes, hair, etc., were monitored. After grouping and administration, the weight of mice was measured twice a week to calculate the rate of weight change; at the same time, the long diameter and short diameter of the tumour were measured with a vernier calliper, and the tumour volume, relative tumour proliferation rate, tumour volume inhibition rate and other indicators were calculated. The formula of tumour volume is TV = 0.5 a × b², wherein a is the long diameter of a tumour and b is the short diameter of a tumour.

**Table 25. Inhibitory Effects of CR19428 in Lung Cancer PDX models**

| Model | TGI (%) | Administration dosage/time |
|---|---|---|
| CALU-3 | 51 | 50 mg/kg q2/w*3 |
| SCLC-21H | 66 | 50 mg/kg q2/w*3 |
| SPC-A1 | 75 | 50 mg/kg q2/w*3 |
| SPC-A1 | 91 | 100 mg/kg q2/w*3 |

CR-19428 is a drug conjugate of ligands FOLR1 and FOLH1 and a payload Dxd. It can be seen from the above table that the drug conjugate has an obvious anti-tumour growth effect in subcutaneous xenograft models of humanized CALU-3, SCLC-21H and SPC-A1 cell lines.

### 9. Study on effectiveness of conjugate compound CBP-1018 in lung cancer LU2505 model (PDX model)

The 3rd generation of lung cancer PDX model LU2505 (from Asian female patients), which is a rapid-growing tumour model, was used in this experiment. BALB/c nude mice bore tumours subcutaneously and, when the tumour volume reached about 150 mm³, were grouped into: low-dose, medium-dose and high-dose test sample groups, a small molecule MMAE control group, a targeting polypeptide 20BK-SM09 control group, and a blank control group (a total of 6 groups, and 8 mice per group); the mice were administered on day 1, day 8 and day 15 after the grouping; and the observation was continued for 14 days after the last administration. The active substance of test sample CBP-1018 is CB-20BK, which is obtained by mixing CB-20BK with auxiliary materials and then lyophilizing same.

**Table 26. Pharmacodynamic Results of CBP-1018 for Injection in Lung Cancer LU2505 models (first experiment)**

| **Test sample /Control sample** | **Dosage (mg/kg)** | **Weight (g)** | **Tumour volume (mm³)** | **Tumour growth inhibition rate TGI%** | **Tumour weight (mg)** |
|---|---|---|---|---|---|
| Blank control group | / | 24.0 | 2219.62 | / | 2031.4 (D22) |
| CBP-1018 | 4.5 | 22.5 | 0.00 | 100.00 | 0.0 (D29) |
| | 1.5 | 22.1 | 386.16 | 82.60 | 400.7 (D29) |
| | 0.5 | 23.6 | 1634.50 | 26.36 | 2084.7 (D26) |
| 20BK-SM09 | 10 | 23.8 | 2024.75 | 8.78 | 1811.5 (D22) |
| MMAE | 0.375 | 23.5 | 1132.61 | 48.97 | 1597.1 (D29) |

Notes: 1) Due to requirements of animal welfare, animals in a group must be euthanized when the mean tumour volume in the group reaches 2000 mm³, leading to different execution time for each group. 2) The data of weight, tumour volume, and tumour growth inhibition rate is the data obtained on day 22 (D22).

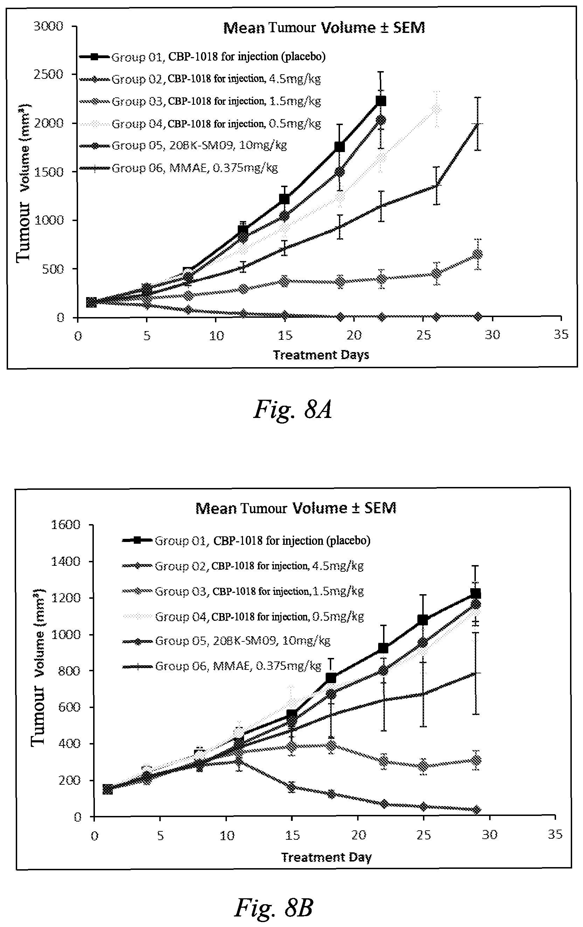

As shown in Table 26 and Fig. 8A:
animals in all groups show no abnormal clinical manifestations and no deaths after administration; and the weight of the animals in each group increases slowly.

The animals in the blank control group, 20BK-SM09 group, and low-dose CBP-1018 group were euthanized on day 22, day 22, and day 26 (D26) because of the mean tumour volume exceeding 2000 mm³. Therefore, the data of weight, tumour volume, and tumour growth inhibition rate in Table 26 is the data obtained on D22.

The effectiveness of CBP-1018 for injection has an obvious dose correlation, wherein CBP-1018 for injection was ineffective in the low-dose group, and effective in the medium-dose group and the high-dose group. The tumour in the high-dose group was completely cured on day 19 (D19), and no signs of tumour growth were seen on day 29 (D29).

The polypeptide 20BK-SM09 group showed no obvious tumour growth inhibition effect, suggesting that a single targeting polypeptide is not enough to produce a clear anti-tumour effect.

The MMAE group showed a clear tumour growth inhibition effect. Equimolar MMAE is contained in MMAE at 0.375 mg/kg and in CBP-1018 at 1.5 mg/kg. It can be seen from the comparison that the tumour growth inhibition effect of CBP-1018 at 1.5 mg/kg is significantly better than that in the MMAE group, suggesting the advantage of ligand targeting (tumour growth inhibition rate: 82.60% vs 48.97%).

### 10. Study on effectiveness of conjugate compound CBP-1018 in lung cancer LU1206 model (PDX model)

The 5th generation of lung cancer PDX model LU1206 (from Asian female patients), which is a rapid-growing tumour model, was used in this experiment. BALB/c nude mice bore tumours subcutaneously and, when the tumour volume reached about 150 mm³, were grouped into: low-dose, medium-dose and high-dose test sample groups, a small molecule MMAE control group, a targeting polypeptide 20BK-SM09 control group, and a blank control group (a total of 6 groups, and 8 mice per group); the mice were administered on day 1, day 8 and day 15 after the grouping; and the observation was continued for 14 days after the last administration.

**Table 27. Pharmacodynamic Results of CBP-1018 for Injection in Lung Cancer LU1206 models (first experiment)**

| Groups | **Dosage (mg/kg)** | **Weight (g)** | **Tumour volume (mm³)** | **Tumour volume Tumour growth inhibition rate (%)** | **Tumou r weight (mg)** | **Tumour weight Inhibition ratio (%)** |
|---|---|---|---|---|---|---|
| Blank control group | / | 22.7 | 1216.67 | / | 856.14 | / |
| CBP-1018 | 4.5 | 23.4 | 31.44 | 97.42 | 13.55 | 98.42 |
| | 1.5 | 23.7 | 301.64 | 75.21 | 185.10 | 78.38 |
| | 0.5 | 22.7 | 1118.30 | 8.08 | 852.19 | 0.46 |
| 20BK-SM09 | 10 | 23.3 | 1158.80 | 4.76 | 914.36 | -6.80 |
| MMAE | 0.375 | 23.6 | 778.32 | 36.03 | 437.73 | 54.53 |

As shown in Table 27 and Fig. 8B:
animals in all groups show no abnormal clinical manifestations and no deaths after administration, and all animals were euthanized on day 29. The weight of the animals in each group remained basically unchanged, and is slightly higher than that on the first day of administration.

The effectiveness of CBP-1018 for injection has an obvious dose correlation, wherein CBP-1018 for injection was ineffective in the low-dose group (with a tumour growth inhibition rate of 8.08%), and effective in the medium-dose group and the high-dose group, with a tumour growth inhibition rate of 75.21% and 97.42%, respectively. The difference in effectiveness between the low-dose group and the medium-dose group is relatively large.

The polypeptide 20BK-SM09 group showed no obvious tumour growth inhibition effect, suggesting that a single targeting polypeptide is not enough to produce a clear anti-tumour effect in this model.

The MMAE group showed a clear tumour growth inhibition effect. Equimolar MMAE is contained in MMAE at 0.375 mg/kg and in CBP-1018 at 1.5 mg/kg. It can be seen from the comparison that the tumour growth inhibition effect of CBP-1018 at 1.5 mg/kg is significantly better than that in the MMAE group, suggesting the advantage of ligand targeting (tumour volume inhibition rate: 75.21% vs 36.03%; tumour weight inhibition rate: 78.38% vs 54.53%).

### 11. Tissue distribution of tumour-bearing mice (PDX model LU2505)

12 female tumour-bearing mice inoculated with tumour mass of an HuPrime® lung cancer LU2505 model and 12 healthy male BALB/c nude mice were given 1.5 mg/140 µCi/kg of [³H]CBP-1018 (isotope labelling on MMAE) via single tail vein injection. At 0.5 hours, 2 hours, 6 hours and 24 hours respectively, 3 male mice and 3 female mice were placed in an induction box and anesthetized by inhaling an appropriate amount of carbon dioxide; and blood was collected by cardiac puncture, and then euthanasia was carried out immediately to collect samples.

**Table 28. Total Radioactivity in Various Tissues at Different Time Points after Single Intravenous Administration of [³H]CBP-1018**

| **Tissue** | **Radioactivity concentration (ng Eq./g)** | | | | | | | |
|---|---|---|---|---|---|---|---|---|
| | 0.5 hours | | 2 hours | | 6 hours | | 24 hours (% Cₘₐₓ) | |
| | ♂ | ♀ | ♂ | ♀ | ♂ | ♀ | ♂ | ♀ |
| **Tumour** | **763** | **/** | **643** | **/** | **566** | **/** | **413** | / |
| Oesophagus | 915 | 784 | 628 | 508 | 521 | 172 | 286 | 91.7 |
| Body fat | 341 | 479 | 288 | 276 | 231 | 177 | 67.2 | 43.7 |
| Skeletal muscle | 321 | 311 | 207 | 137 | 188 | 95.5 | 90.8 | 61.5 |
| Spleen | 744 | 693 | 701 | 524 | 671 | 421 | 253 | 163 |
| Stomach wall | 658 | 623 | 414 | 370 | 460 | 259 | 211 | 123 |
| Whole brain | 66.3 | 67.9 | 53.8 | 39.5 | 82.0 | 48.2 | 76.4 | 68.3 |
| Testicular epididymis | 350 | 494 | 279 | 298 | 183 | 248 | 92.3 | 64.8 |
| Heart | 785 | 795 | 415 | 326 | 219 | 143 | 114 | 69.2 |
| **Lung** | **1493** | **1807** | 997 | 920 | 416 | 392 | 109 | 93.7 |
| **Kidney** | **5682** | **6118** | **3300** | **4197** | **1227** | **1391** | **241** | **238** |
| **Liver** | **1423** | **1306** | **1257** | **865** | **1138** | **599** | **712** | **518** |
| Small intestine wall | 726 | 778 | 641 | 816 | 490 | 334 | 210 | 90.9 |
| Large intestine wall | 550 | 520 | 672 | 823 | 1074 | 490 | 215 | 128 |
| **Whole blood** | **2087** | **3194** | **684** | **691** | **211** | **132** | **86.5** | **64.6** |
| Plasma | 7372 | 7610 | 1844 | 1760 | 495 | 306 | 214 | 140 |

It can be seen from the above table that the distribution in animal tissues shows no difference between male and female; after administration, the drug is mainly distributed in kidney, whole blood (mainly distributed in plasma), liver and lung; in all tissues, the highest concentration appears at 0.5 hours, and then the drug is eliminated rapidly, wherein the slowest elimination appears in liver and tumour; and the slow elimination in tumour can explain the advantages of CBP-1018 in terms of effectiveness.

### 12. Excretion experiment in rats

Six normal SD rats, half male and half female, were given 0.75 mg/70 µCi/kg of [³H]CBP-1018 via single tail vein injection. At designated time intervals, samples such as urine, faeces, cage flushing/cleaning solution and corpses of integral rats were collected before administration and 0-168 hours after administration, and samples such as bile, urine, faeces and cage flushing/cleaning solution of BDC rats were collected before administration and 0-72 hours after administration. The above-mentioned samples were cryopreserved in a low-temperature refrigerator (-10°C to -30°C).

To each sample, an appropriate amount of scintillation fluid was added and uniformly mixed, and then the amount of radioactivity was measured using a liquid scintillation counter. The amount of radioactivity measured in samples such as bile, urine, faeces, cage flushing/cleaning solution and corpses was used to calculate the percentage in the given dose. The amount of radioactivity in the plasma sample was used to calculate the total radioactivity in each gram of the sample. The main pharmacokinetic parameters of the total plasma radioactivity were calculated using WinNonLin software (version 7.0, Pharsight) according to a non-compartmental model.

**Table 29. Results of Mass Balance Study on Integral Rats After Administration**

| Sex (number of rats) | Urine (%) | Faeces (%) | Cage flushing/cleaning solution (%) | Corpse (%) | Total recovery (%) |
|---|---|---|---|---|---|
| Female (n = 3) | 50.26 ± 6.11 | 23.93 ± 0.98 | 5.70 ± 2.63 | 4.51 ± 1.19 | 84.40 ± 4.02 |
| Male (n = 3) | 53.60 ± 3.68 | 20.61 ± 1.74 | 4.24 ± 2.16 | 4.83 ± 0.42 | 83.28 ± 0.71 |
| Female and male (n = 3 female and 3 male) | 51.93 ± 4.87 | 22.27 ± 2.21 | 4.97 ± 2.30 | 4.67 ± 0.82 | 83.84 ± 2.65 |

It can be seen from the above table that CBP-1018 is mainly excreted in urine, which accounts for about 57% of the total given dose, and the amount excreted in faeces accounts for less than 25%. The result of mainly excretion in urine through the kidney is consistent with the finding of large distribution in the kidney in the tissue distribution of nude mice.

### 13. Plasma stability

CBP-1008 (the active substance thereof is LDC10B described in WO 2017025047 A1, and CBP-1008 is obtained by mixing LDC10B with auxiliary materials and lyophilizing same; WO 2017025047A1 is incorporated by reference in its entirety) and CBP-1018 at a concentration of 1 µg/mL, 10 µg/mL and 100 µg/mL were incubated with plasma of different species at 37°C for 2 hours to observe the stability of the two compounds. The results show (in Table 30) that CBP-1018 is stable in plasma of various species, and the remaining percentage thereof after 2 hours of incubation is above 90% relative to the concentration at 0 hours. However, CBP-1008 is only stable in plasma of rats (with 87.92%-91.82% remaining), and is unstable in plasma of other species, with only 0.751%-24.52% remaining.

The experimental results suggest that the plasma stability of CBP-1018 is better than that of CBP-1008.

**Table 30. Summary of Plasma Stability Data of CBP-1008 and CBP-1018 at Different Concentrations in Various Species (% relative to 0 hours)**

| Compound | Concentration | Mouse | Rat | Cynomolgus monkey | Human |
|---|---|---|---|---|---|
| CBP-1008 | 1 µg/mL | 0.805 | 87.92 | 0.961 | 0.751 |
| | 10 µg/mL | 1.27 | 91.47 | 0.988 | 0.807 |
| | 100 µg/mL | 24.52 | 91.82 | 1.10 | 0.953 |
| CBP-1018 | 1 µg/mL | 94.4 | 92.2 | 97.3 | 99.1 |
| | 10 µg/mL | 96.5 | 95.0 | 95.8 | 102 |
| | 100 µg/mL | 98.1 | 94.8 | 101 | 103 |

Notes:
1) The data in the table is the percentage of the compound concentration in plasma after plasma incubation for 2 hours relative to 0 hours.
2) Since the data in the table is obtained from two experiments, the effective digits after the decimal point are inconsistent. For the traceability and authenticity, the data in the report is directly referred without uniformity.

### 14. Half-life in PK

Consistent with the comparison of plasma stability, the pharmacokinetic half-life of CBP-1018 (about 1 hour) is significantly longer than that of CBP-1008 (about 20 minutes) in rats and cynomolgus monkeys, suggesting that CBP-1018 is more stable than CBP-1008.

**Table 31. Comparison of Half-life (in hour) of CBP-1008 and CBP-1018 in PK of Rats**

| | **0.15 mg/kg** | | **0.5 mg/kg** | | **1.5 mg/kg** | |
|---|---|---|---|---|---|---|
| | **Male** | **Female** | **Male** | **Female** | **Male** | **Female** |
| **CBP-1008** | 0.245 | 0.215 | 0.249 | 0.202 | 0.222 | 0.368 |
| **CBP-1018** | 0.928 | 0.833 | 1.37 | 0.952 | 1.49 | 1.51 |

## Claims

1. A conjugate compound or a pharmaceutically acceptable salt thereof, comprising a payload and two targeting molecules, wherein the two targeting molecules are a synergistic molecule moiety and a prostate-specific membrane antigen ligand moiety, respectively.

2. The conjugate compound or the pharmaceutically acceptable salt thereof according to claim 1, wherein the prostate-specific membrane antigen ligand has the following structure:

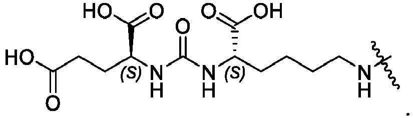

3. The conjugate compound or the pharmaceutically acceptable salt thereof according to claim 1, wherein the prostate-specific membrane antigen ligand has the following structure:

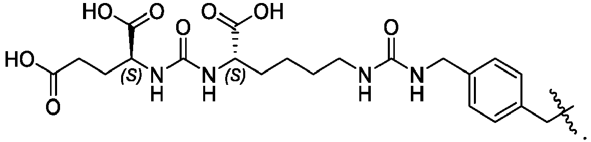

4. The conjugate compound or the pharmaceutically acceptable salt thereof according to claim 1, wherein the prostate-specific membrane antigen ligand has the following structure:

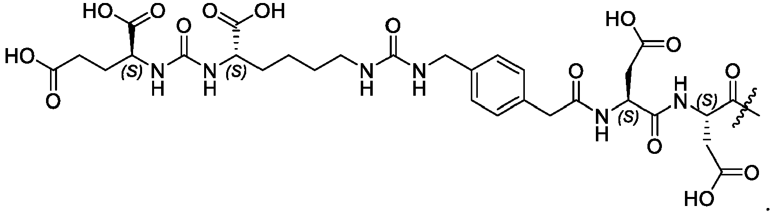

5. The conjugate compound or the pharmaceutically acceptable salt thereof according to claim 1, wherein the prostate-specific membrane antigen ligand has the following structure:

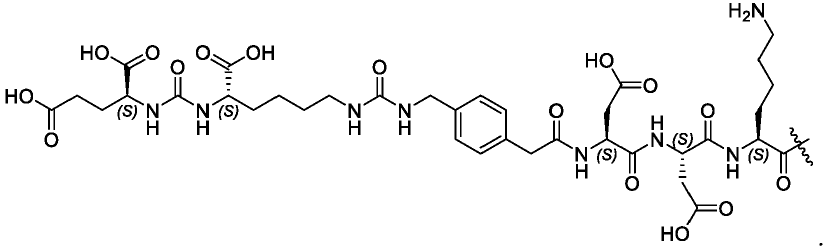

6. A conjugate compound or a pharmaceutically acceptable salt thereof, comprising a payload and two targeting molecules, wherein the two targeting molecules are a synergistic molecule moiety and a ligand moiety represented by formula (I), respectively:

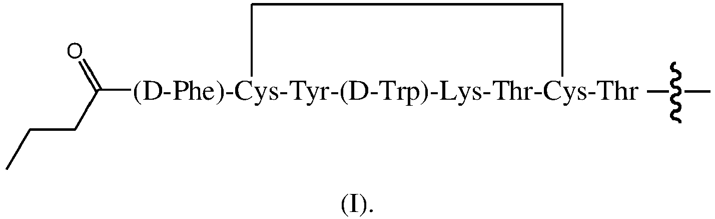

7. A conjugate compound or a pharmaceutically acceptable salt thereof, comprising a payload and two targeting molecules, wherein the two targeting molecules are a synergistic molecule moiety and P10, respectively, and the payload is camptothecin or any derivative thereof.

8. The conjugate compound or the pharmaceutically acceptable salt thereof according to any one of claims 1-7, wherein the two targeting molecules are different.

9. The conjugate compound or the pharmaceutically acceptable salt thereof according to any one of claims 1-8, wherein the synergistic molecule is a cell-interacting molecule.

10. The conjugate compound or the pharmaceutically acceptable salt thereof according to any one of claims 1-9, wherein the two targeting molecules are cell-interacting molecules that interact with different cell molecules.

11. The conjugate compound or the pharmaceutically acceptable salt thereof according to any one of claims 1-10, wherein the synergistic molecule is an endocytosis molecule capable of mediating endocytosis.

12. The conjugate compound or the pharmaceutically acceptable salt thereof according to any one of claims 1-11, wherein the synergistic molecule binds to a molecule selected from the group consisting of FOLR1, TRPV6, FOLH1 (PMSA), GNRHR, Her2, Trop2, Her3, NECTIN4, LRP1, GLUT1, EGFR1, AXL, CA9, CD44, Claudin18.2, APN, DLL3, CEACAM5, FZD10, TFRC, MET, IGFR1, SSTR2, CCKBR, LFA1, ICAM, GPR87, GM-CSF, GM-CSFR, TIM3, TLR family, CD40, CD40L, OX40, OX40L, GITRL, GITR, 4-BBL, 4-1BB, CD70, CD27, ICOSL, ICOS, HHLA2, CD28, CD86/80, CD28, MHCII antigen, TCR, CTLA-4, CD155, CD122, CD113, IGIT, PD-L1, PD1, Galectin-9, TIM-3, HVEM, BTLA, CD160, VISTA, B7-H4, B7-H3, phosphatidylserine, HHLA2, LAG3, Galectin-3, LILRB4, SIGLEC15, NKG2A, NKG2D, SLAMF7, KIR2DL1, KIR2DL2, KIR2DL3, FGFR1, FGFR2, FGFR4, NeuGcGM3 and CXCR4.

13. The conjugate compound or the pharmaceutically acceptable salt thereof according to any one of claims 1-12, wherein the synergistic molecule binds to a molecule selected from the group consisting of FOLR1, TRPV6, FOLH1 (PMSA), SSTR2 and GNRHR.

14. The conjugate compound or the pharmaceutically acceptable salt thereof according to claim 1, wherein the synergistic molecule binds to a molecule selected from the group consisting of FOLR1, TRPV6, SSTR2 and GNRHR.

15. The conjugate compound or the pharmaceutically acceptable salt thereof according to claim 6, wherein the synergistic molecule binds to a molecule selected from the group consisting of FOLR1, TRPV6, FOLH1 (PMSA) and GNRHR.

16. The conjugate compound or the pharmaceutically acceptable salt thereof according to any one of claims 1-15, wherein the synergistic molecule is a folate or an analogue thereof.

17. The conjugate compound or the pharmaceutically acceptable salt thereof according to claim 16, wherein the analogue of a folate is selected from the group consisting of 5-methyltetrahydrofolate, 5-formyltetrahydrofolate, methotrexate, and 5,10-methylenetetrahydrofolate.

18. The conjugate compound or the pharmaceutically acceptable salt thereof according to claim 1 or 6, comprising one, two, three, four or more payloads.

19. The conjugate compound or the pharmaceutically acceptable salt thereof according to claim 1 or 6, wherein the payload is selected from the group consisting of a small molecule compound, a nucleotide, a peptide, and a protein.

20. The conjugate compound or the pharmaceutically acceptable salt thereof according to claim 19, wherein the payload is a small molecule compound.

21. The conjugate compound or the pharmaceutically acceptable salt thereof according to claim 20, wherein the small molecule compound is selected from the group consisting of camptothecin and any derivative thereof, auristatin and any derivative thereof, maytansine and any derivative thereof, a radionuclide complex, a cyclooxygenase-2 inhibitor, paclitaxel and any derivative thereof, epothilone and any derivative thereof, bleomycin and any derivative thereof, dactinomycin and any derivative thereof, plicamycin and any derivative thereof, and mitomycin C.

22. The conjugate compound or the pharmaceutically acceptable salt thereof according to claim 21, wherein the small molecule compound is camptothecin or any derivative thereof, auristatin or any derivative thereof, a radionuclide complex or a cyclooxygenase-2 inhibitor.

23. The conjugate compound or the pharmaceutically acceptable salt thereof according to any one of claims 1-22, wherein the payload is linked to at least one of the targeting molecules via a linker.

24. The conjugate compound or the pharmaceutically acceptable salt thereof according to claim 23, wherein the linker is a peptide linker, a disulphide linker, a pH-dependent linker or a combination of the above-mentioned linkers.

25. The conjugate compound or the pharmaceutically acceptable salt thereof according to claim 24, wherein the peptide linker is cleavable under a certain physiological environment by protease cleavage or reduction.

26. The conjugate compound or the pharmaceutically acceptable salt thereof according to claim 24 or 25, wherein the peptide linker is selected from the group consisting of cysteine, lysine, lysine-lysine, valine-citrulline, phenylalanine-lysine, valine-lysine, cysteine-lysine, cysteine-glutamic acid, aspartic acid-aspartic acid, and aspartic acid-aspartic acid-lysine, and optionally, the carboxylic acid in the above-mentioned amino acids is amidated.

27. The conjugate compound or the pharmaceutically acceptable salt thereof according to claim 24, wherein the disulphide linker is selected from the group consisting of DMDS, MDS, DSDM and NDMDS.

28. The conjugate compound or the pharmaceutically acceptable salt thereof according to claim 24, wherein the pH-dependent linker is a cis-aconitic anhydride.

29. The conjugate compound or the pharmaceutically acceptable salt thereof according to claim 23, wherein the linker has the following structures: 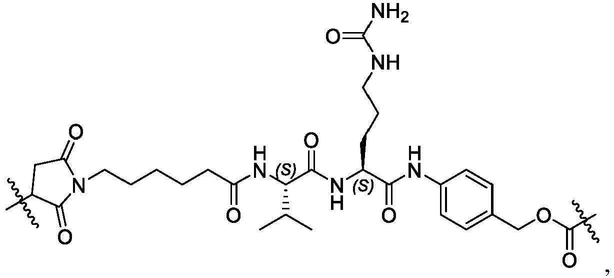 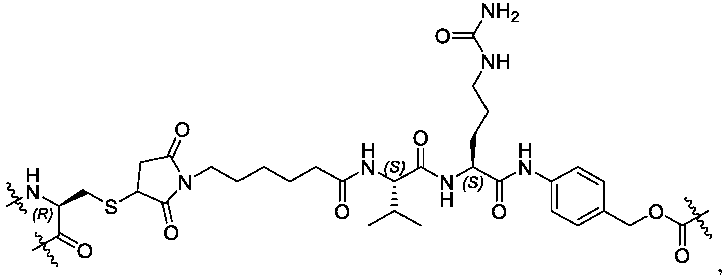 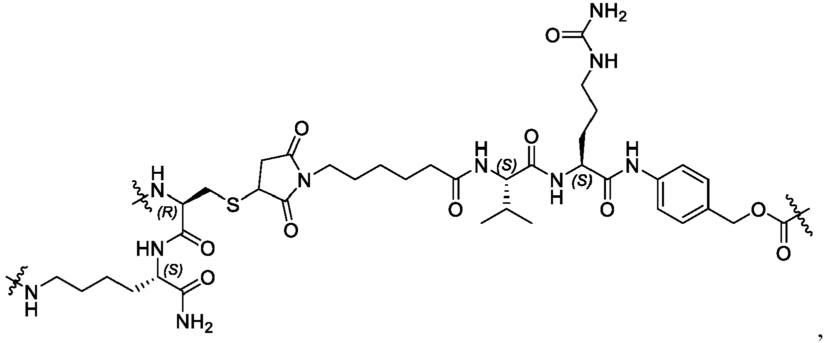 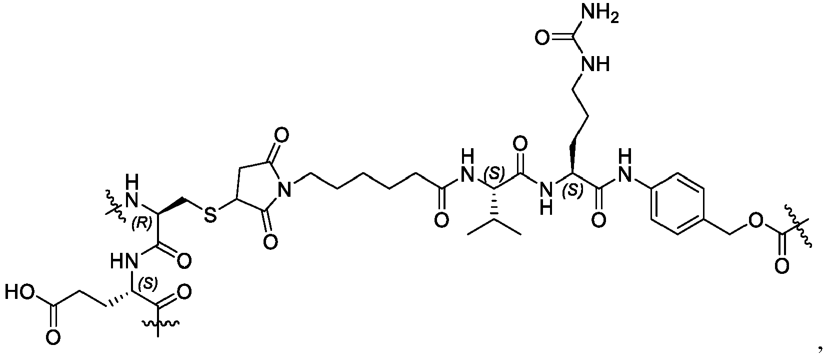 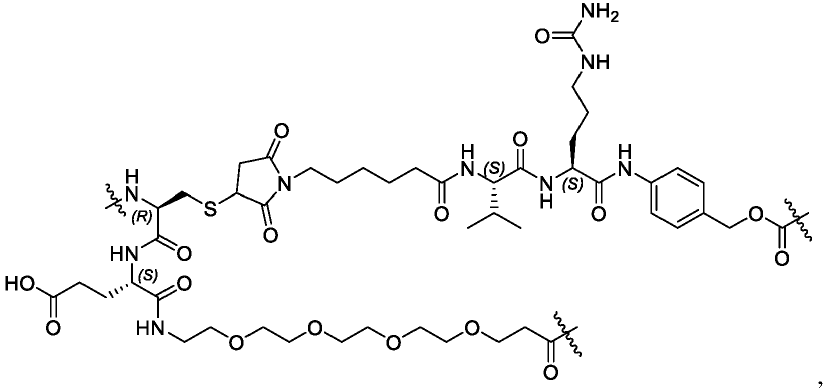 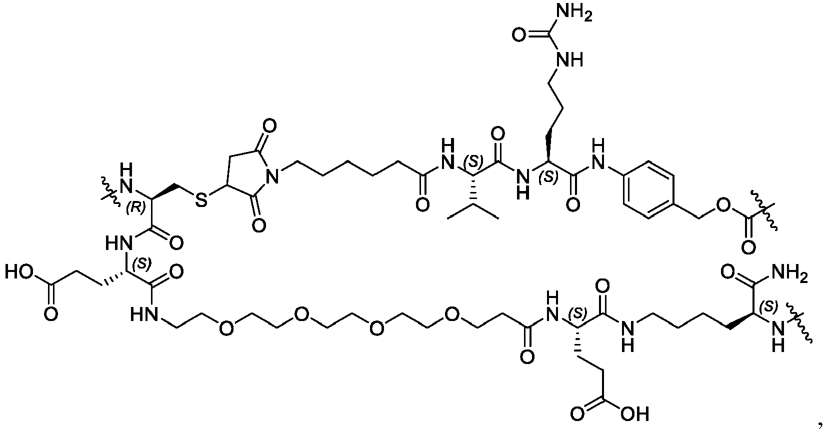 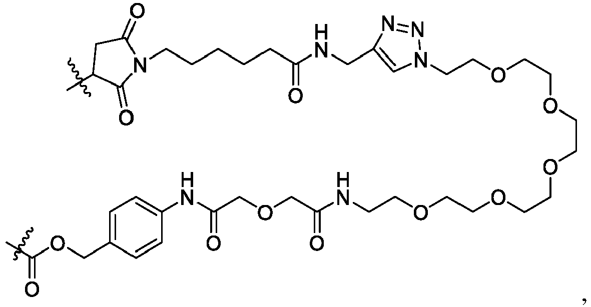 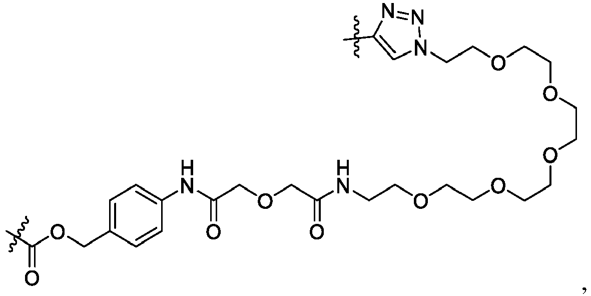 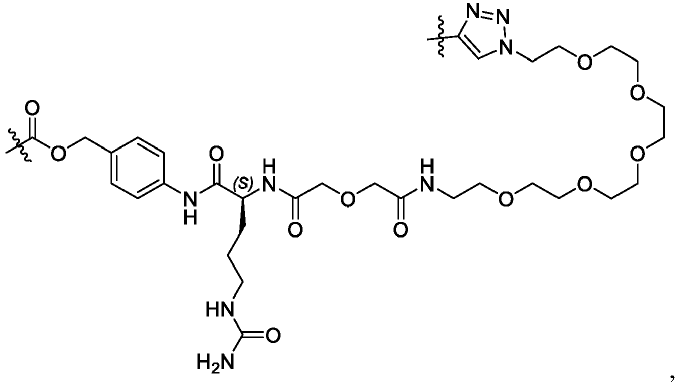 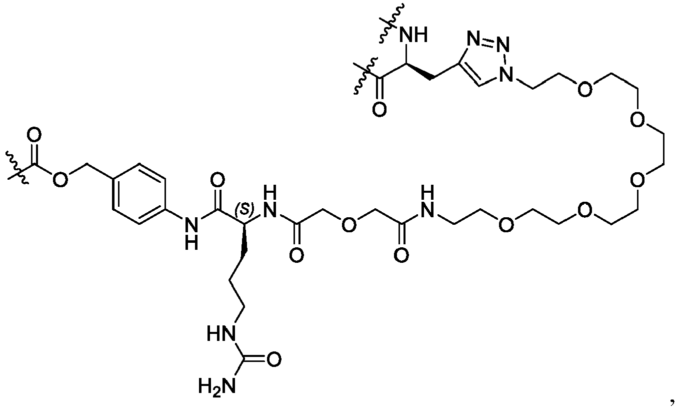 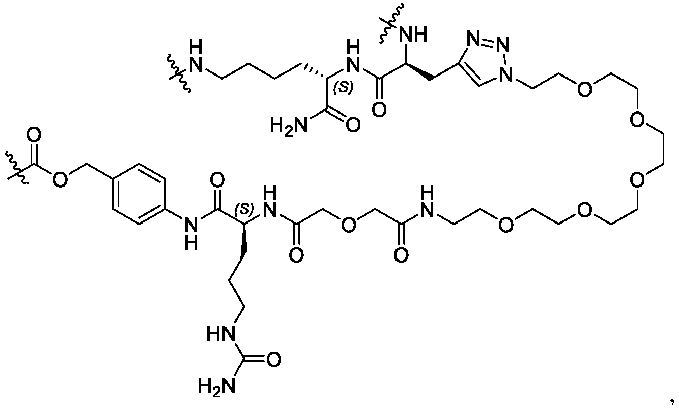 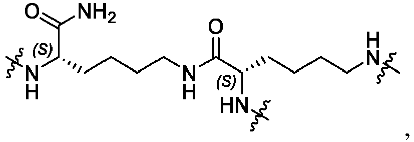 or the linker is a combination of the above-mentioned structures and a peptide linker.

30. The conjugate compound or the pharmaceutically acceptable salt thereof according to any one of claims 1-29, wherein the two targeting molecules are linked to each other via a spacer.

31. The conjugate compound or the pharmaceutically acceptable salt thereof according to claim 30, wherein the spacer comprises the amino acid sequence selected from the group consisting of SEQ ID NOs: 1-14, Arg-Arg, Ala-Ser-Asn, Ala-Ala-Ala, Ser-Ser-Arg, Pro-Arg and Pro-Leu-Gly.

32. The conjugate compound or the pharmaceutically acceptable salt thereof according to claim 1, wherein the conjugate compound is selected from the group consisting of the following compounds: 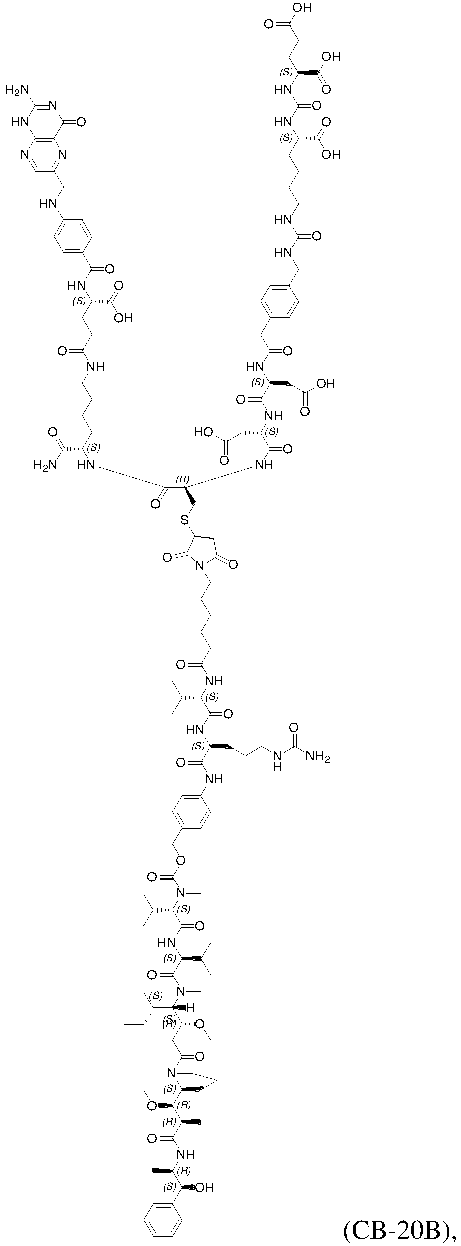 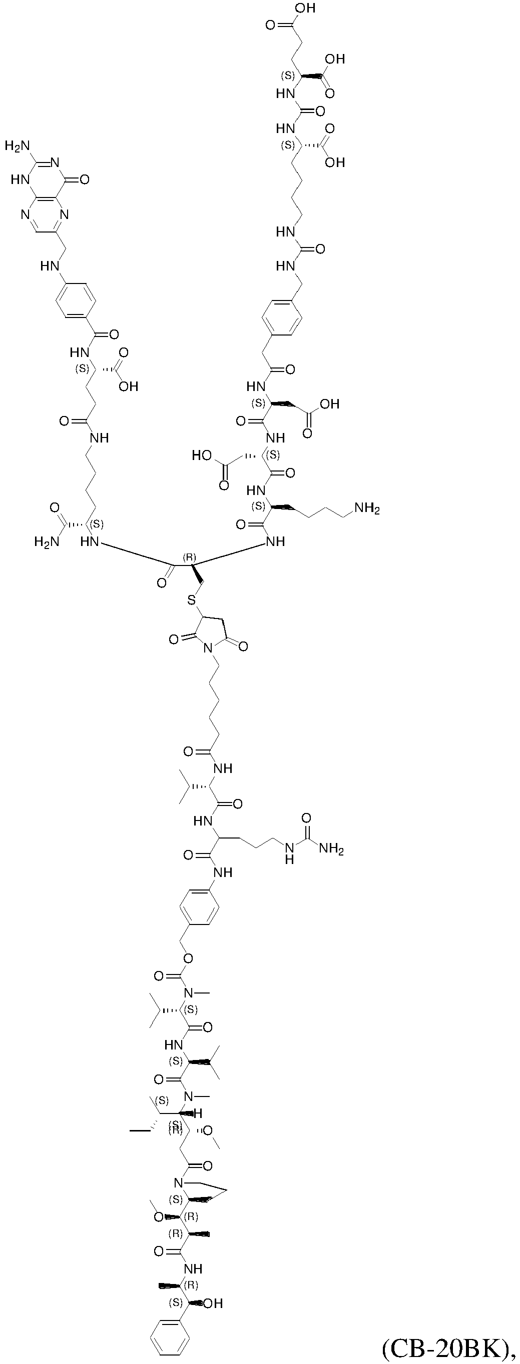 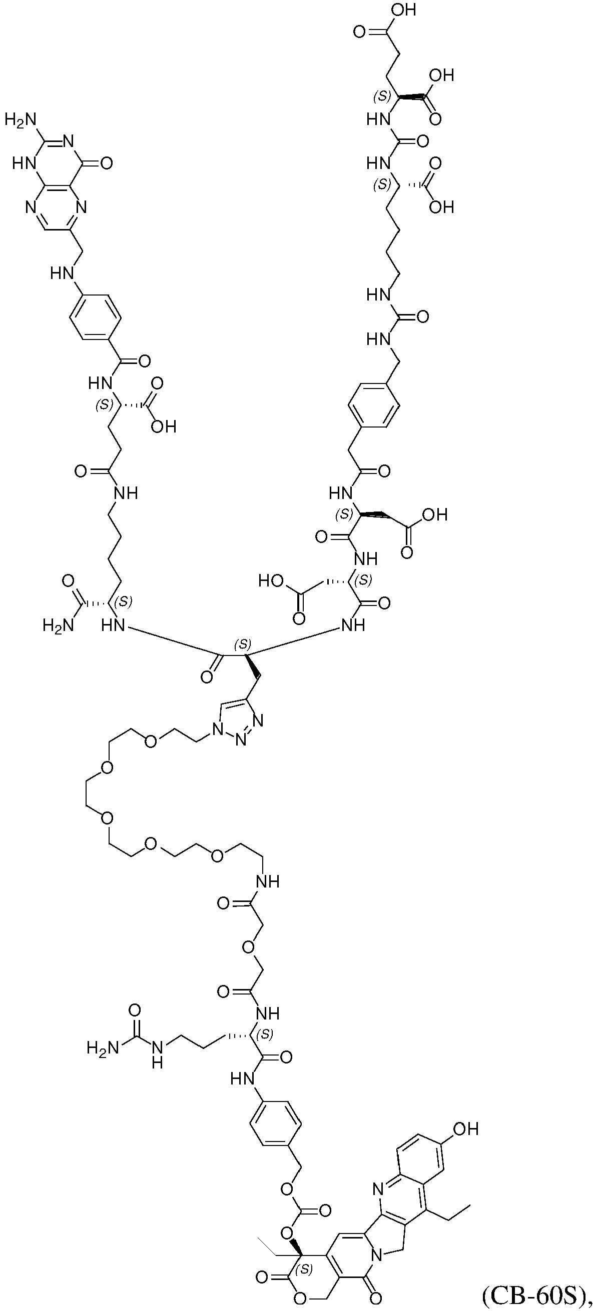 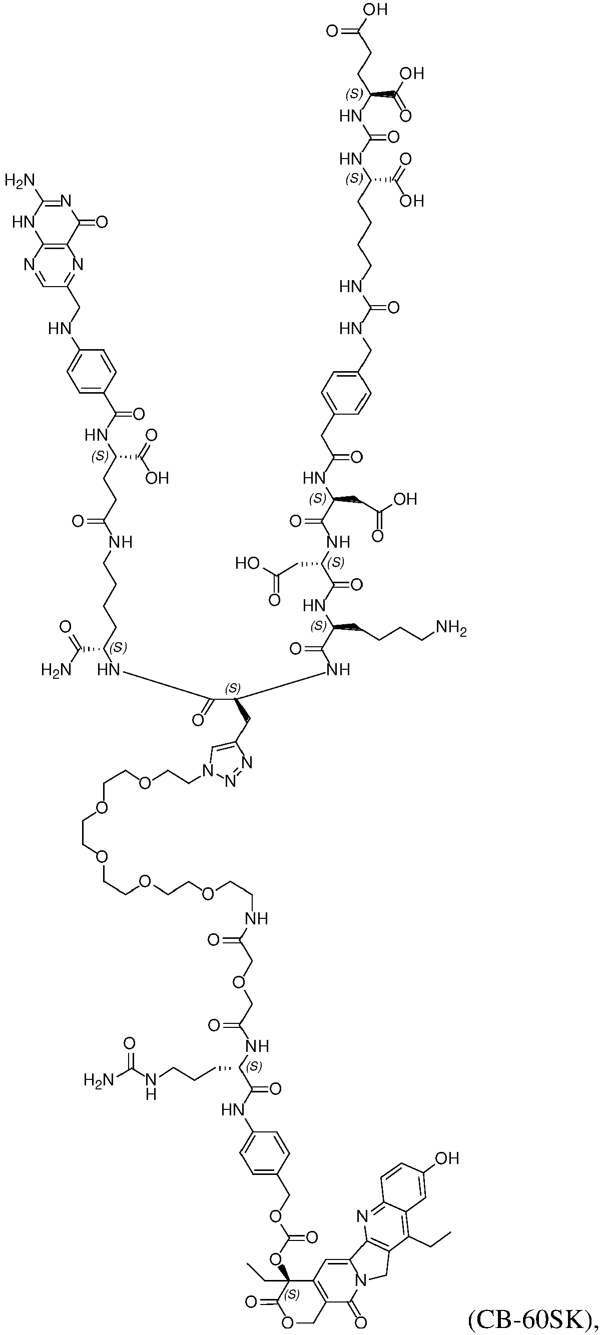 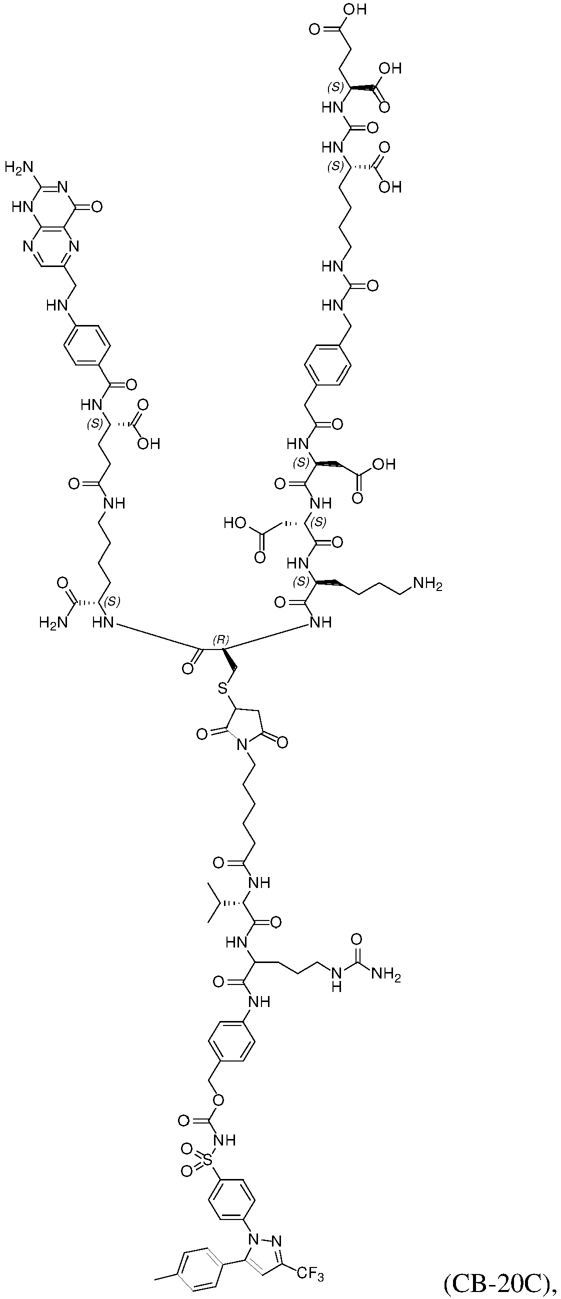 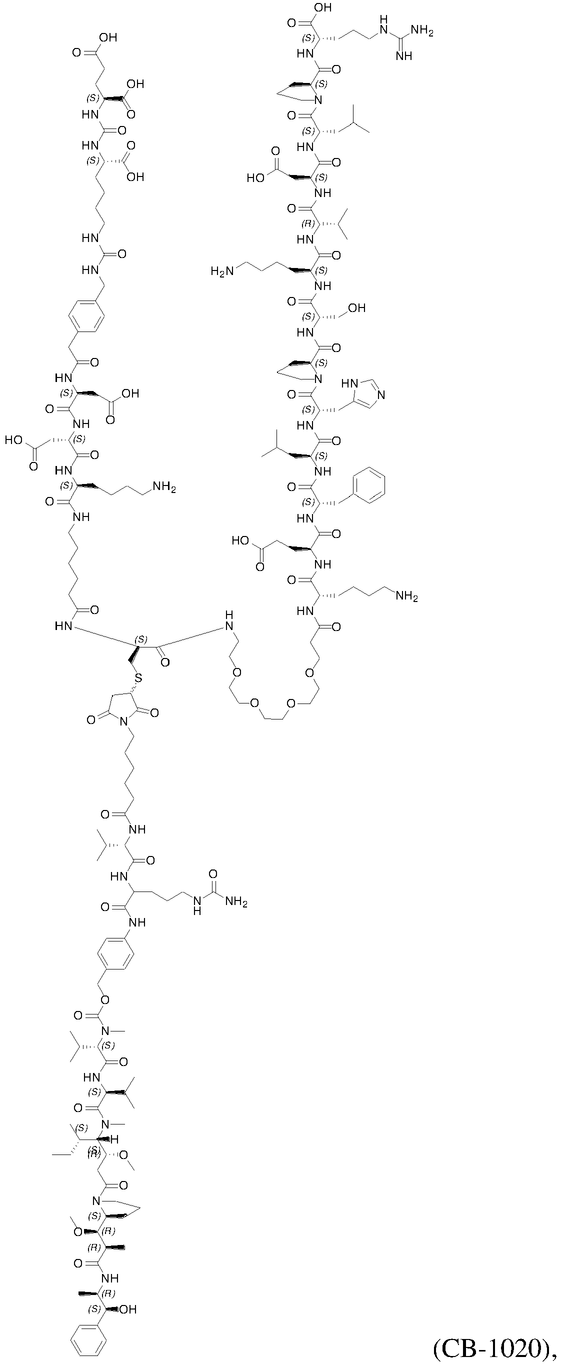 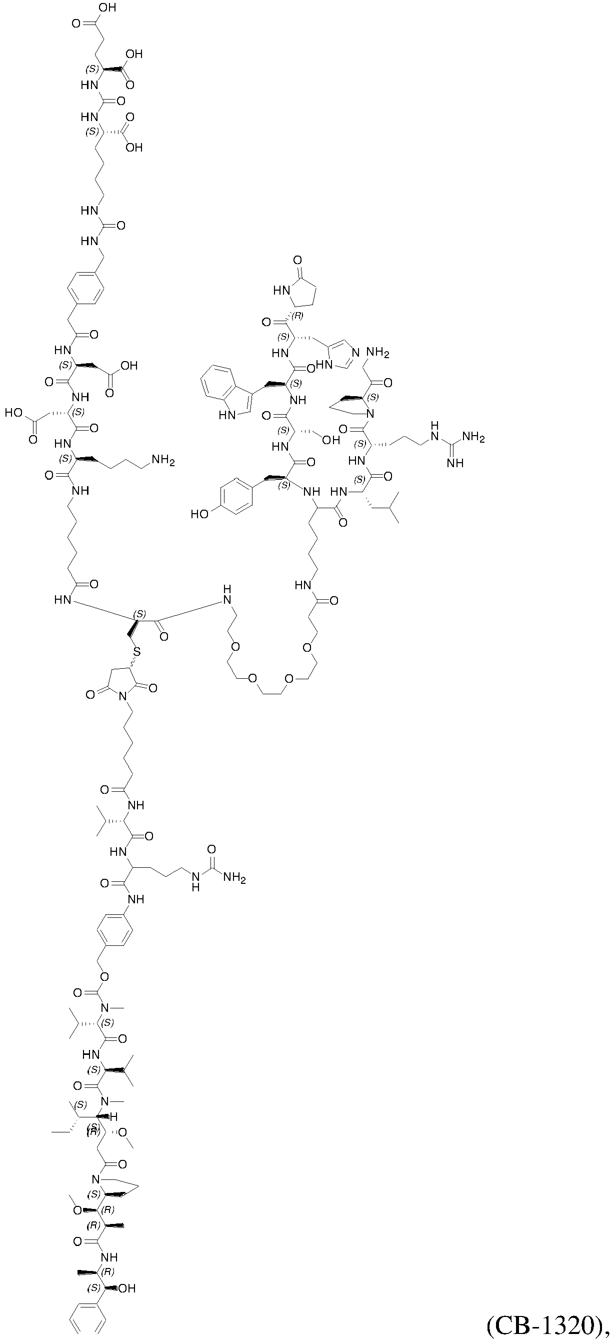 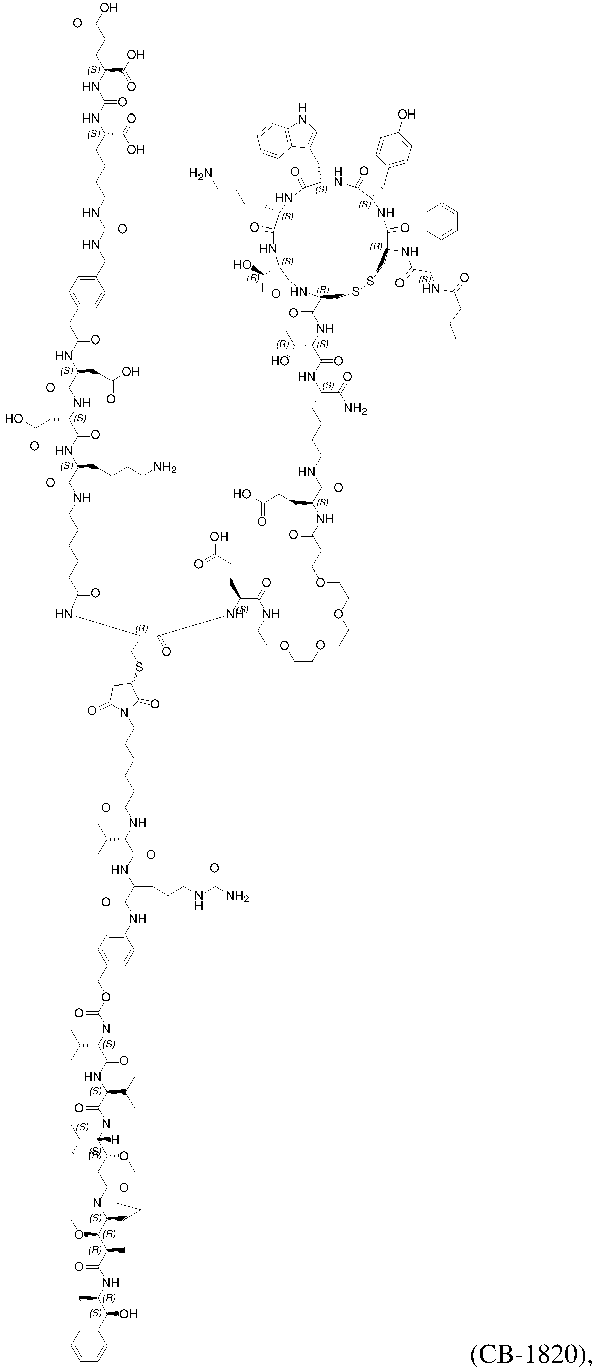 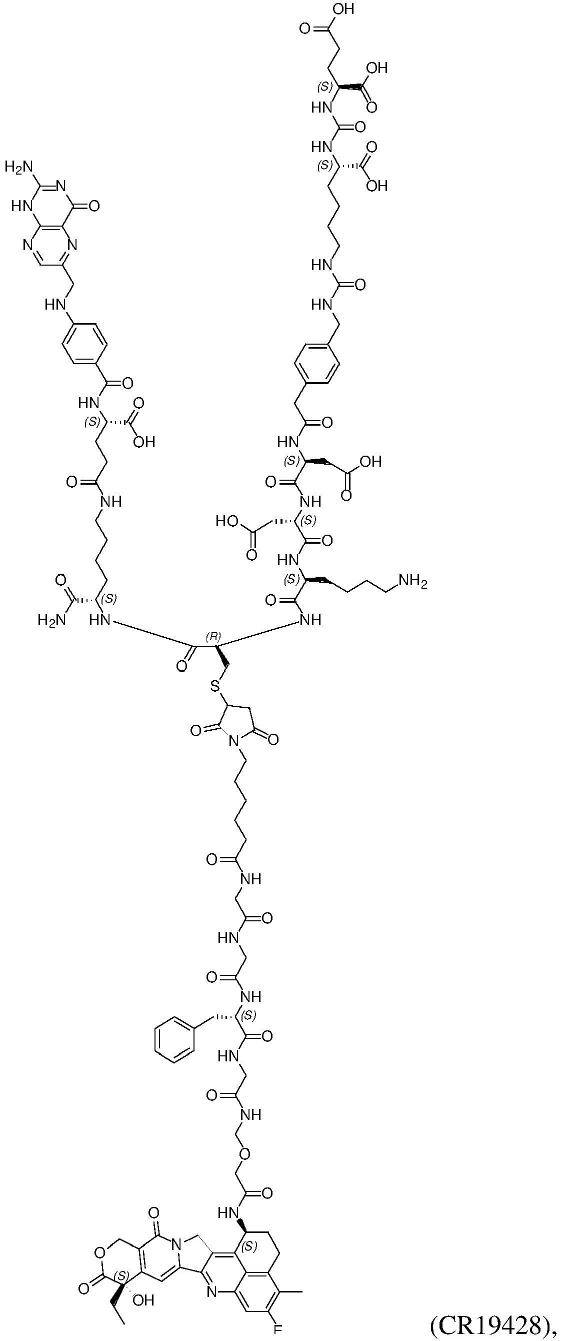 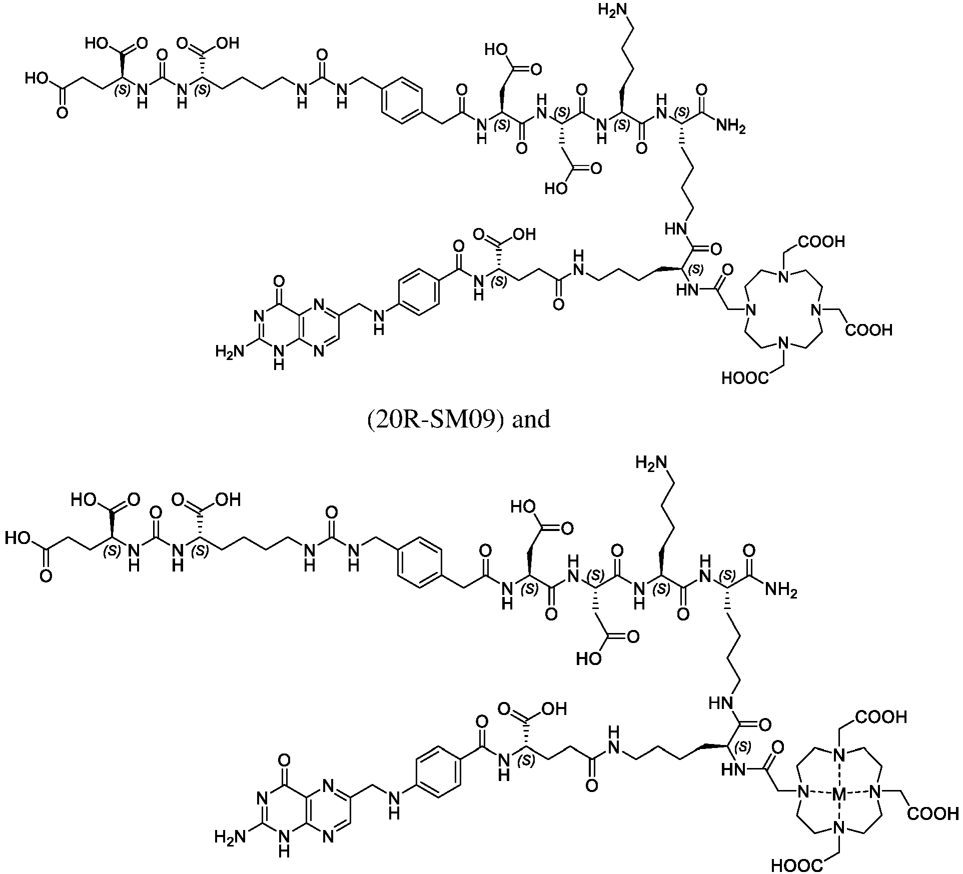 (CB-20R), wherein M is a radionuclide.

33. The conjugate compound or the pharmaceutically acceptable salt thereof according to claim 6, wherein the conjugate compound is:

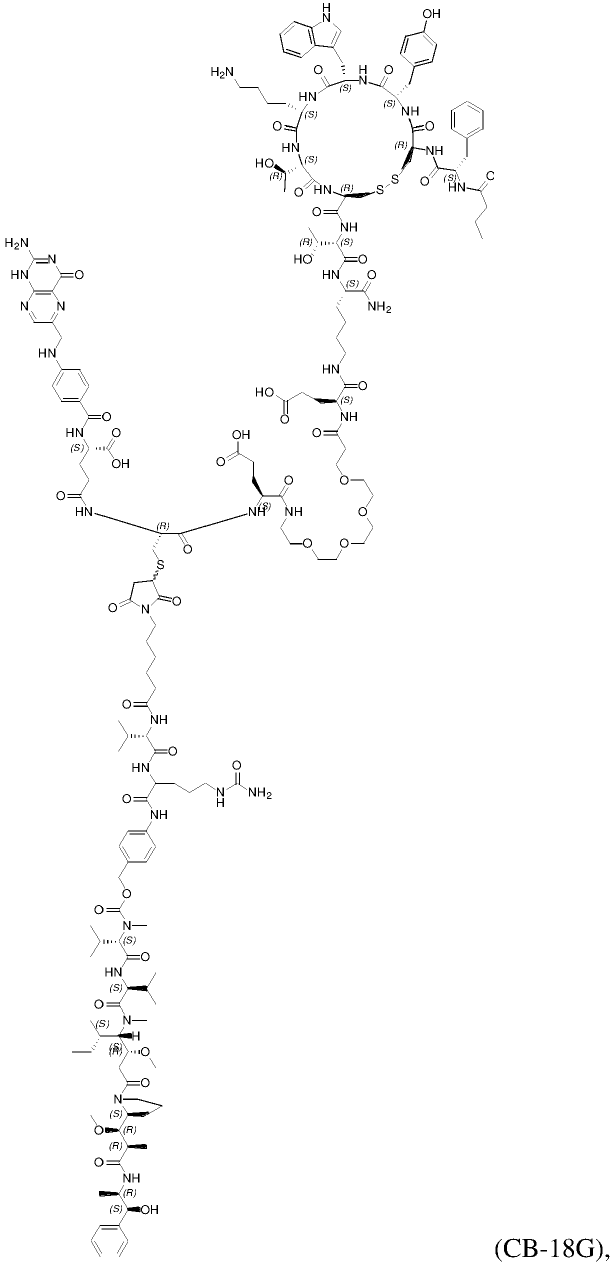

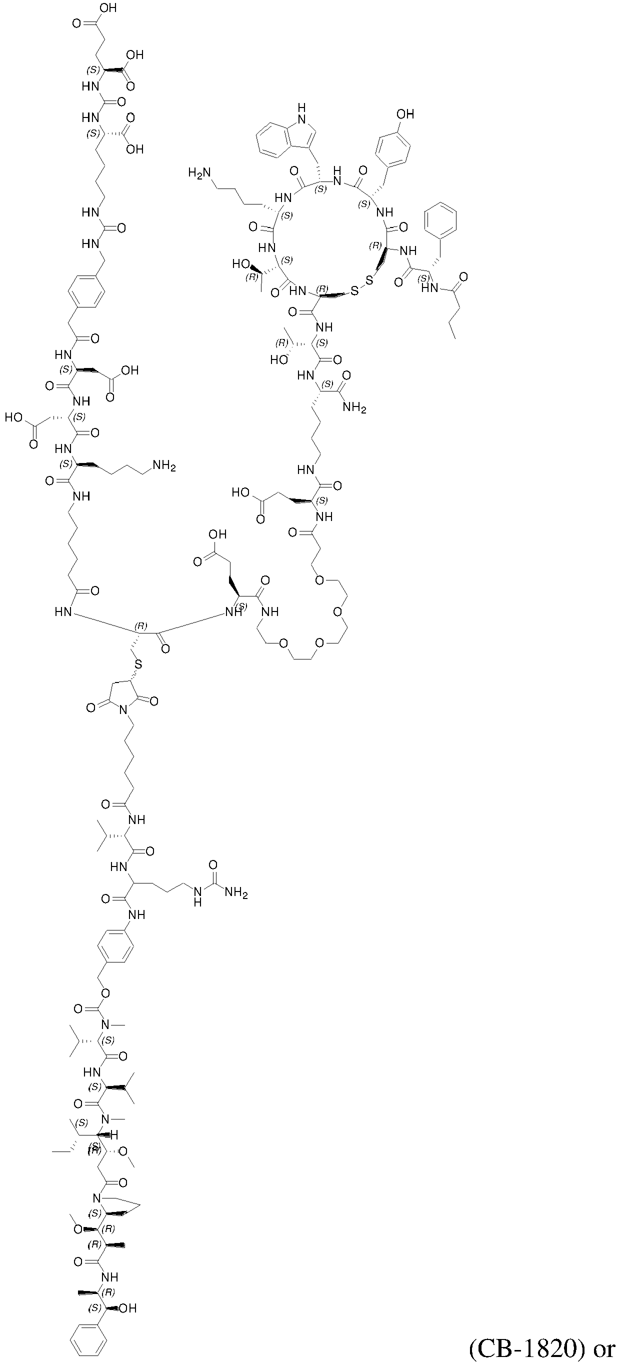

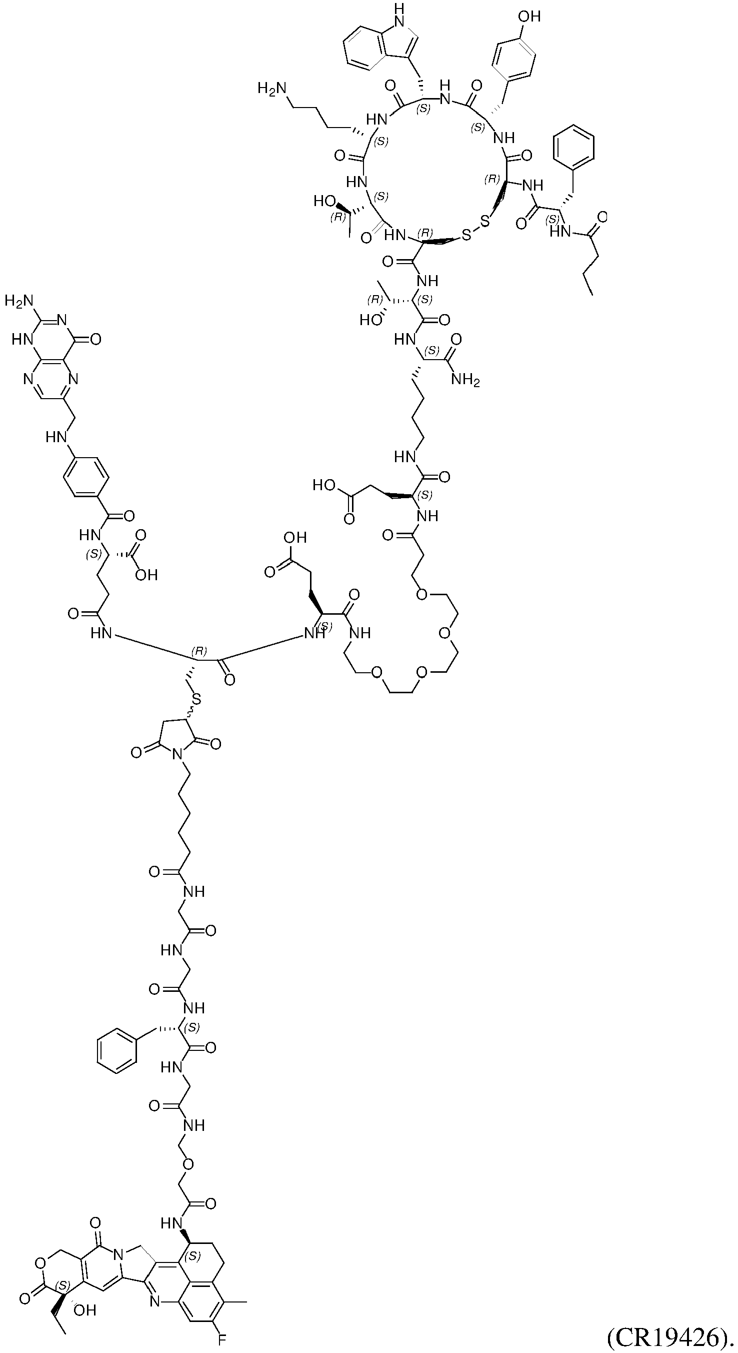

34. The conjugate compound or the pharmaceutically acceptable salt thereof according to claim 7, wherein the conjugate compound is

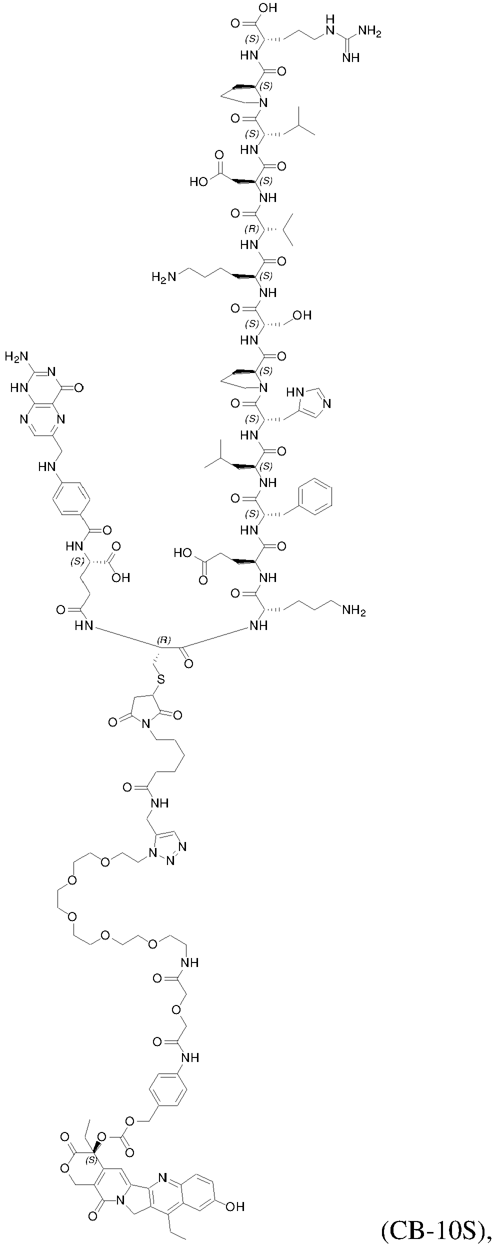

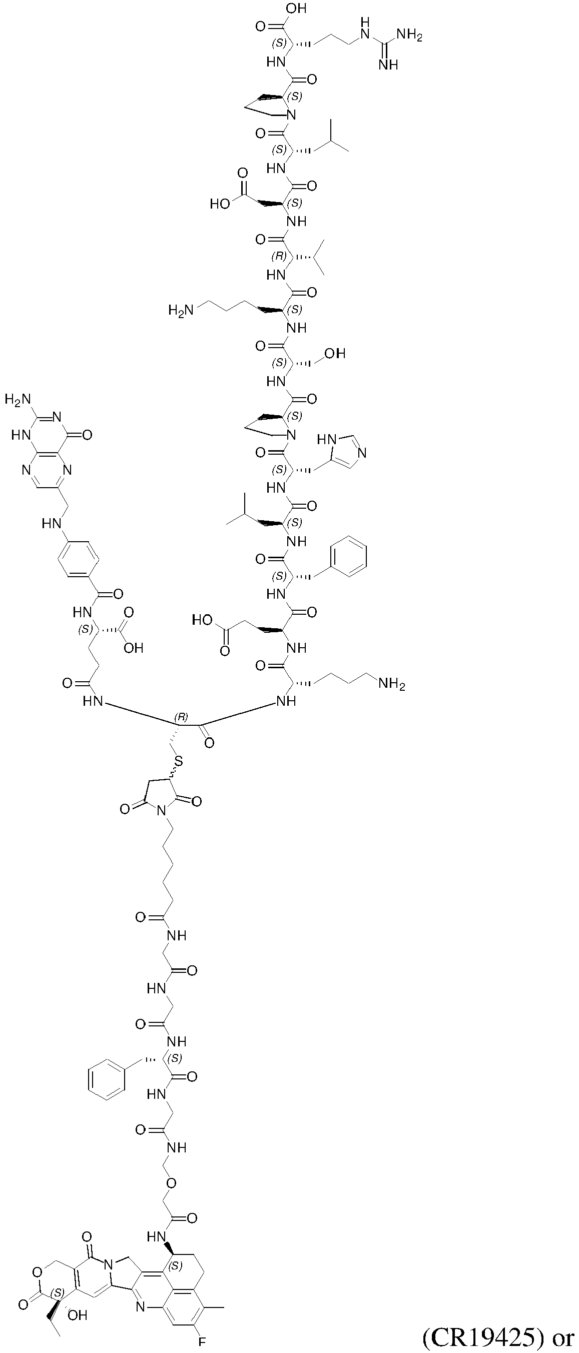

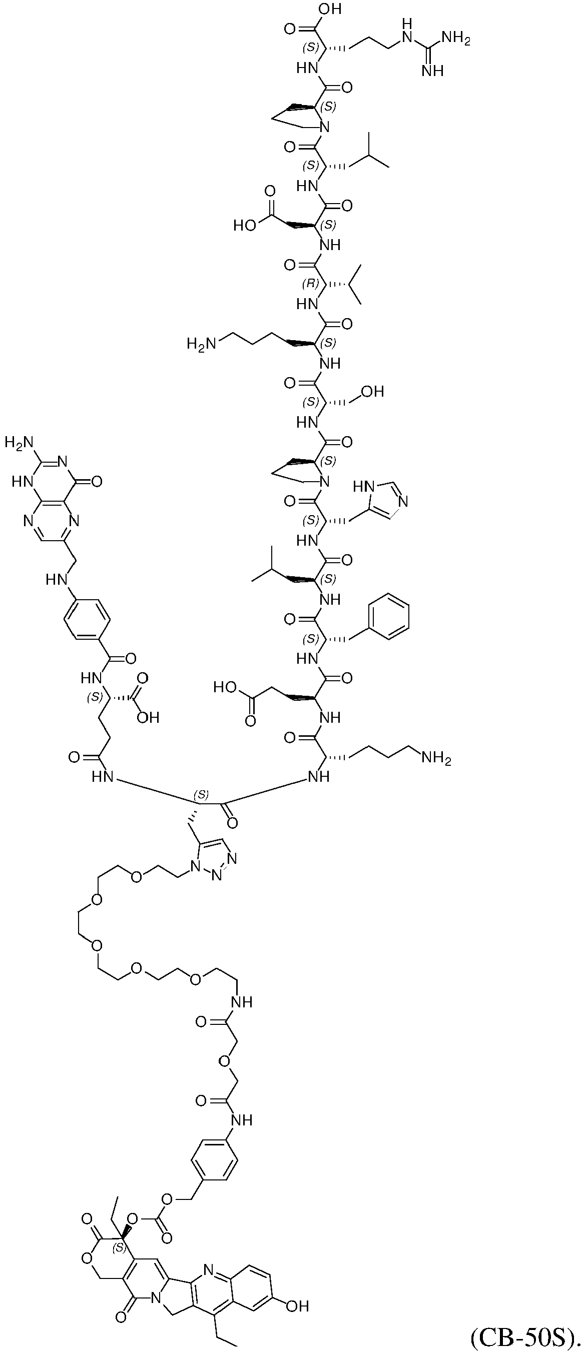

35. A pharmaceutical composition comprising the conjugate compound or the pharmaceutically acceptable salt thereof according to any one of claims 1-34, and a pharmaceutically acceptable carrier.

36. The pharmaceutical composition according to claim 35, wherein the composition is administered intravenously, subcutaneously, orally, intramuscularly or intraventricularly.

37. A method for delivering a payload to a subject in need thereof, comprising administering to the subject a therapeutically effective amount of the conjugate compound or the pharmaceutically acceptable salt thereof according to any one of claims 1-34, or the pharmaceutical composition according to claim 35 or 36.

38. A method for treating a disease in a subject, comprising administering to the subject a therapeutically effective amount of the conjugate compound or the pharmaceutically acceptable salt thereof according to any one of claims 1-34, or the pharmaceutical composition according to claim 35 or 36.

39. The method according to claim 38, wherein the disease is selected from the group consisting of a cancer, an immunological disease, a cardiovascular disease, a metabolic disease, and a neurological disease.

40. The method according to claim 39, wherein the cancer is selected from the group consisting of prostatic cancer, breast cancer, lung cancer, renal cancer, leukaemia, ovarian cancer, gastric cancer, uterine cancer, endometrial carcinoma, liver cancer, colon cancer, thyroid cancer, pancreatic cancer, colorectal cancer, oesophageal cancer, testicular cancer, skin cancer, lymphoma, and multiple myeloma.

41. The method according to claim 39, wherein the immunological disease is an autoimmune disease.

42. The method according to claim 41, wherein the autoimmune disease is selected from the group consisting of connective tissue disease, systemic sclerosis, rheumatoid arthritis, and systemic lupus erythematosus.

43. The method according to claim 39, wherein the cardiovascular disease is selected from the group consisting of angina, myocardial infarction, stroke, heart attack, hypertensive heart disease, rheumatic heart disease, cardiomyopathy, arrhythmia, and congenital heart disease.

44. The method according to claim 39, wherein the metabolic disease is selected from the group consisting of diabetes, gout, obesity, hypoglycaemia, hyperglycaemia, and dyslipidaemia.

45. The method according to claim 39, wherein the neurological disease is selected from the group consisting of Alzheimer's disease, Parkinson's disease, Huntington's disease, head injury, multiple sclerosis, vertigo, coma, and epilepsy.

46. The method according to any one of claims 38-45, wherein the method further comprises administering one or more therapeutic agents in combination with the conjugate compound or the pharmaceutically acceptable salt thereof, or the pharmaceutical composition.
